(19) Europäisches Patentamt
European Patent Office
Office européen des brevets

(11) EP 4 385 995 A1

(12) EUROPEAN PATENT APPLICATION

(43) Date of publication:
**19.06.2024 Bulletin 2024/25**

(21) Application number: **22306855.2**

(22) Date of filing: **12.12.2022**

(51) International Patent Classification (IPC):
***C07F 15/00*** *(2006.01)* ***C07F 17/02*** *(2006.01)*
***A61K 31/351*** *(2006.01)* ***A61P 35/00*** *(2006.01)*
***C12N 5/095*** *(2010.01)*

(52) Cooperative Patent Classification (CPC):
**C07F 15/0033; A61P 35/00; C07F 17/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicants:
- **Centre National de la Recherche Scientifique 75016 Paris (FR)**
- **UNIVERSITE TOULOUSE III - PAUL SABATIER 31062 Toulouse Cedex 9 (FR)**
- **Centre Hospitalier Universitaire De Toulouse 31059 Toulouse Cedex 9 (FR)**

(72) Inventors:
- **GORNITZKA, Heinz 31077 TOULOUSE CEDEX 4 (FR)**
- **HEMMERT, Catherine 31077 TOULOUSE CEDEX 4 (FR)**
- **DERAEVE, Céline 31077 TOULOUSE Cedex 4 (FR)**
- **CUVILLIER, Olivier 31077 TOULOUSE CEDEX 4 (FR)**
- **MADDELEIN, Marie-Lise 31077 TOULOUSE CEDEX 4 (FR)**
- **MALAVAUD, Bernard 31077 TOULOUSE CEDEX 4 (FR)**
- **QIN, Xue 31077 TOULOUSE CEDEX 4 (FR)**
- **WANG, Xing 31077 TOULOUSE Cedex 4 (FR)**

(74) Representative: **Brevalex 56, Boulevard de l'Embouchure B.P. 27519 31075 Toulouse Cedex 2 (FR)**

(54) **VECTORIZED NHC IRIDIUM(III) COMPLEXES AND USE THEREOF IN PHOTODYNAMIC THERAPY AND/OR DIAGNOSTIC**

(57) The present invention concerns vectorized iridium(III) complex with N-heterocyclic carbene-pyridine and a pharmaceutical composition comprising such a complex. The present invention also concerns their use for photodynamic therapy implemented to treat and/or prevent cancers and their use for photodynamic diagnosis.

EP 4 385 995 A1

## Description

### TECHNICAL FIELD

The present invention relates to the general technical field of photodynamic molecules useful as therapeutic agents and/or as *in vivo* or *ex vivo* diagnosis agents.

More particularly, the present invention proposes iridium(III) complexes with particular C^N ligands and specific entities capable of vectoring the complexes to targets of interest and linked to the N-heterocyclic carbene (NHC) which is carbene-pyridine. These new complexes are particularly useful in the treatment of cancers.

### BACKGROUND OF THE INVENTION

In the context of the global surge in cancer, chemotherapy is an effective and widespread way of cancer treatment worldwide. Despite the wide use of the metal-based compounds, the lack of clear distinction between the therapeutic and toxic doses was a major challenge in cancer chemotherapy. More and more researches are thus trying to develop therapeutic procedures with minimal negative consequences. Therefore, photodynamic therapy (PDT) agents for cancer therapy have been widely used due to their various molecular structures, fascinating biological activity and enhanced cancer-targeting ability.

PDT is a modern and rapidly developing method for the treatment of a wide range of diseases. The method is based on selective accumulation of photodynamic compound (PDC) (or photosensitizer (PS)) in the tumor tissue, which is capable of generating cytotoxic agents that cause the death of tumor cells under local exposure to light. PDT requires the simultaneous presence of three essential components: 1) a photosensitizer, 2) oxygen, and 3) light. Absorption of light excites the photosensitizer from the ground electronic state to an excited singlet state, which is converted to a long-lived triplet excited state by intersystem crossing (ISC). Quenching of this state by biomolecules (type I reaction) or directly by molecular oxygen $^3O_2$ (type II reactions, the main photosensitization mechanism in PDT) produces oxygen radicals and singlet oxygen ($^1O_2$), respectively. Both these reactive oxygen species (ROS) can induce severe oxidative damage to cellular biological molecules, resulting in an apoptotic, autophagy, and/or necrotic mode of cell death. Importantly, the photosensitizer returns to the ground state upon quenching and becomes newly available for another cycle. Therefore, in principle, only a small amount of photosensitizer is required to achieve therapeutic efficacy. In addition, it should be noted that several types of photosensitizers can be used not only for treatment purposes but also for diagnosis purposes for tumors which is known as photodynamic diagnosis (PDD).

The clinical photosensitizers actually proposed are difficult to synthetize and purify, present a low solubility, a low absorption and a poor reactive oxygen species (ROS) production.

Amongst the cancers, the prostate cancer is the most common cancer in non-smoking men and bladder cancer is one of the most expensive to treat.

Two drugs approved by the FDA and the EMA use light for therapeutic purposes in urology, the first for photodynamic diagnosis of bladder cancer (Hexvix®) and the second for photodynamic treatment of prostate cancer (Tookad®).

Despite a high level of medical evidence, both are struggling to find their market. Indeed, Hexvix® that is hexaminolevulinate hydrochloride or hexyl 5-amino-4-oxopentanoate hydrochloride requires instillation one hour before rigid endoscopy in the bladder cavity. As it does not have true selectivity for cancer cells, it is subject to frequent false positives. Relatively expensive, it requires special optical equipment to reveal the fluorescence it induces.

Moreover Tookad® is a palladium-metalated bacteriopherophorbide derived from BChl, the bacterial equivalent of plant chlorophyll. After intravenous injection, this photosensitizer diffuses throughout the anatomy without any organ selectivity. It is in fact the secondary illumination of the prostatic tissue by means of vectors inserted under ultrasound control through the trans-perineal route that ensures the activation of the drug and the thrombosis of the small vessels thus illuminated. Tissue death occurs by ischemia without selectivity for either the glandular component or for the tumor cells that would have originated from it. The complexity of implementation, the side effects such as photosensitization when exposed to daylight for 12 hours and the lack of selectivity limit the diffusion of this rather elegant procedure.

There is a need for photodynamic compounds (PDC) that are useful as photosensitizers for PDT that are both disease-modifying and effective in treating patients suffering from prostate cancers or with bladder cancers. There is also a need for PDC that are useful as *in vivo* or *ex vivo* diagnostic agents for such cancers. Moreover, it is desired to provide PDCs having: (1) increased photostability, (2) increased absorption at activation wavelength, (3) red-light absorption, (4) a poor cytotoxicity associated with a high photocytotoxicity and (5) a high selectivity for cancer cells.

### DISCLOSURE OF THE INVENTION

The present inventors have proposed particular cyclometalated complexes in order to solve the drawbacks of

the prior art photosensitizers. In particular, the present inventors have designed modulable, more specific and more efficient cyclometalated cationic iridium(III) complexes in which the lipophilic/hydrophilic balance of the complexes can vary by modulating the ligands coordinated to the metal (hereinafter designated as "C^N ligands"), and in which the specificity can be adapted to the cancer cells and the excitation wavelengths can be varied for activation, which often represents a major problem in PDT.

Indeed, the iridium(III) complexes of the invention comprises (i) a targeting vector directly or indirectly linked to the N-heterocyclic carbene-pyridine (NHC-pyridine) thanks to which the selectivity towards cancer cells and, in particular, prostate cancer cells and bladder cancer cells is increased and (ii) C^N ligands, the chemical structure may differ in order to modulate the photophysical and biological properties of the resulting complexes.

More particularly, when the targeting vector is capable of targeting the iridium(III) complexes of the invention to prostate cancer cells, the selectivity of these complexes, compared to the effects on the epithelial cells at the origin of prostate cancer and the fibroblasts that surround them, makes it possible to preserve the architecture of the prostate and the erectile nerves that run in contact with it, thus limiting the consequences of focused ablative treatments on continence and erection.

In addition, combining two minimally invasive procedures, intravesical instillation of the iridium(III) complexes of the invention and illumination with a flexible video-endoscope, our solution is particularly adapted to a fragile population with a disease that often recurs, such as bladder cancer that does not invade the bladder muscle (formerly called superficial bladder cancer).

The cyclometalated iridium(III) complexes of the invention have excellent phosphorescence performance particularly useful for diagnosis, large Stokes shift, long-life phosphorescence, and excellent color adjustment ability. In addition, the photophysical and biological properties of iridium(III) complexes can be easily adjusted by the C^N ligands. Therefore, the integration of the anticancer and phosphorescent properties of cyclometalated iridium(III) complexes provides an opportunity to construct a new therapeutic and diagnostic platform that may be useful not only in bladder cancers and prostate cancers but also in any type of cancers. Indeed, the cyclometalated iridium(III) complexes of the invention can present a good selectivity depending on their charge and targeting entities, adaptable for different types of cancers.

Moreover, the cyclometalated iridium(III) complexes of the invention, are easily obtained by a few-steps-synthesis well-known by the one skilled in the art and easy purification with high yields.

The present invention concerns an iridium(III) complex of formula (I)

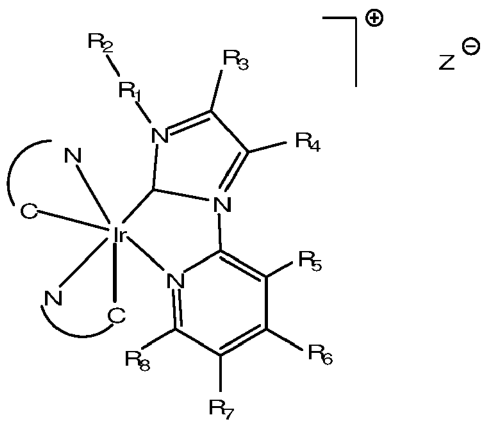

(I)

in which

- $Z^-$ represents a halide anion, a nitrate anion ($-NO_3^-$) or a non-coordinating anion,
- $-R_1-$ represents a covalent link or a spacer segment,
- the radical $R_2$ represents a targeting entity or ferrocene,
- the radicals $R_3$ and $R_4$, identical or different, represent a hydrogen atom, a halogen atom, an alkyl group optionally substituted, an aryl group optionally substituted, an alkylaryl group optionally substituted, an arylalkyl group optionally substituted, an ether group (-OR), an amine group (-NRR'), a borane group (-BRR'), a thioether group (-SR), and a phosphine group (-PRR');
- the radicals $R_5$, $R_6$, $R_7$ and $R_8$, identical or different, represent a hydrogen atom, a halogen atom, a cyano group (-CN), a nitro group ($-NO_2$), a carboxylic group or a carboxylic acid ester group (-C(=O)OR), an alkyl group optionally substituted, an aryl group optionally substituted, an alkylaryl group optionally substituted, an arylalkyl group optionally substituted, a hydroxyl group or an ether group (-OR), an aldehyde group or a ketone group (-C(=O)R), an amine

group (-NRR'), a cyanate group (-OC≡N), an isocyanate group (-N=C=O), a carboxamide group (-NRR'), a boronic acid group ($-B(OR)_2$), a sulfhydryl group or a thioether group (-SR), an iminothiol group (-C(NR)SR') and a trimethylsilylacetylene group (-CCSiMe3);
the ligands

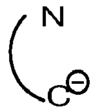

(also designated as C^N ligands) are either of formula (II) or of formula (III)

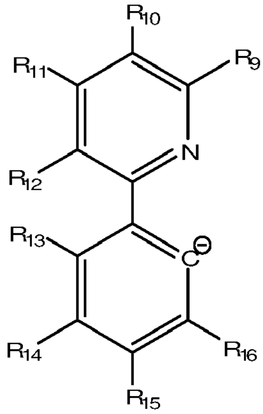
(II) or
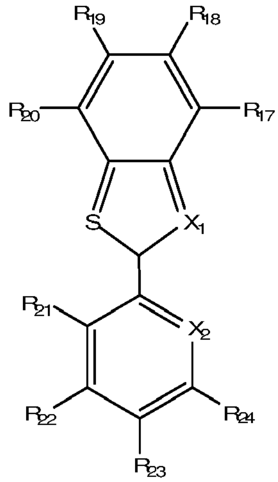
(III)

in which

($X_1$, $X_2$) represents either (N, CΘ) or (CΘ, N),
the radicals $R_9$ to $R_{24}$, identical or different, represent a hydrogen atom, a halogen atom, a cyano group (-CN), a nitro group ($-NO_2$), a carboxylic group (-COOH), a carboxylic acid ester group (-COOR), an alkyl group optionally substituted, an aryl group optionally substituted, an alkylaryl group optionally substituted or an arylalkyl group optionally substituted;
the radicals $R_9$ and $R_{10}$, the radicals $R_{10}$ and $R_{11}$, the radicals $R_{11}$ and $R_{12}$, the radicals $R_{12}$ and $R_{13}$, the radicals $R_{13}$ and $R_{14}$, the radicals $R_{14}$ and $R_{15}$, the radicals $R_{15}$ and $R_{16}$, the radicals $R_{17}$ and $R_{18}$, the radicals $R_{18}$ and $R_{19}$, the radicals $R_{19}$ and $R_{20}$, the radicals $R_{21}$ and $R_{22}$, the radicals $R_{22}$ and $R_{23}$ and the radicals $R_{23}$ and $R_{24}$ may form with the carbons to which they are attached a conjugated system of delocalized electrons,
R and R', identical or different, representing a hydrogen atom or an alkyl group optionally substituted.

In the iridium(III) complex of the invention, $Z^-$ may represent a halide anion such as a chloride anion, a fluoride anion, a bromide anion or an iodide anion. Advantageously, Z- represents a chloride anion or a bromide anion. In particular, Z- represents a chloride anion.
In the iridium(III) complex of the invention, $Z^-$ may represent a nitrate anion ($-NO_3^-$).
In the iridium(III) complex of the invention, $Z^-$ may be a non-coordinating anion.
As used herein, the term "non-coordinating anion" is used interchangeably with the term "weakly coordinating anion" and means an anion that either does not coordinate to a cation or is only weakly coordinated to a cation thus remaining sufficiently labile to be displaced by a neutral Lewis base. Any non-coordinating anion known to the person skilled in the art can be used in the context of the present invention. Advantageously, this non-coordinating anion may be selected from the group consisting of tetrafluoroborate ($BF_4^-$), hexafluorophosphate ($PF_6^-$), benzenesulfonate ($CH_5SO_3^-$), bis(trifluoromethanesulfonyl)imide ($(N[SO_2(CF_3)]_2)^-$), methanesulfonate (mesylate, $CH_3SO_3^-$), perchlorate ($ClO_4^-$), tetrachloroaluminate ($AlCl_4^-$), tetrakis[3,5-bis(trifluoromethyl)phenyl]borate ($B[3,5-(CF_3)_2C_6H_3]_4^-$), tetrakis(hexafluoroisopropyl)aluminate ($Al[OC(CF_3)_3]_4^-$), tetrakis(pentafluorophenyl)borate ($B[C_6F_5]_4^-$), p-toluene sulfonate (tosylate, $CH_3C_6H_4SO_2^-$) and trifluoromethanesulfonate (triflate, $CF_3SO_3^-$).
"Alkyl group" is taken to mean, within the scope of the present invention, a linear, branched or cyclic alkyl group, comprising from 1 to 15 carbon atoms, particularly from 1 to 10 carbon atoms and in particular, from 1 to 6 carbon atoms,

which can optionally comprise at least one heteroatom and/or at least one carbon-carbon double bonds or triple bonds, such as, for example, a methyl, ethyl, n-propyl, isopropyl, butyl, isobutyl, sec-butyl, tert-butyl, pentyl, isopentyl, hexyl, heptyl, octyl or nonyl group.

"Aryl group" is taken to mean, within the scope of the present invention, any functional group or substituent derived from at least one simple aromatic ring wherein an aromatic ring corresponds to any planar cyclic compound having a delocalized $\pi$ system in which each atom of the ring comprises a p-orbital, said p-orbitals overlapping themselves. An aryl group comprises one aromatic ring or several aromatic rings, identical or different, joined or connected by a single bond or by a hydrocarbon group, each ring having from 4 to 20 carbon atoms, particularly from 4 to 14 carbon atoms, in particular, from 4 to 8 carbon atoms and which can optionally comprise at least one heteroatom. As examples of aryl groups, phenyl, biphenyl, naphthyl, anthracenyl, cyclopentadienyl, pyrenyl, tetrahydronaphthyl, furanyl, pyrrolyl, thiophenyl, oxazolyl, pyrazolyl, isoquinolinyl, thiazolyl, imidazolyl, triazolyl, pyridinyl, pyranyl, quinolinyl, pyrazinyl and pyrimidinyl groups may be cited.

"Alkylaryl group" is taken to mean, within the scope of the present invention, any group derived from an aryl group as defined above wherein a hydrogen atom is replaced by an alkyl group as defined above.

"Arylalkyl group" is taken to mean, within the scope of the present invention, any group derived from an alkyl group as defined above wherein a hydrogen atom is replaced by an aryl group as defined above.

"Substituted alkyl group", "substituted aryl group", "substituted alkylaryl group" or "substituted arylalkyl group" are taken to mean, within the scope of the present invention, an alkyl, aryl, alkylaryl or arylalkyl group as defined previously substituted by a group or several groups, identical or different, selected from the group consisting of a halogen atom; an amine; a diamine; a cyano; a carboxyl; a carboxylate; an aldehyde; an ester; an ether; a hydroxyl; an aryl optionally substituted as defined previously and particularly such as a phenyl, a benzyl or a naphthyl; an alkyl optionally substituted as defined previously and particularly such as a methyl, an ethyl, a propyl or a hydroxypropyl; an amide; a sulphonyl; a sulphoxide; a sulphonic acid; a sulphonate; an acyl; a vinyl; an epoxy; a phosphonate; an isocyanate; a sulfhydryl; a glycidoxy; an acryloxy; a thiophene; a furan; or a selenophene.

As particular examples of substituted alkyl group that may be used in the present invention, one can cite an alkyl group substituted by at least one halogen atom, a perhalogenated alkyl group, an alkyl group substituted by at least one ether group, an alkyl group substituted by at least one amine group, and an alkyl group substituted by at least one cyano group.

"Heteroatom" is taken to mean, within the scope of the present invention, an atom chosen from the group consisting of nitrogen, oxygen, phosphorous, sulphur, silicon, fluorine, chlorine and bromine.

"Halogen" is taken to mean, within the scope of the present invention, an atom chosen from the group consisting of fluorine, chlorine, iodine and bromine. Advantageously, when a halogen is implemented in the present invention, it is a fluorine or a chlorine.

"Conjugated system of delocalized electrons" is taken to mean, within the scope of the present invention, a system of alternating pi- and sigma bonds. Optionally, one or more two-atom structural units having the pi-bond between its atoms can be replaced by an atom bearing at least one lone electron pair, typically by a divalent atom selected from O, S, Se and Te or by a trivalent atom selected from N, P, As, Sb and Bi.

Advantageously, the conjugated system of delocalized electrons in the complex of the invention comprises at least one aromatic ring adhering to the Hückel rule. It is clear that the aryl groups as previously defined belong to the conjugated systems of delocalized electrons.

In a particular embodiment, the conjugated system of delocalized electrons in the complex of the invention may comprise or consist of a condensed aromatic skeleton comprising at least 6 delocalized electrons, such as, for example, a benzene, naphthalene, anthracene, phenanthrene, pyrene, quinoline, indole or carbazole skeleton.

In another particular embodiment, the conjugated system of delocalized electrons in the complex of the invention may comprise or consist of at least two aromatic rings, identical or different, directly attached or connected by a single bond or by a spacer segment as hereinafter defined. As examples of such systems, one can cite a biphenyl, bithienyl, phenylthiophene, phenylpyridine, and polythiophene.

In the iridium(III) complex of the invention, $-R_1-$ as defined in formula (I) represents a covalent link which means that the targeting entity is covalently linked to one of the nitrogen atoms of the N-heterocyclic carbene-pyridine entity (i.e. NHC-pyridine) of the complex.

Alternatively, $-R_1-$ as defined in formula (I) represents a spacer segment. In this alternative, a first atom of this spacer segment is covalently linked to one of the nitrogen atoms of the NHC-pyridine of the complex and a second atom of this spacer segment, different from the first one, is covalently linked to the targeting entity.

In this alternative, this spacer segment is selected from the group consisting of an alkylene chain optionally substituted, an alkenylene chain optionally substituted, an alkynylene chain optionally substituted and an arylene chain optionally substituted.

"Alkylene chain" is taken to mean, within the scope of the present invention, a linear, branched or cyclic alkylene chain, comprising from 1 to 40 carbon atoms, particularly from 2 to 30 carbon atoms and which can optionally comprise

at least one heteroatom. By way of examples of alkylene chains that may be used, methylene, ethylene, n-propylene, isopropylene, butylene, isobutylene, sec-butylene, tert-butylene, pentylene, isopentylene, hexylene, cyclopentylene, cyclohexylene, $-(CH_2)_n-O-(CH_2)_m-$, $-(CH_2)_n-S-(CH_2)_m-$, $-(CH_2)_n-S-S-(CH_2)_m-$, $-(CH_2)_n-C(O)O-(CH_2)_m-$, $-(CH_2)_n-N(R_{25})-(CH_2)_m-$, $-(CH_2)_n-C(O)-N(R_{25})-(CH_2)_m-$, $-(CH_2)_n-N(R_{25})-(CH_2)_p-N(R_{26})-(CH_2)_m-$ and $-[-O-(CH_2)_2-]_n-O-$ groups may be cited with $R_{25}$ and $R_{26}$, identical or different, representing a hydrogen atom, an alkyl group optionally substituted or an aryl group optionally substituted and n, m and p, identical or different, representing a whole number comprised between 0 and 20.

"Alkenylene or alkynylene chain" is taken to mean, within the scope of the present invention, a linear, branched or cyclic alkenylene or alkynylene chain, comprising from 4 to 40 carbon atoms and particularly from 4 to 30 carbon atoms and which can optionally comprise at least one heteroatom. As examples of alkenylene or alkynylene chains that may be used, butenylene or butynylene, isobutenylene or isobutynylene, *sec*-butenylene or *sec*-butynylene, *tert*butenylene or *tert*-butynylene, pentenylene or pentynylene, isopentenylene or isopentynylene, cyclopentenylene or cyclopentynylene, cyclohexenylene or cyclohexynylene groups may be cited.

"Arylene chain" is taken to mean, within the scope of the present invention, an arylene chain with one ring or several rings joined or connected by a single bond or by a hydrocarbon group, each ring having from 4 to 50 carbon atoms and particularly from 4 to 40 carbon atoms and which can comprise optionally at least one heteroatom. As examples of arylene chains that may be used, phenylene, biphenylene, naphthylene, anthracenylene, cyclopentadienylene, pyrenylene, tetrahydronaphthylene, furanylene, pyrrolylene, thiophenylene, selenophenylene, oxazolylene, pyrazolylene, isoquinolinylene, thiazolylene, imidazolylene, triazolylene, pyridinylene, pyranylene, quinolinylene, pyrazinylene and pyrimidinylene groups may be cited.

"Substituted alkylene chain", "substituted alkenylene or alkynylene chain" or "substituted arylene chain" are taken to mean, within the scope of the present invention, an alkylene chain, an alkenylene or an alkynylene chain or arylene chain as defined previously having one substitution or several substitutions, identical or different, this or these substitutions corresponding, preferably, to halogen atoms or to aliphatic groups optionally comprising at least one heteroatom such as, for example, a $-COOR_{27}$, -CHO, $-OR_{27}$, $-SR_{27}$, $-SCOR_{27}$, $-SO_2R_{27}$, $-NR_{28}R_{29}$, $-CONR_{28}R_{29}$, $-C(Hal)_3$, $-OC(Hal)_3$, -C(O)Hal or -CN group in which $R_{27}$, $R_{28}$ and $R_{29}$, identical or different, represent a hydrogen atom, an alkyl group optionally substituted or an aryl group optionally substituted and Hal represents a halogen atom, particularly fluorine, chlorine or bromine.

As spacer segment which can be more particularly implemented within the scope of the present invention, one may cite

- a chain $-(CH_2)_n-N(R_{25})-(CH_2)_m-$ with n, $R_{25}$ and m as previously defined and, in particular, a chain $-(CH_2)_n-N(R_{25})-(CH_2)_m-$ in which n is 3, $R_{25}$ is an hydrogen atom and m is 0; and
- a chain $-(CH_2)_n-C(O)-N(R_{25})-(CH_2)_m-$ with n, $R_{25}$ and m as previously defined and, in particular, a chain $-(CH_2)_n-C(O)-N(R_{25})-(CH_2)_m-$ in which n is 0, $R_{25}$ is an hydrogen atom and m is 3.

In the iridium(III) complex of the invention, $-R_2$ as defined in formula (I) is either a ferrocene or a targeting entity.

A "targeting entity" is taken to mean, within the scope of the present invention, an entity capable of targeting the iridium(III) complex according to the invention to a binding site. The targeting implemented involves molecular recognition between the targeting entity and the binding site. The binding site may be located or produced in an animal or plant organism such as a cell, cell type, tissue, or virus. It may be present on a protein such as an enzyme, a membrane protein such as Ras, a membrane receptor, a membrane transporter, an antibody or it may be present on a polynucleotide.

Advantageously, the targeting entity implemented in the iridium(III) complex of the invention is selected from a protein; a polypeptide; a peptide; a steroid; a cytokine; a neurotransmitter; an antigen; an antibody; an antibody fragment such as a Fab, F(ab')2, Fv, scFv and diabody fragment; a membrane or nuclear receptor; a receptor agonist; a receptor antagonist; a small molecule; a sugar or polysaccharide; a glycan; a leptin; a polynucleotide; an enzyme; an enzyme substrate; an enzyme modulator; an enzyme inhibitor and a growth factor.

The term "polynucleotide" means, within the present invention, a nucleic acid, nucleic acid sequence, nucleic acid sequence, oligonucleotide, polynucleotide sequence, nucleotide sequence, single-stranded DNA, doublestranded DNA, RNA or aptamer. A polynucleotide in the context of the present invention may comprise natural nucleotides and unnatural nucleotides.

In particular, the targeting entity implemented in the iridium(III) complex of the invention targets this iridium(III) complex to a binding site present on or within cancer cells.

More particularly, the targeting entity implemented in the complex of the invention is selected from the group consisting in

- PSMA (for "Prostate Specific Membrane Antigen") inhibitor such as any antibody specifically binding PSMA or the molecule Lys-urea-Glu,

- the cyclic tripeptide Arg-Gly-Asp (RGD) (the integrin $\alpha v\beta 3$ plays a significant role in tumor angiogenesis, and is a receptor for the extracellular matrix proteins with the exposed arginine-glycine-aspartic (RGD) tripeptide sequence),
- Herceptin, humanized antibody specifically binding to the extracellular domain of the human Epidermal growth factor receptor 2 ($HER_2$) which is overexpressed on the cell surface of many human carcinomas, including breast, ovarian, lung and gastric cancers,
- Trastuzumab, another humanized antibody specifically binding to the extracellular domain of the human Epidermal growth factor receptor 2 ($HER_2$),
- antibody specifically binding to the nectin 4 antigen that is a tumorassociated antigen for breast carcinoma,
- biotin (see hereinafter paragraph 111.2 for more information) and
- FTS (for "Farnesyl Thiosalicylic Acid" also known as salirasib that is a RAS inhibitor).

Even more particularly, the targeting entity implemented in the complex of the invention is selected from the group consisting in PSMA inhibitor, biotin and FTS.

In the iridium(III) complex of the invention, the radicals $R_3$ and $R_4$ as defined in formula (I) i.e. the radicals substituting the carbene of the N-heterocyclic carbene-pyridine may be any one of the combinations defined in the below Table 1 with R, R', R", R‴ and R″″, identical or different, representing a hydrogen atom or an alkyl group optionally substituted.

Table 1

| Combination | $R_3$ | $R_4$ |
|---|---|---|
| 1 | H | H |
| 2 | Me | Me |
| 3 | CRR'R" | H, SR‴, NR‴R″″, OR‴ |
| 4 | OR | H, OR' |
| 5 | NR'R" | H, NR‴R″″ |
| 6 | SR | H, SR' |
| 7 | PRR' | H, PR"R‴ |
| 8 | F | H |
| 9 | BRR' | H |
| 10 | H, SR‴, NR‴R″″, OR‴ | CRR'R" |
| 11 | H, OR', | OR |
| 12 | H, NR‴R″″ | NR'R" |
| 13 | H, SR' | SR |
| 14 | H, PR"R‴ | PRR' |
| 15 | H | F |
| 16 | H | BRR' |

As particular examples of radicals CRR'R", one can cite $-CH_3$, $-CH_2CH_3$, - $CH(CH_3)_2$, $-C(CH_3)_3$, $-CH_2(C_2H_5)$, $-CH(CH_3)(C_2H_5)$, $-C(CH_3)_2(C_2H_5)$, $-CH(C_2H_5)_2$, and - $C(C_2H_5)_3$.

As particular examples of radicals SR, SR" or SR"', one can cite SH, $SCH_3$, and $SC_2H_5$.

As particular examples of radicals NR'R" or NR"'R"", one can cite $-NH_2$, - $NHCH_3$, $-N(CH_3)_2$, $-NH(C_2H_5)$, $-N(CH_3)(C_2H_5)$, and $-N(C_2H_5)_2$.

As particular examples of radicals OR, OR' or OR"', one can cite -OH, $OCH_3$, $-OC_2H_5$.

As particular examples of radicals PRR' or PR"R"', one can cite $-PH_2$, - $PHCH_3$, $-P(CH_3)_2$, $-PH(C_2H_5)$, $-P(CH_3)(C_2H_5)$, and $-P(C_2H_5)_2$.

As particular examples of radicals BRR', one can cite $-BH_2$, $-BHCH_3$, - $B(CH_3)_2$, $-BH(C_2H_5)$, $-B(CH_3)(C_2H_5)$, and $-B(C_2H_5)_2$.

In the iridium(III) complex of the invention, the radicals $R_5$, $R_6$, $R_7$, and $R_8$ as defined in formula (I) i.e. the radicals substituting the pyridine of the N-heterocyclic carbene-pyridine may be any one of the combinations defined in

the below Table 2 with R and R', identical or different, representing a hydrogen atom or an alkyl group optionally substituted.

Table 2

| Combination | $R_5$ | $R_6$ | $R_7$ | $R_8$ |
|---|---|---|---|---|
| 20 | H | H | H | H |
| 21 | H | H | H | Me, CN, OMe, $CF_3$, C(O)R, $CH_2OH$, $CHF_2$, NHR, pyridine, COOR, $NR_2$, F, B $(OR)_2$ |
| 22 | H | H | Me, CN, OMe, $CF_3$, C(O)R, $CH_2OR$, $CHF_2$, NHR, pyridine, | H |
| | | | COOR, $NR_2$, F, $CH_2NHR$, B $(OR)_2$, $CH_2CN$, NCO, $NO_2$, CCSiMe3, SMe, C(NR)SR' | |
| 23 | H | Me, Et, CN, OMe, $CF_3$, C(O) R, $CH_2OR$, $CRF_2$, NHR, pyridine, COOR, $NR_2$, F, $CH_2NHR$, $B(OR)_2$, $CH_2CN$, NCO, $NO_2$, CCSiMe3, SMe, C(NR)SR', C(O)NHR | H | H |
| 24 | Me, CN, OMe, $CF_3$, C(O)R, $CH_2OR$, $CHF_2$, NHR, pyridine, COOR, $NR_2$, F, $CH_2NHR$, $B(OR)_2$, $CH_2CN$, NCO, $NO_2$, CCSiMe3, SMe, C(NR)SR' | H | H | H |
| 25 | Me, $NH_2$, C(O)H | H | F | H |
| 26 | $NH_2$, $NO_2$ | H | Me | H |
| 27 | F, | H | COOR | H |
| 28 | Me, CN | H | $NO_2$ | H |
| 29 | C(O)R | H | F, $CF_3$, Ph | H |
| 30 | H | H | $NH_2$, COOR | $NH_2$ |
| 31 | H | H | Me, F, COOR | Me |
| 32 | H | H | C(O)H | F |
| 33 | H | H | OMe, $B(OR)_2$, COOR, C(O)R | OMe |
| 34 | H | H | Me | C(O)R |

(continued)

| Combination | $R_5$ | $R_6$ | $R_7$ | $R_8$ |
|---|---|---|---|---|
| 35 | $NH_2$ | $NH_2$, Me, $CF_3$ | H | H |
| 36 | $CF_3$ | F, COOR | H | H |
| 37 | COOR | NHR | H | H |
| 38 | CN | $B(OR)_2$ | H | H |
| 39 | F | C(O)R | H | H |
| 40 | $NO_2$ | Me | H | H |
| 41 | $NO_2$ | H | H | OMe |
| 42 | COOR | H | H | $CF_3$ |
| 43 | NHR | H | H | $CH_2OR$, COOR |
| 44 | H | Me, COOR | NHR | H |
| 45 | H | Me | $NO_2$ | H |

As particular examples of radicals C(O)R, one can cite -C(O)H, -C(O)$CH_3$, and -C(O)($C_2H_5$).
As particular examples of radicals COOR, one can cite -COOH, -$COOCH_3$, and -$COOC_2H_5$.
As particular examples of radicals $CH_2OR$, one can cite -$CH_2OH$, - $CH_2OCH_3$, and -$CH_2OC_2H_5$.
As particular examples of radicals NHR, one can cite -$NH_2$, -$NHCH_3$, and - NH($C_2H_5$).
As particular examples of radicals $CH_2NHR$, one can cite -$CH_2NH_2$, - $CH_2NHCH_3$, and -$CH_2NH(C_2H_5)$.
As particular examples of radicals C(O)NHR, one can cite -C(O)$NH_2$, - C(O)$NHCH_3$, and -C(O)NH($C_2H_5$).
As particular examples of radicals $B(OR)_2$, one can cite -$B(OH)_2$, - $B(OCH_3)_2$, and -B($OC_2H_5$).
As particular examples of radicals $CRF_2$, one can cite -$CHF_2$, -C($CH_3$)$F_2$, and -C($C_2H_5$)$F_2$.
As particular examples of radicals C(NR)SR', one can cite -C(NH)SH, - C($NCH_3$)SH, -C(NH)$SCH_3$, -C($NCH_3$)$SCH_3$, -C($NC_2H_5$)SH, -C(NH)$SC_2H_5$, -C($NCH_3$)$SC_2H_5$, -C($NC_2H_5$)$SCH_3$ and -C($NC_2H_5$)$SC_2H_5$.
Within the scope of the invention, any combination from the combinations 1 to 16 of Table 1 can be associated to any combination from the combinations 20 to 45 of Table 2. A particular example of such an association is the association of combination 1 of Table 1 with the combination 20 of Table 2 i.e. the case in which $R_3$ = $R_4$ = $R_5$ = $R_6$ = $R_7$ = $R_8$ = H.
In a particular embodiment, the iridium(III) complex according to the invention is of any one of the following formulae (IV), (V), (VI) and (VII):

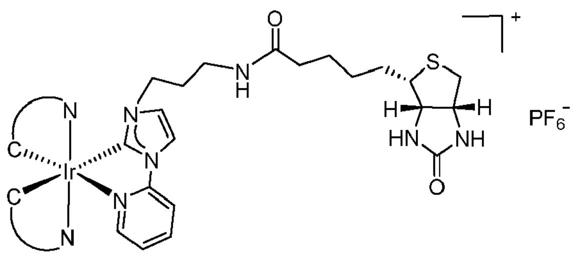

IV

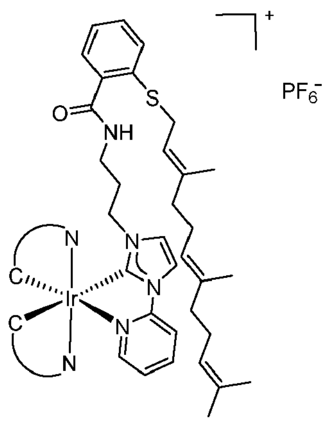

V

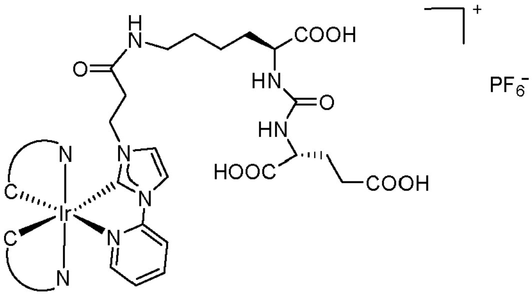

VI

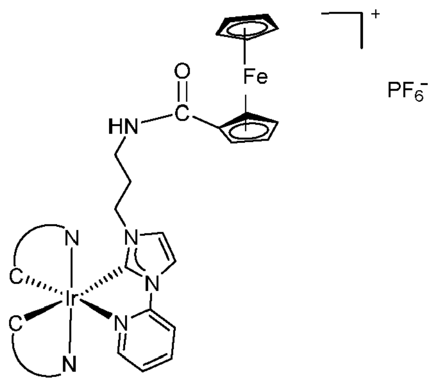

VII

The formula (IV) corresponds to an iridium(III) complex according to the invention in which the targeting entity is biotin.

The formula (V) corresponds to an iridium(III) complex according to the invention in which the targeting entity is FTS.

The formula (VI) corresponds to an iridium(III) complex according to the invention in which the targeting entity is Lys-urea-Glu.

The formula (VII) corresponds to an iridium(III) complex according to the invention comprising a ferrocene.

In these formulae, the ligands C^N may be any ligands C^N such as previously defined.

As particular ligands C^N for the iridium(III) complex of the invention, one can cite the following ligands:

- 2-phenylpyridine (ppy (**a**)) : ligand of formula (II) as previously defined in which $R_9 = R_{10} = R_{11} = R_{12} = R_{13} = R_{14} = R_{15} = R_{16} = H$;
- 2-*p*-tolylpyridine (4-Me-ppy (**b**)) : ligand of formula (II) as previously defined in which $R_9 = R_{10} = R_{11} = R_{12} = R_{13} = R_{14} = R_{16} = H$ and $R_{15} = -CH_3$,
- methyl 4-(pyridin-2-yl)benzoate (4-COOMe-ppy (**c**)) : ligand of formula (II) as previously defined in which $R_9 = R_{10} = R_{11} = R_{12} = R_{13} = R_{14} = R_{16} = H$ and $R_{15} = -COOCH_3$,

- methyl 3-(pyridin-2-yl)benzoate (3-COOMe-ppy (**d**)) : ligand of formula (II) as previously defined in which $R_9 = R_{10} = R_{11} = R_{12} = R_{13} = R_{14} = R_{15}$ = H and $R_{16}$ = $-COOCH_3$,
- methyl 6-phenylnicotinate (*p*-3-COOMe-ppy (**e**)) : ligand of formula (II) as previously defined in which $R_9 = R_{11} = R_{12} = R_{13} = R_{14} = R_{15} = R_{16}$ = H and $R_{10}$ = - $COOCH_3$,
- 6-phenylnicotinic acid (p-3-COOH-ppy (**f**)) : ligand of formula (II) as previously defined in which $R_9 = R_{11} = R_{12} = R_{13} = R_{14} = R_{15} = R_{16}$ = H and $R_{10}$ = - COOH,
- 2-phenylquinoline (pq (**g**)) : ligand of formula (II) as previously defined in which $R_{11} = R_{12} = R_{13} = R_{14} = R_{15} = R_{16}$ = H and $R_9$ and $R_{10}$ form with the carbons to which they are attached a benzene,
- 1-phenylisoquinoline (piq (**h**)) : ligand of formula (II) as previously defined in which $R_9 = R_{10} = R_{13} = R_{14} = R_{15} = R_{16}$ = H and $R_{11}$ and $R_{12}$ form with the carbons to which they are attached a benzene,
- 7,8-benzoquinoline (bzq (**i**)) : ligand of formula (II) as previously defined in which $R_9 = R_{10} = R_{11} = R_{14} = R_{15} = R_{16}$ = H and $R_{12}$ and $R_{13}$ form with the carbons to which they are attached a benzene,
- 2-(2-pyridyl)benzothiophene (thpy (**j**)) : ligand of formula (III) as previously defined in which $(X_1, X_2) = (C\Theta, N)$ and $R_{17} = R_{18} = R_{19} = R_{20} = R_{21} = R_{22} = R_{23} = R_{24}$ = H,
- 2-phenylbenzothiazole (pbt (**k**)) : ligand of formula (III) as previously defined in which $(X_1, X_2) = (N, C\Theta)$ and $R_{17} = R_{18} = R_{19} = R_{20} = R_{21} = R_{22} = R_{23} = R_{24}$ = H, and
- 2-(2,4-difluorophenyl)pyridine (2,4-F-ppy (**l**)) : ligand of formula (II) as previously defined in which $R_9 = R_{10} = R_{11} = R_{12} = R_{14} = R_{16}$ = H and $R_{13} = R_{15}$ = F.

Typically, the iridium(III) complex according to the invention may be any one of the complexes synthetized and tested in the detailed description of particular embodiments.

The present invention relates to an iridium(III) complex according to the present invention for use as a medicament. Such a drug is usable not only in human medicine but also in veterinary medicine. Accordingly, subjects susceptible to receiving such a medicament particularly in the treatment of cancers include animals, including mammals and, in particular, human beings. More particular examples of such subjects include human beings, non-human primates, dogs, cats, horses, cows, goats, pigs, sheep, rabbits, guinea pigs or rodents.

Thus, the present invention relates to a pharmaceutical composition comprising, as active ingredient, an iridium(III) complex according to the invention and a pharmaceutically acceptable vehicle.

By "pharmaceutically acceptable carrier" is meant according to the present invention any substance which is added to an iridium(III) complex according to the present invention to promote its transport, avoid its substantial degradation in said composition and/or increase its half-life. Advantageously, such a pharmaceutically acceptable vehicle is sterile and pyrogen-free. It is selected according to the type of application of the pharmaceutical composition of the invention and in particular according to its mode of administration.

The pharmaceutical composition according to the invention thus consists of at least one iridium(III) complex according to the present invention, in free form or in the form of an addition salt with a pharmaceutically acceptable acid, in the pure state or in the form of a composition in which it is associated with any other pharmaceutically compatible product. The pharmaceutical compositions according to the invention can be used systemically; parenterally, for example intravenously, intra-arterially, intraperitoneally, intrathecally, intraventricularly, intrasternally, intracranially, intramuscularly or subcutaneously; topically; orally; rectally; intranasally or by inhalation.

As solid compositions for oral administration, tablets, pills, powders, etc. may be used in which at least one iridium(III) complex according to the invention is mixed with one or more classically used inert diluents, and optionally with other substances such as, for example, a lubricant, a colorant, a coating, etc.

As liquid compositions for oral or ocular administration, pharmaceutically acceptable suspensions, solutions, emulsions, syrups containing conventionally used inert diluents, and optionally other substances such as wetting agents, sweeteners, thickeners, etc., can be used.

Sterile compositions for parenteral administration can be aqueous or non-aqueous solutions, suspensions or emulsions. As a solvent or vehicle, water, propylene glycol, vegetable oils or other suitable organic solvents may be used. These compositions may also contain adjuvants, such as wetting agents, isotonizing agents, emulsifiers, etc.

The compositions for topical administration may be, for example, creams, lotions, mouthwashes, nasal or eye drops or aerosols.

The person skilled in the art will recognize that the amount of an iridium(III) complex according to the invention to be administered will be an amount that is sufficient to induce an improvement of the undesirable symptoms to be treated. Such an amount may vary depending on such factors as age, weight, general physical condition of the subject, etc., and may be determined on a case-by-case basis. The amount may also vary depending on the other components of a treatment protocol. The one skilled in the art will recognize that these parameters are normally developed during clinical trials. Such an amount is defined hereinafter as "pharmaceutically effective amount". Furthermore, administration of an iridium(III) complex according to the present invention can be done in single or divided doses.

The iridium(III) complex according to the invention is useful as an anticancer agent and more particularly as

anti-cancer agent for photodynamic therapy. The expression "photodynamic therapy" shall mean a treatment for destroying cancerous cells or tissues or cells or tissues suspected to become cancerous through use of a drug that can be activated by light of a certain wavelength and dose. In the present case, the drug is an iridium(III) complex according to the invention.

The present invention thus relates to an iridium(III) complex or a pharmaceutical composition as previously defined for use in the treatment and/or prevention of cancer. Illustrative and non-limiting cancers include melanoma, colorectal cancer, colon cancer, Kaposi's sarcoma, glioblastoma, ovarian cancer, breast cancer, liver cancer, pancreatic cancer, lung cancer, bladder cancer, prostate cancer, or neuroendocrine tumors.

In a particular embodiment, the iridium(III) complex is of formula (IV) as previously defined in which the C^N ligands are either 2-phenylquinolines (pq (**g**)) or 2-phenylbenzothiazoles (pbt (**k**)) and the cancer to be treated and/or prevented is bladder cancer.

In another particular embodiment, the iridium(III) complex is of formula (V) as previously defined in which the C^N ligands are selected in the group consisting of 1-phenylisoquinolines (piq (**h**)), 2-(2-pyridyl)benzothiophenes (thpy (j)) and 2-phenylbenzothiazoles (pbt (**k**)) and the cancer to be treated and/or prevented is bladder cancer.

In another particular embodiment, the iridium(III) complex is of formula (VI) as previously defined in which the C^N ligands are either 1-phenylisoquinolines (piq (**h**)) or 2-phenylbenzothiazoles (pbt (**k**)) and the cancer to be treated and/or prevented is prostate cancer.

In another particular embodiment, the iridium(III) complex is of formula (VII) as previously defined in which the C^N ligands are either 1-phenylisoquinolines (piq (**h**)) or 2-(2-pyridyl)benzothiophenes (thpy (**j**)) and the cancer to be treated and/or prevented is bladder cancer or prostate cancer.

In other words, the present invention relates to a method for treating and/or preventing a cancer as previously defined. This method comprises administering to a subject in need a pharmaceutically effective amount of an iridium(III) complex according to the present invention or a pharmaceutical composition according to the present invention and irradiating the cells or tissue of said subject in need at an appropriate wavelength and dose.

Clearly the cells or tissue irradiated in the treatment or prevention method of the invention are cancerous cells or tissue or cells or tissue suspected to be cancerous.

The appropriate wavelength and dose will depend on the iridium(III) complex implemented and will be selected by the one skilled in the art without inventive step. In particular, this wavelength may be between about 450 nm and 495 nm (blue light) and in particular will be of about 458 nm (i.e. 458 nm $\pm$ 5 nm). The dose may be between 9 W and 10 W and in particular of about 9.6 W (i.e. 9.6 W $\pm$ 0.3 W) with an irradiance between 3 and 6 $mW/cm^2$ and in particular of about 4.5 $mW/cm^2$ (i.e. 4.5 $mW/cm^2 \pm 0.5$ $mW/cm^2$). The duration of illumination may be between 5 min and 30 min and, in particular, of about 10 min (i.e. 10 min $\pm$ 3 min).

Finally, the present invention concerns an iridium(III) complex or a pharmaceutical composition as previously defined for use in diagnosis and, in particular, in photodynamic diagnosis.

In other words, the present invention relates to a method for diagnosing a cancer as previously defined. This method comprises administering to a subject in need a pharmaceutically effective amount of an iridium(III) complex according to the present invention or a pharmaceutical composition according to the present invention and detecting the luminescence *in vivo* i.e.at the level of cells or tissue of the subject in need or *ex vivo* i.e. on cell or tissue biopsy taken from the subject in need.

The particular iridium(III) complexes above disclosed as particular embodiments for treating and/or preventing bladder cancer or prostate cancers are also useful in diagnosis such cancers.

Further features and advantages of the present invention will become apparent to the person skilled in the art from the following illustrative and non-limiting examples, with reference to the attached Figures.

## BRIEF DESCRIPTION OF THE FIGURES

Figure 1 presents plot of the absorbance at 380 nm as a function of light irradiation time in phosphate buffered solution (PBS, pH 7.4) containing **a)** complexes (**I-e**), (**I-g**), (**I-h**), (**I-j**) and (**I-k**), **b)** complexes (**II-e**), (**II-g**), (**II-h**), (**II-j**) and (**II-k**), **c)** complexes (**III-h**), (**III-i**), (**III-j**) and (**III-k**) and **d)** complexes **(IV-h), (IV-i),** (**IV-j**) and (**IV-k**) with ABDA. $Ru(bip\gamma)_3Cl_2$ was used as a reference.

Figure 2A presents the light fluence. Light fluence was quantified at 458 nm, and expressed per unit surface area and per unit time ($mW/min/cm^2$).

Figure 2B presents the effect of blue light (time and distance of irradiation) on cell viability. NIH3T3, T24 and PC3 cell lines were incubated for 72 h without complex according to the invention, with and without irradiation for 5 min or 10 min, at 5 cm or 10 cm of distance. Data are expressed as percentage (%) of cell viability compared to non-irradiated cells (control).

Figure 3 presents the heatmap of $GI_{50}$ values at 458 nm for the series of biotin-vectorized complexes according to the invention.

Figure 4 presents the heatmap of $GI_{50}$ values at 458 nm for the series of salarisib-vectorized complexes according to the invention.

Figure 5 presents the heatmap of $GI_{50}$ values at 458 nm for the series of PSMA inhibitor-vectorized complexes according to the invention.

Figure 6 presents the heatmap of $GI_{50}$ values at 458 nm for the series of ferrocene-vectorized complexes according to the invention.

## DETAILED DESCRIPTION OF PARTICULAR EMBODIMENT

### I. Synthesis of the complexes according to the invention.

#### I.1. General information.

All complexation reactions were performed under an inert atmosphere of dry nitrogen by using standard vacuum line and Schlenk tube techniques. Reactions involving silver compounds were performed with the exclusion of light. 2-(1*H*-imidazol-1-yl)pyridine (**1**) [1], 2,5-dioxopyrrolidin-1-yl-5-((3*αS*,4S,6*αR*)-2-oxohexahydro-1*H*-thieno[3,4-d]imidazol-4-yl)pentanoate (**4**) [2], 2-(((2*E*,6*E*)-3,7,11-trimethyldodeca-2,6,10-trien-1-yl)thio)benzoic acid (**5**) [3], (*S*)-2-[(imidazole-1-carbonyl)amino]pentanedioic acid di-*tert*-butyl ester (**6**) [4], (*S*)-2-[3(*S*)-(5-benzyloxycarbonylamino)-1-*tert*-butyl ester] (**7**) [4], 2-[3-(5-amino-1-*tert*-butoxycarbonylpentyl)ureido]pentanedioic acid di-*tert*-butyl ester (**8**) [4], ferrocenecarboxylic acid (**10**) [5] and dichloro iridium dimers [6,7] were synthesized according modified literature procedures. All other reagents were used as received from commercial suppliers. $^1H$ (300 or 400 MHz) and $^{13}C$ NMR spectra (75 or 101 MHz) and 2D experiments were recorded at 298 K on Bruker AV300, Bruker AV400 or Bruker Avance 600 spectrometers in $CDCl_3$, $CD_3OD$ and DMSO-$d_6$ as solvent. All chemical shifts for $^1H$ and $^{13}C$ are relative to TMS using $^1H$ (residual) or $^{13}C$ chemical shifts of the solvent as a secondary standard. High-Resolution Mass Spectrometry (HRMS) analyses were performed with a Xévo G2 QTOF Waters spectrometer using electrospray ionization (ESI) by the "Service de Spectrométrie de Masse de Chimie UPS-CNRS (Toulouse)". Elemental analyses were carried out by the "Service de Microanalyse du Laboratoire de Chimie de Coordination (Toulouse)".

#### I.2. Synthesis of vectorized proligands (**I**)-(**IV**).

A. Synthesis of substituted imidazoles.

The substituted imidazolium salts 1-(3-aminopropyl)-3-(pyridin-2-yl)-1*H*-imidazol-3-ium bromide hydrobromide (**2**) and 1-(2-carboxyethyl)-3-(pyridin-2-yl)-1*H*-imidazol-3-ium bromide (**3**) were easily obtained by a quaternization step of 2-(1*H*-imidazol-1-yl)pyridine (**1**) [1], with 3-bromopropylamine hydrobromide for (**2**) and 3-bromopropionic acid for (**3**) (below Scheme 1).

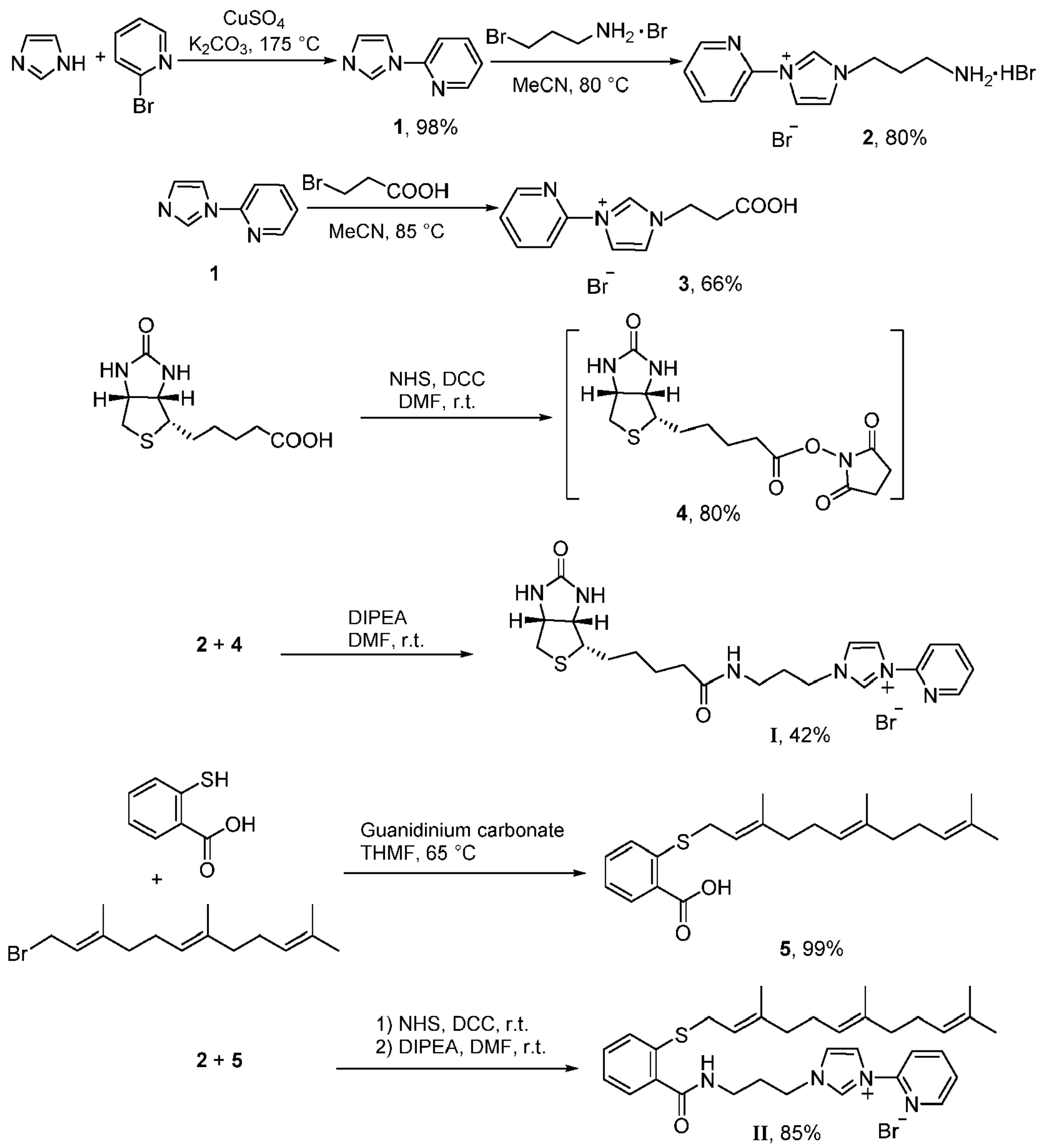


Scheme 1

A.1. *Synthesis of 2-(1H-imidazol-1-yl)pyridine (**1**)*

Imidazole (500 mg, 7.35 mmol), 2-bromopyridine (0.35 mL, 3.68 mmol), $K_2CO_3$ (530 mg, 3.68 mmol) and a catalytic amount of $CuSO_4$ were stirred in a pressure flask at 175°C for 2 days. After cooling down to room temperature, the crude product was extracted by $CH_2Cl_2$, filtered and evaporated to dryness. The resulting residue was purified by column chromatography on silica gel with $CH_2Cl_2$ and MeOH (50:1) as eluent to give a colorless oil (530 mg, 98% yield). $^1$H NMR (300 MHz, $CDCl_3$): $\delta$ 8.49 (ddd, *J* = 4.9, 1.9, 0.9 Hz, 1H), 8.37 (s, 1H), 7.83 (ddd, *J* = 8.2, 7.4, 1.9 Hz, 1H), 7.66 (s, 1H), 7.37 (dd, *J* = 8.2, 0.9 Hz, 1H), 7.29-7.24 (m, 1H), 7.24-7.19 (m, 1H).

A.2. *Synthesis of 1-(3-aminopropyl)-3-(pyridin-2-yl)-1H-imidazol-3-ium bromide hydrobromide (**2**)*

(**1**) (500 mg, 3.43 mmol) and 3-bromopropylamine hydrobromide (750 mg, 3.43 mmol) were dissolved in 5 mL of dry MeCN in a pressure flask. The mixture was stirred at 80°C for 1 day. After cooling down to room temperature, the crude product was filtered and washed by MeOH, the filtrate was evaporated to give a white solid (1 g, 80% yield). Anal. Calcd. For $C_{11}H_{16}Br_2N_4$: C, 36.29; H, 4.43; N, 15.39. Found: C, 35.96; H; 4.62; N, 15.28. $^1$H NMR (400 MHz, DMSO-*d*$_6$): $\delta$ 10.22 (t, *J* = 1.6 Hz, 1H), 8.66 (ddd, *J* = 4.9, 1.9, 0.8 Hz, 1H), 8.59 (dd, *J* = 1.9 Hz, 1H), 8.24

(ddd, *J* = 8.3, 7.5, 1.8 Hz, 1H), 8.13 (dd, *J* = 1.9 Hz, 1H), 8.10 (dd, J = 8.2, 1.0 Hz, 1H), 7.99 (s, 2H), 7.66 (ddd, *J* = 7.5, 4.8, 0.9 Hz, 1H), 4.46 (t, *J* = 6.7 Hz, 2H), 2.96-2.83 (m, 2H), 2.29-2.17 (m, 2H). $^{13}$C NMR (101 MHz, MeOD): $\delta$ 149.3 (1C), 146.6 (1C), 140.2 (1C), 135.0 (1C), 125.1 (1C), 123.3 (1C), 119.5 (1C), 113.9 (1C), 47.6 (1C), 36.3 (1C), 27.6 (1C). HRMS (ES$^+$): calcd. for $C_{11}H_{15}N_4$ 203.1291; found 203.1297 [M-Br$^-$]$^+$.

A.3. *Synthesis of 1-(2-carboxyethyl)-3-(pyridin-2-yl)-1H-imidazol-3-ium bromide (**3**)*

To a mixture of 2-(1H-imidazol-1-yl)pyridine (**1**) (0.5 g, 3.43 mmol) in MeCN was added 3-bromopropionic acid (0.53 g, 3.43 mmol) in a sealed flask and stirred at 85°C for 24 h. After cooling down to room temperature, the solution was filtered out the precipitate and washed with DCM (3 × 10 mL) and diethyl ether (3 × 10 mL), and then dried under vacuum to afford the desired product as a white solid (0.66 g, 66%). Anal. Calcd. For $C_{11}H_{12}BrN_3O_2$: C, 44.31; H, 4.06; N, 14.09. Found: C, 44.19; H, 4.32; N, 14.25. $^1$H NMR (400 MHz, DMSO-*d*$_6$): $\delta$ 12.69 (s, 1H), 10.09 (dd, *J* = 1.7 Hz, 1H), 8.66 (ddd, *J* = 4.8, 1.8, 0.8 Hz, 1H), 8.52 (dd, *J* = 1.9 Hz, 1H), 8.23 (ddd, *J* = 8.2, 7.5, 1.9 Hz, 1H), 8.05-8.01 (m, 2H), 7.66 (ddd, *J* = 7.5, 4.8, 0.9 Hz, 1H), 4.50 (t, *J* = 6.7 Hz, 2H), 3.02 (t, *J* = 6.7 Hz, 2H). $^{13}$C NMR (101 MHz, DMSO-*d*$_6$): $\delta$ 172.1 (1C), 149.7 (1C), 146.8 (1C), 141.1 (1C), 135.9 (1C), 125.7 (1C), 124.3 (1C), 119.5 (1C), 114.7 (1C), 45.9 (1C), 34.0 (1C). HRMS (ES$^+$): calcd. for $C_{11}H_{12}N_3O_2$ 218.0930; found 218.0935 [M-Br$^-$]$^+$.

B. Synthesis of vectorized carbene precursors (**I**) to (**IV**).

B.1. *Synthesis of biotin vectorized proligand (**I**)*

The proligand containing a biotin moiety, 3-(3-(5-((3$\alpha$*S*,4*S*,6$\alpha$*R*)-2-oxohexahydro-1*H*-thieno[3,4-*d*]imidazol-4-yl)pentanamido)propyl)-1-(pyridin-2-ylmethyl)-1*H*-imidazol-3-ium bromide (**I**), was prepared by reacting (**2**) with 2,5-dioxopyrrolidin-1-yl-5-((3$\alpha$*S*,4*S*,6$\alpha$*R*)-2-oxohexahydro-1*H*-thieno[3,4-*d*]imidazol-4-yl)pentanoate (**4**) [2] under the mild alkaline condition of DIPEA (above Scheme 1).

i. *Synthesis of 2,5-dioxopyrrolidin-1-yl-5-((3α5,4S,6αR)-2-oxohexahydro-1H-thieno[3,4-d]imidazol-4-yl)pentanoate (**4**)*

*d*-Biotin (500 mg, 2.05 mmol) and *N*-hydroxysuccinimide (236 mg, 2.05 mmol) were dissolved in 10 mL of hot anhydrous DMF. DCC (550 mg, 2.67 mmol) was added to the flask, the solution was stirred for 12 h at room temperature. The formed dicyclohexylurea was filtered off and the residue was precipitated into diethyl ether, filtered and dried under vacuum to give a white powder (562 mg, 80% yield). $^1$H NMR (400 MHz, DMSO-*d*$_6$): $\delta$6.41 (s, 1H), 6.36 (s, 1H), 4.36-4.25 (m, 1H), 4.15 (m, 1H), 3.15-3.03 (m, 1H), 2.85(*J* = 5.1 Hz, 1H), 2.83-2.78 (m, 4H), 2.67 (t, *J* = 7.3 Hz, 2H), 2.58 (d, *J* = 12.4 Hz, 1H), 1.77-1.30 (m, 6H).

ii. *Synthesis of 3-(3-(5-((3αS,4S,6αR)-2-oxohexahydro-1H-thieno[3,4-d]imidazol-4-yl)pentanamido)propyl)-1-(pyridin-2-ylmethyl)-1H-imidazol-3-ium bromide (**I**)*

(**2**) (746 mg, 2.05 mmol) and (**4**) (700 mg, 2.05 mmol) were dissolved in 10 mL of DMF. Then DIPEA (1.41 mL, 10.25 mmol) was added, the mixture was stirred at room temperature for 12 h. The reaction mixture was concentrated under vacuum and the residue was purified by column chromatography on silica gel with $CH_2Cl_2$ and MeOH (5:1) as eluent to give a yellow oil (439 mg, 42% yield). Anal. Calcd. For $C_{21}H_{29}BrN_6O_2S$: C, 49.51; H, 5.74; N, 16.50. Found: C, 49.46; H; 5.64; N, 16.65. $^1$H NMR (600 MHz, DMSO-*d*$_6$): $\delta$10.20 (s, 1H), 8.65 (d, *J* = 4.8 Hz, 1H), 8.55 (s, 1H), 8.21 (dd, *J* = 7.9, 1.8 Hz, 1H), 8.11 (s, 1H), 8.08 (m, 2H), 7.64 (dd, *J* = 7.5, 4.9 Hz, 1H), 6.40 (s, 1H), 6.37 (s, 1H), 4.35-4.30 (m, 3H), 4.13 (m, 1H), 3.10 (m, 3H), 2.81 (d, *J* = 12.4 Hz, 1H), 2.57 (d, *J* = 12.4 Hz, 1H), 2.09 (t, *J* = 22.3, 7.0 Hz, 2H), 2.05 (m, 2H), 1.51 (m, 2H), 1.61-1.45 (m, 2H), 1.30 (m, 2H). $^{13}$C NMR (151 MHz, DMSO-*d*$_6$): $\delta$ 172.8 (1C), 163.2 (1C), 149.7 (1C), 146.9 (1C), 141.1 (1C), 135.8 (1C), 125.7 (1C), 124.1 (1C), 119.6 (1C), 114.1 (1C), 61.6 (1C), 59.7 (1C), 55.9 (1C), 47.9 (1C), 40.3 (1C), 35.6 (1C), 35.5 (1C), 30.0 (1C), 28.7 (1C), 28.5 (1C), 25.7 (1C). HRMS (ES$^+$): calcd. for $C_{21}H_{29}N_6O_2S$ 429.2067; found 429.2079 [M-Br$^-$]$^+$.

B.2. *Synthesis of FTS vectorized proligand (**II**)*

The proligand containing a *farnesyl* thiosalicylic acid (*FTS*) derivative, 3-(pyridin-2-yl)-1-(3-(2-(((2E,6E)-3,7,11-trimethyldodeca-2,6,10-trien-1-yl)thio)benzamido)propyl) -1*H*-imidazol-3-ium bromide (**II**), was obtained by reaction of 2-(((2E,6E)-3,7,11-trimethyldodeca-2,6,10-trien-1-yl)thio)benzoic acid (**5**) [3] activated by NHS/DCC and (**2**) in the presence of triethylamine (above Scheme 1).

i. *Synthesis of 2-(((2E,6E)-3,7,11-trimethyldodeca-2,6,10-trien-1-yl)thio)benzoic acid (**5**)*

Thiosalicylic acid (300 mg, 1.95 mmol) was dissolved in 3 mL of anhydrous THF under constant stirring and inert atmosphere. Then, guanidinium carbonate (175.7 mg, 1.95 mmol) and *trans,trans*-farnesyl bromide (529 μL, 1.95 mmol) were added. The reaction mixture was stirred under reflux for 8h. After cooling to room temperature, a solution of HCl 1M was added, and the crude product was extracted with ethyl ether (3 × 50 mL). The combined organic layers were dried with sodium sulfate, filtered and evaporated. The product was purified by column chromatography in silica gel with increasing hexane/ethyl acetate gradients to give a grey solid (690 mg, 99% yield). $^1$H NMR (400 MHz, DMSO-$d_6$): $\delta$ 7.86 (dd, *J* = 7.8, 1.6 Hz, 1H), 7.49 (dd, *J* = 7.6, 1.6 Hz, 1H), 7.38 (d, *J* = 8.1 Hz, 1H), 7.20 (dd, *J* = 7.5 Hz, 1H), 5.27 (t, *J* = 7.6 Hz, 1H), 5.05 (m, 2H), 3.57 (d, *J* = 7.6 Hz, 2H), 2.09-1.87 (m, 8H), 1.69 (d, *J* = 1.3 Hz, 3H), 1.63 (d, *J* = 1.5 Hz, 3H), 1.55 (m, 6H).

ii. *Synthesis of 3-(pyridin-2-yl)-1-(3-(2-(((2E,6E)-3,7,11-trimethyldodeca-2,6,10-trien-1-yl)thio)benzamido)propyl)-1H-imidazol-3-ium bromide (**II**)*

(**5**) (700 mg, 1.95 mmol) and N-hydroxysuccinimide (224 mg, 1.95 mmol) were dissolved in 20 mL of hot anhydrous $CH_2Cl_2$. DCC (523 mg, 2.54 mmol) was added and the resulting mixture was stirred for 12 h at room temperature. After filtration of the formed dicyclohexylurea, (**2**) (710 mg, 1.95 mmol) dissolved in 5 mL of DMF and triethylamine (1.41 mL, 10.25 mmol) were added at 0°C. The mixture was stirred at room temperature for 12 h. The solvents were evaporated and the residue was purified by column chromatography on silica gel with $CH_2Cl_2$ and MeOH (10:1) as eluent to give a yellow oil (1.1 g, 85% yield). Anal. Calcd. For $C_{33}H_{43}BrN_4OS$: C, 63.55; H, 6.95; N, 8.98. Found: C, 63.44; H, 6.84; N, 8.88. $^1$H NMR (400 MHz, DMSO-$d_6$): $\delta$ 10.16 (d, *J* = 1.8 Hz, 1H,), 8.66 (dd, *J* = 4.9, 2.1 Hz, 1H), 8.59 (dd, *J* = 7.8, 1.9 Hz, 1H), 8.47 (t, *J* = 5.8 Hz, 1H), 8.23 (td, *J* = 7.8, 1.9 Hz, 1H), 8.13 (dd, *J* = 7.2, 1.9 Hz, 1H), 8.07 (d, *J* = 8.3 Hz, 1H), 7.66 (dd, *J* = 7.5, 4.9 Hz, 1H), 7.46-7.34 (m, 3H), 7.24 (ddd, *J* = 8.5, 5.9, 2.7 Hz, 1H), 5.25 (m, 1H), 5.05 (m, 2H), 4.42 (t, *J* = 6.9 Hz, 2H), 3.60-3.53 (m, 2H), 3.33-3.25 (m, 2H), 2.16 (tt, *J* = 6.6 Hz, 2H), 2.01-1.86 (m, 8H), 1.65-1.57 (m, 6H), 1.57-1.51 (m, 6H). $^{13}$C NMR (101 MHz, DMSO-$d_6$): $\delta$ 168.6 (1C), 150.0 (1C), 147.0 (1C), 141.1 (1C), 140.2 (1C), 137.7 (1C), 136.0 (1C), 135.8 (1C), 135.1 (1C), 131.1 (1C), 130.3 (1C), 129.2 (1C), 128.0 (1C), 125.6 (2C), 124.6 (1C), 124.1 (2C), 119.8 (1C), 119.3 (1C), 114.7 (1C), 47.9 (1C), 39.7 (2C), 36.1 (1C), 31.5 (1C), 29.9 (1C), 26.6 (2C), 26.0 (1C), 18.0 (1C), 16.3 (2C). HRMS (ES$^+$): calcd. for $C_{33}H_{43}N_4OS$ 543.3152; found 543.3166 $[M-Br^-]^+$.

B.3. *Synthesis of PSMA vectorized proligand (**III**)*

The proligand containing a prostate specific membrane antigen (PSMA) inhibitor derivative, 3-((7*S*,11*S*)-7,11-bis(*tert*-butoxycarbonyl)-2,2-dimethyl-4,9,17-trioxo-3-oxa-8,10,16-triazanonadecan-19-yl)-1-(pyridin-2-yl)-1*H*-imidazol-3-ium bromide (**III**) was obtained by reaction of (**8**) [4] activated by NHS/DCC and (**3**) in the presence of triethylamine, followed by deprotection of the acids with trifluoroacetic acid (Scheme 2).

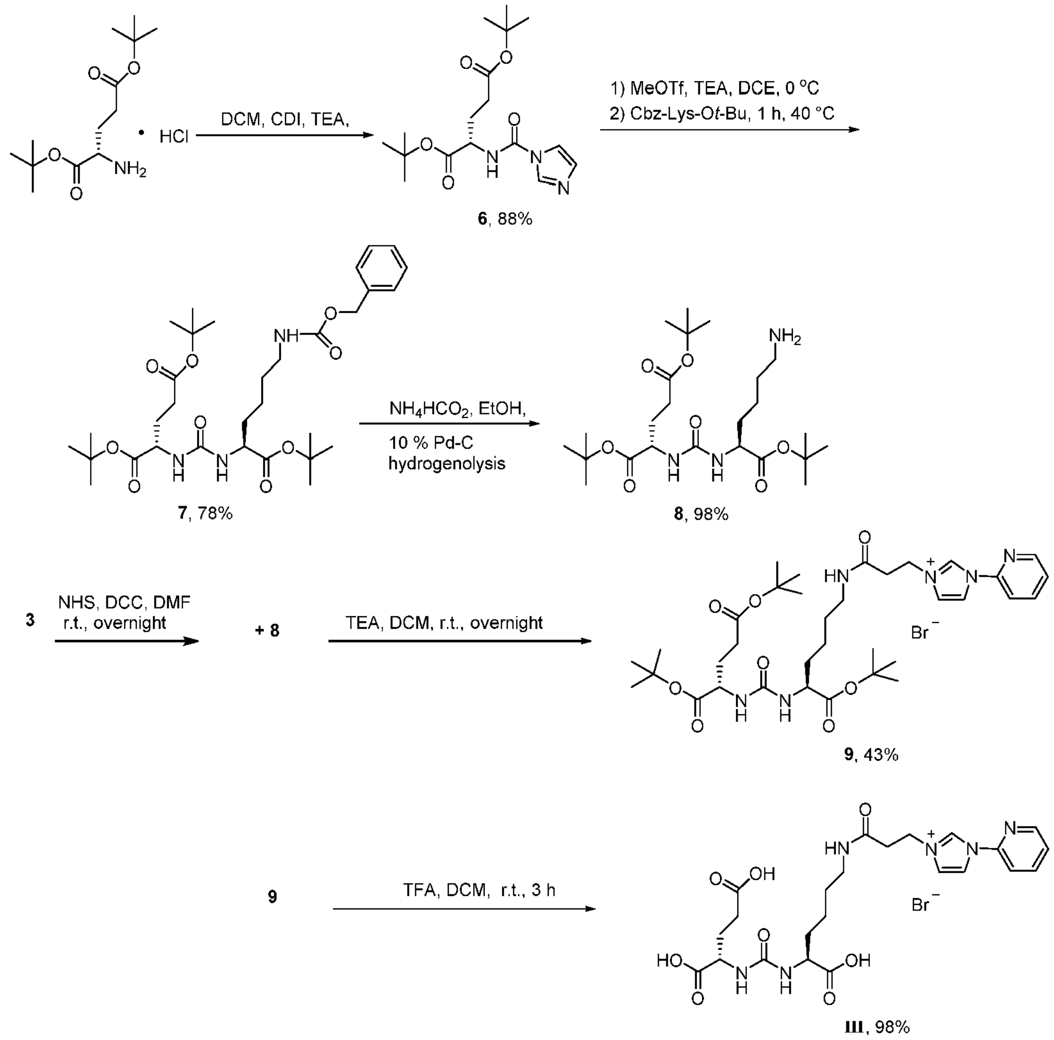


Scheme 2

i. *Synthesis of (S)-2-[(imidozole-1-carbonyl)amino]pentanedioic acid di-tert-butyl ester (**6**)*

To a suspension of *L*-di-*tert*-butyl glutamate hydrochloride (1.0 g, 3.40 mmol) in DCM (10 mL) cooled to 0°C were added TEA (1.5 mL) and DMAP (17 mg). After the mixture was stirred for 5 min, CDI (0.6 g, 3.74 mmol) was added and the mixture was stirred overnight with warming to room temperature. The mixture was diluted with DCM (20 mL) and washed with saturated sodium bicarbonate (60 mL), water (2 × 100 mL), and brine (100 mL). The organic layer was dried over sodium sulfate and concentrated to afford the crude product as semisolid, which slowly solidified upon standing. The crude material was triturated with hexane/ethyl acetate to afford a white solid which was filtered, washed with hexane (100 mL), and dried under vacuum to afford the desired product (1.06 g, 2.99 mmol, 88%). $^1$H NMR (300 MHz, $CDCl_3$): $\delta$ 8.19 (s, 1H), 7.55 (d, *J* = 6.7 Hz, 1H), 7.43 (dd, *J* = 1.5 Hz, 1H), 7.10 (dd, *J* = 1.6, 0.9 Hz, 1H), 4.46 (m, 1H), 2.44 (m, 2H), 2.16 (m, 2H), 1.45 (s, 9H), 1.50 (s, 9H).

*(**7**)* ii. *Synthesis of (S)-2-[3(S)-(5-benzyloxycarbonylamino)-1-tert-butyl ester]*

To a solution of (**6**) (1.0 g, 2.82 mmol) in DCE (10 mL) at 0°C were added MeOTf (0.47 g, 2.85 mmol) and TEA (0.57 g, 5.65 mmol). After the solution was stirred for 30 min, Cbz-Lys-O*t*-Bu (1.06 g, 2.82 mmol) was added in one portion and allowed to stir for 1 h at 40°C. The mixture was concentrated to dryness and purified by column chromatography ($SiO_2$) with $CH_2Cl_2$/MeOH (50:1) as the eluent to afford the desired product as a white solid (1.37 g, 78%). $^1$H NMR (300 MHz, $CDCl_3$): $\delta$ 7.28 (m, 5H), 5.26-5.09 (m, 3H), 5.08 (d, *J* = 7.4 Hz, 2H), 4.26 (m, 2H), 3.09 (m, 2H), 2.22 (m, 2H), 2.08-1.45 (m, 8H), 1.39-1.34 (m, 27H).

iii. *Synthesis of 2-[3-(5-amino-1-tert-butoxycarbonylpentyl)ureido] pentanedioic acid di-tert-butyl ester (**8**)*

To a solution of (**7**) (630 mg, 1.00 mmol) in ethanol (20 mL) was added ammonium formate (630 mg, 10 eqv.) followed by 10% Pd-C, and the suspension was stirred overnight until the hydrogenolysis was complete. The reaction was followed by TLC. The mixture was filtered through a pad of celite and concentrated to dryness to afford the desired product (479 mg, 0.98 mmol, 98%) as a waxy solid. $^1$H NMR (400 MHz, $CDCl_3$): $\delta$ 7.06-5.03 (bm, 4H), 4.33 (m, 2H), 3.02 (m, 2H), 2.33 (m, 2H), 2.16-1.52 (m, 8H), 1.50-1.41 (m, 27H).

iv. *Synthesis of (**9**)*

(**3**) (1.0 g, 3.36 mmol), NHS (0.38 g, 3.36 mmol) and DCC (0.9 g, 4.36 mmol) in DMF (10 mL) were stirred at r.t.. After one night, the solution was filtered and cold diethyl ether (100 mL) was added to the filtrate, affording precipitation. The crude was filtered out and washed with DCM (3 × 10 mL) and cold diethyl ether (3 × 10 mL) to afford the NHS-activated intermediate as a white solid. To a solution of this intermediate, (**8**) (1.5 g, 3.08 mmol) and TEA (2 mL, 14.64 mmol) in DCM (10 mL) were added and the resulting mixture was stirred at r.t.. After one night, the solution was concentrated to dryness and purified by column chromatography ($Al_2O_3$) with $CH_2Cl_2$/MeOH (50:1) as the eluent to afford the desired product as a pale-yellow oil (1.0 g, 43%). Anal. Calcd. For $C_{35}H_{55}BrN_6O_8$: C, 54.75; H, 7.22; N, 10.95. Found: C, 54.53; H, 7.16; N, 10.81. $^1$H NMR (300 MHz, $CDCl_3$): $\delta$ 10.80 (s, 1H), 8.48 (ddd, *J* = 4.8, 1.9, 0.8 Hz, 1H), 8.44-8.38 (m, 1H), 8.35 (dd, *J* = 1.9 Hz, 1H), 8.31 (ddd, *J* = 8.3, 0.9 Hz, 1H), 8.02 (ddd, *J* = 8.3, 7.5, 1.9 Hz, 1H), 7.97-7.94 (m, 1H), 7.41 (ddd, *J* = 7.5, 4.8, 0.8 Hz, 1H), 6.68 (d, *J* = 7.5 Hz, 1H), 6.49 (d, *J* = 8.2 Hz, 1H), 4.76 (t, *J* = 6.5 Hz, 2H), 4.31 (td, *J* = 8.4, 5.0 Hz, 1H), 4.00 (td, *J* = 7.5, 4.6 Hz, 1H), 3.28 (dd, *J* = 6.2 Hz, 2H), 3.21-2.99 (m, 2H), 2.36-2.28 (m, 2H), 2.09-1.93 (m, 2H), 1.80 (dtd, *J* = 13.7, 8.8, 7.0 Hz, 2H), 1.54 (ddd, J = 14.6, 8.2, 4.5 Hz, 2H), 1.38 (s, 9H), 1.37 (s, 9H), 1.35 (s, 9H), 1.20-1.14 (m, 2H). $^{13}$C NMR (101 MHz, $CDCl_3$): $\delta$ 173.3 (1C), 172.5 (1C), 172.4 (1C), 169.0 (1C), 158.1 (1C), 149.0 (1C), 146.0 (1C), 140.9 (1C), 134.7 (1C), 125.2 (1C), 124.2 (1C), 118.9 (1C), 114.7 (1C), 81.3 (1C), 80.7 (1C), 80.3 (1C), 53.5 (1C), 52.9 (1C), 47.8 (1C), 37.8 (1C), 35.5 (1C), 31.9 (1C), 30.4 (1C), 28.2 (1C), 28.1 (3C), 28.0 (6C), 27.9 (1C), 21.4 (1C). ($ES^+$): calcd. for $C_{35}H_{55}N_6O_8$ 687.4081; found 687.4066 $[M-Br^-]^+$.

v. *Synthesis of (**III**)*

To a solution of (**9**) (0.83 g, 1.078 mmol) in DCM (10 mL) cooled to 0°C was added TFA (1 mL). After the mixture was stirred for 5 min, the solution was warmed to room temperature and stirred for 3 h. The solution was concentrated to dryness and washed with DCM (3 × 10 mL) and dried under vacuum to afford the desired product as a pale-yellow oil (0.63 g, 98%). Anal. Calcd. For $C_{23}H_{31}BrN_6O_8$: C, 46.08; H, 5.21; N, 14.02. Found: C, 46.14; H, 5.03; N, 14.74. $^1$H NMR (400 MHz, MeOD): $\delta$ 9.89 (s, 1H), 8,65 (dd, *J* = 4.9, 1.8 Hz, 1H), 8.41 (dd, *J* = 1.9 Hz, 1H), 8.15 (td, *J* = 7.9, 1.8 Hz, 1H), 7.94 (d, *J* = 8.2 Hz, 1H), 7.87 (dd, *J* = 1.9 Hz, 1H), 7.61 (dd, *J* = 7.9, 4.9 Hz, 1H), 4.66 (t, J = 6.3 Hz, 2H), 4.33 (dd, *J* = 8.7, 5.0 Hz, 1H), 4.21 (dd, *J* = 8.6, 4.8 Hz, 1H), 3.23-3.13 (m, 2H), 2.93 (t, *J* = 6.3 Hz, 2H), 2.54-2.32 (m, 2H), 2.24-2.08 (m, 1H), 1.94-1.85 (m, 1H), 1.84-1.75 (m, 1H), 1.64-1.54 (m, 1H), 1.54-1.42 (m, 2H), 1.42-1.30 (m, 2H). $^{13}$C NMR (151 MHz, MeOD): $\delta$ 175.1 (1C), 175.0 (1C), 174.6 (1C), 169.9 (1C), 158.7 (1C), 149.4 (1C), 146.5 (1C), 140.3 (1C), 135.1 (1C), 125.1 (1C), 123.5 (1C), 119.2 (1C), 113.6 (1C), 52.5 (1C), 52.2 (1C), 46.3 (1C), 38.7 (1C), 34.9 (1C), 31.8 (1C), 29.8 (1C), 28.3 (1C), 27.4 (1C), 22.5 (1C). HRMS ($ES^+$): calcd. for $C_{23}H_{31}N_6O_8$ 519.2203; found 519.2216 $[M-Br^-]^+$.

B.4. *Synthesis of ferrocene vectorized proligand (**IV**)*

The proligand containing a ferrocene (Fc) moiety 1-(3-((ferrocenylcarbonyl)amino)propyl)-3-(pyridin-2-yl)-1*H*-imidazol-3-ium bromide (**IV**), was obtained by reaction of ferrocenoyl chloride (obtained from (**10**) [5] and oxalyl chloride) and (**2**) in the presence of triethylamine (Scheme 3).

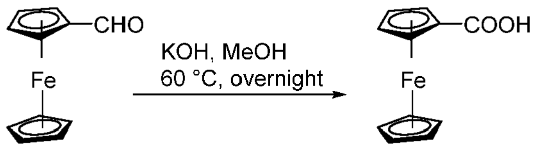


**10**, 47%

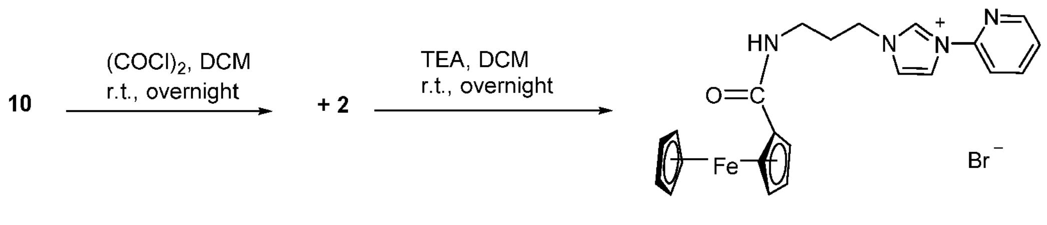


**IV**, 75%

Scheme 3

i. *Synthesis of ferrocenecarboxylic acid (**10**)*

A mixture of ferrocene carboxaldehyde (1.0 g, 4.67 mmol) and KOH (4.0 g, 71 mmol) in MeOH (4 mL) was stirred at 60°C overnight. After cooling down to room temperature, DCM (20 mL) was poured into the crude and the product was extracted with deionized water (3 × 20 mL). The water layer was concentrated to remove MeOH, acidified by HCl (1 M) to pH = 5 in an ice bath, resulting in precipitation. After filtration and drying under vacuum, the desired product was obtained as a yellow solid (0.48 g, 47%). $^1$H NMR (400 MHz, $CDCl_3$): $\delta$4.88 (s, 2H), 4.49 (s, 2H), 4.28 (s, 5H).

ii. *Synthesis of 1-(3-((Ferrocenylcarbonyl)amino)propyl)-3-(pyridin-2-yl)-1H-imidazol-3-ium bromide (**IV**)*

To a solution of (**10**) (1.0 g, 4.3 mmol) in DCM (8 mL) stirred at 0°C for 5 min, was added slowly $(COCl)_2$ (0.74 mL, 8.6 mmoL) in DCM (2 mL) at 0°C. The resulting mixture was stirred overnight at r.t. and dried under vacuum to afford the intermediate ferrocenoyl chloride (FcCOCl), which was then reacted with (**2**) (1.5 g, 4 mmol), and TEA (2.2 mL, 16 mmol) in DCM (10 mL) at r.t.. After one night, the solution was concentrated to dryness and purified by column chromatography on $Al_2O_3$ with $CH_2Cl_2$/MeOH (50:1) as the eluent, yielding a brown waxy solid (1.30 g, 75%). Anal. Calcd. For $C_{22}H_{23}BrFeN_4O$: C, 53.36; H, 4.68; N, 11.31. Found: C, 53.11; H, 4.74; N, 11.28. $^1$H NMR (400 MHz, $CDCl_3$): $\delta$12.09 (s, 1H), 8.53-8.49 (m, 3H), 8.24 (dd, *J* = 1.9 Hz, 1H), 8.13-7.91 (m, 1H), 7.60 (dd, *J* = 1.8 Hz, 1H), 7.47 (dd, *J* = 7.7, 5.1 Hz, 1H), 5.09 (dd, *J* = 2.0 Hz, 2H), 4.74-4.53 (m, 2H), 4.26-4.18 (m, 7H), 3.57-3.48 (m, 2H), 2.50-2.28 (m, 2H). $^{13}$C NMR (101 MHz, $CDCl_3$): $\delta$171.7 (1C), 148.9 (1C), 145.9 (1C), 140.6 (1C), 136.5 (1C), 125.1 (1C), 122.3 (1C), 118.8 (1C), 114.9 (1C), 75.7 (1C), 70.4 (2C), 69.7 (5C), 68.7 (2C), 48.3 (1C), 35.7 (1C), 29.5 (1C). HRMS ($ES^+$): calcd. for $C_{22}H_{23}N_4OFe$ 415.1221; found 415.1223 $[M-Br^-]^+$.

I.3. Synthesis of vectorized iridium(III) complexes.

Commercially available C^N-proligands including 2-phenylpyridine (ppy (**a**)), 2-*p*-tolylpyridine (4-Me-ppy (**b**)), methyl 4-(pyridin-2-yl)benzoate (4-COOMe-ppy (**c**)), methyl 3-(pyridin-2-yl)benzoate (3-COOMe-ppy (**d**)), methyl 6-phenylnicotinate (*p*-3-COOMe-ppy (**e**)), 6-phenylnicotinic acid (*p*-3-COOH-ppy (**f**)), 2-phenylquinoline (pq (**g**)), 1-phenylisoquinoline (piq (**h**)), 7,8-benzoquinoline (bzq (**i**)), 2-(2-pyridyl)benzothiophene (thpy (**j**)), 2-phenylbenzothiazole (pbt (**k**)), and 2-(2,4-difluorophenyl)pyridine (2,4-F-ppy (**l**)) (Schemes 4 and 5) were incorporated into the Ir(III) center of the vectorized iridium complexes, in order to modulate the lipophilic/hydrophilic balance and the excitation wavelengths of the corresponding iridium complexes. The dichloro-bridged dimers $[IrCl(C^\wedge N)_2]_2$ [6,7] were prepared according to the literature (Schemes 4 and 5).

The vectorized iridium(III) complexes (**I-a**) to (**I-l**), (**II-a-e**) to (**II-g-l**), (**III-a-b**), (**III-d-e**) to (**III-g-l**) and **(IV-a)** to (**IV-l**) were prepared by reacting the suitable iridium dimer $[IrCl(C^\wedge N)_2]_2$ with the suitable proligand (**I**), (**II**), (**III**) or (**IV**) in the presence of the mild base $Ag_2O$, followed by an anion exchange with an excess of $KPF_6$ or $NH_4PF_6$ (Schemes 4 and 5). Complex (**I-f**) was a byproduct of (**I-e**).

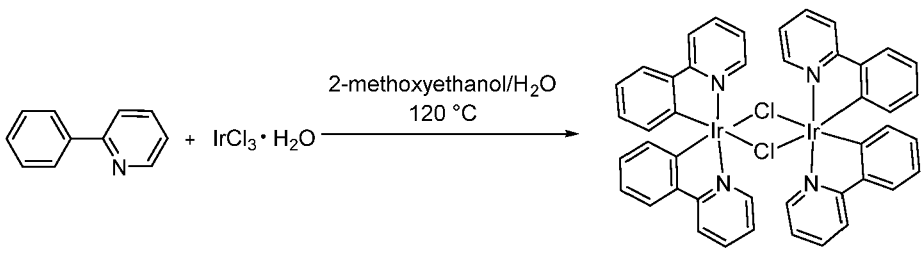


$[(ppy)_2IrCl]_2$

C^N = ppy (**a**)

**I** + $[(ppy)_2IrCl]_2$

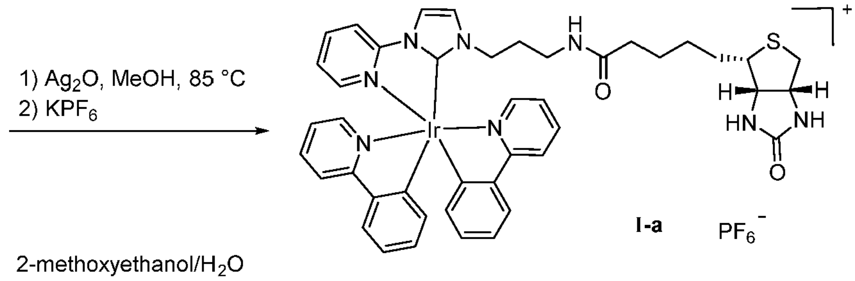


**I-a** $PF_6^-$

H(C^N) + $IrCl_3 \cdot H_2O$ — 2-methoxyethanol/$H_2O$, 120 °C — $[(C^N)_2IrCl]_2$

**I** + $[(C^N)_2IrCl]_2$ — 1) $Ag_2O$, MeOH, 85 °C 2) $KPF_6$ — $[(C^N)Ir(I-H)]PF_6$

**I-a** to **I-l**

**II** + $[(C^N)_2IrCl]_2$ — 1) $Ag_2O$, MeOH, 85 °C 2) $KPF_6$ — $[(C^N)Ir(II-H)]PF_6$

**II-a-e** and **IIg-l**

| C^N = | 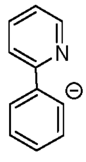 | 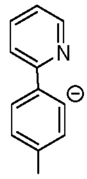 | 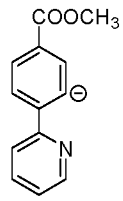 | 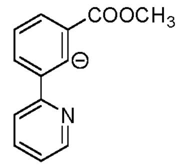 | 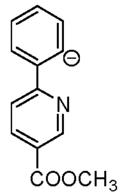 | 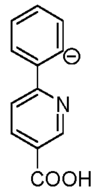 |
|---|---|---|---|---|---|---|
| | ppy (**a**) | 4-Me-ppy (**b**) | 4-COOMe-ppy (**c**) | 3-COOMe-ppy (**d**) | *p*-3-COOMe-ppy (**e**) | *p*-3-COOH-ppy (**f**) |
| **Complex** | **I-a** | **I-b** | **I-c** | **I-d** | **I-e** | **I-f** |
| **Yield** | 56% | 27% | 55% | 17% | 17% | 17% |
| **Complex** | **II-a** | **II-b** | **II-c** | **II-d** | **II-e** | |
| **Yield** | 31% | 52% | 42% | 21% | 38% | |

| C^N = | 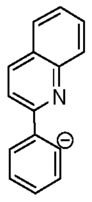 | 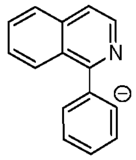 | 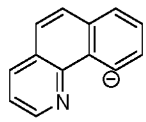 | 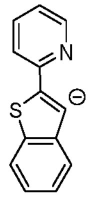 | 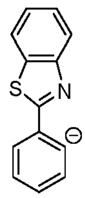 | 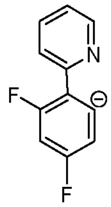 |
|---|---|---|---|---|---|---|
| | pq (**g**) | piq (**h**) | bzq (**i**) | thpy (**j**) | pbt (**k**) | 2,4-F-ppy (**l**) |
| **Complex** | **I-g** | **I-h** | **I-i** | **I-j** | **I-k** | **I-l** |
| **Yield** | 42% | 43% | 40% | 46% | 36% | 45% |
| **Complex** | **II-g** | **II-h** | **II-i** | **II-j** | **II-k** | **II-l** |
| **Yield** | 43% | 41% | 51% | 30% | 31% | 36% |

Scheme 4

**III** + $[(ppy)_2IrCl]_2$

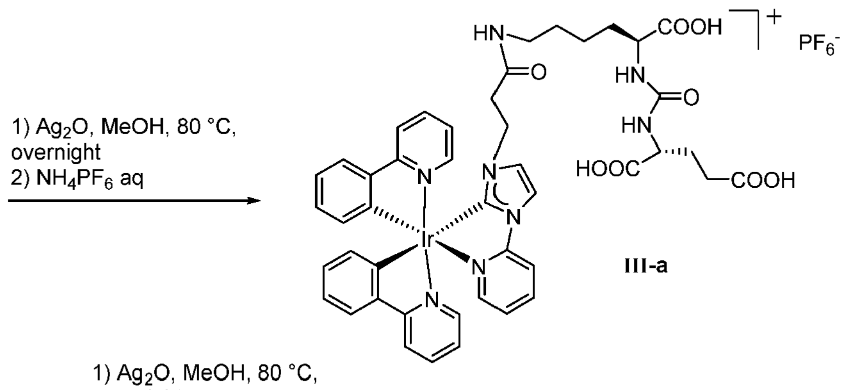


**III-a**

**III** + $[(C\text{^}N)_2IrCl]_2$

1) $Ag_2O$, MeOH, 80 °C, overnight
2) $NH_4PF_6$ aq

$[(C\text{^}N)Ir(III\text{-}H)]PF_6$

**III-a-b**, **IIId-e** and **IIIg-l**

**IV** + $[(C\text{^}N)_2IrCl]_2$

1) $Ag_2O$, $CH_3CN$, 80 °C, overnight
2) $NH_4PF_6$ aq

$[(C\text{^}N)Ir(IV\text{-}H)]PF_6$

**IV-a** to **IV-l**

| | | | | | | |
|---|---|---|---|---|---|---|
| C^N = | 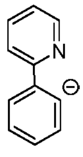 | 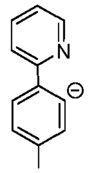 | 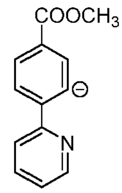 | 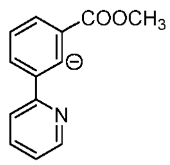 | 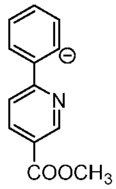 | 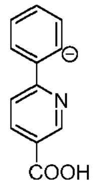 |
| | ppy (**a**) | 4-Me-ppy (**b**) | 4-COOMe-ppy (**c**) | 3-COOMe-ppy (**d**) | *p*-3-COOMe-ppy (**e**) | *p*-3-COOH-ppy (**f**) |
| **Complex** | **III-a** | **III-b** | | **III-d** | **III-e** | |
| **Yield** | 21% | 19% | | 39% | 43% | |
| **Complex** | **IV-a** | **IV-b** | **IV-c** | **IV-d** | **IV-e** | **IV-f** |
| **Yield** | 61% | 63% | 57% | 32% | 40% | 37% |

| | | | | | | |
|---|---|---|---|---|---|---|
| C^N = | 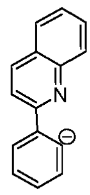 | 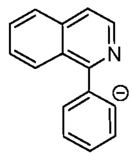 | 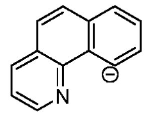 | 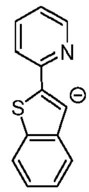 | 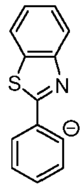 | 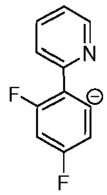 |
| | pq (**g**) | piq (**h**) | bzq (**i**) | thpy (**j**) | pbt (**k**) | 2,4-F-ppy (**l**) |
| **Complex** | **III-g** | **III-h** | **III-i** | **III-j** | **III-k** | **III-l** |
| **Yield** | 40% | 37% | 37% | 46% | 51% | 56% |
| **Complex** | **IV-g** | **IV-h** | **IV-i** | **IV-j** | **IV-k** | **IV-l** |
| **Yield** | 50% | 35% | 31% | 40% | 66% | 56% |

Scheme 5

A. Synthesis of complexes (**Ia**)-(**Ie**), (**Ig**)-(**Il**) and (**IIa**) to (**II-l**).

The iridium(III) dimers $[(C\text{^}N)_2IrCl]_2$were prepared according to literature procedures [6,7].

A.1. *General protocol for the synthesis of complexes (Ia)-(Ie), (Ig)-(Il) and (IIa) to (II-l)*

A mixture of one equivalent of $[(C\text{^}N)_2IrCl]_2$, two equivalents of (**I**) or (**II**) and two equivalents of $Ag_2O$ in MeOH or DMF or MeCN was heated at 85°C overnight. After cooling down, the mixture was filtered through a pad of celite and evaporated to dryness. The resulting residue was purified by column chromatography on aluminium oxide with a mixture of $CH_2Cl_2$ and MeOH as eluent to give a solid. The solid was dissolved in 5 mL MeOH, an aqueous solution of $KPF_6$ (200 mg in 10 mL deionized water) was slowly added under stirring, affording precipitation of the complex. The resulting solid was filtered and dried under vacuum.

A.2. *Complex (**I-a**)*

From $[(ppy)_2IrCl]_2$ (105 mg, 0.1 mmol), (**I**) (100 mg, 0.2 mmol) and $Ag_2O$ (44 mg, 0.2 mmol) in 5 mL of MeOH. Eluent $CH_2Cl_2$ and MeOH (10:1). Yellow solid (119 mg, 56% yield). Anal. Calcd. For $C_{43}H_{44}F_6IrN_8O_2PS$: C, 48.08; H, 4.13; N, 10.43. Found: C,48.27; H; 4.31; N, 10.31. $^1H$ NMR (600 MHz, DMSO-$d_6$): $\delta$ 8.61 (s, 1H), 8.41 (d, $J$ = 8.4 Hz, 1H), 8.28-8.20 (m, 2H), 8.21 (m, 1H), 7.95 (m, 3H), 7.90 (d, $J$ = 8.0 Hz, 2H), 7.75 (s, 1H), 7.71 (s, 1H), 7.65 (d, $J$ = 6.0 Hz, 1H), 7.61 (d, $J$ = 5.6 Hz, 1H), 7.44 (dd, $J$ = 6.1 Hz, 1H), 7.16 (m, 2H), 7.03 (dd, $J$ = 7.1, 4.4 Hz, 1H), 6.94 (m, 2H), 6.83 (dd, $J$ = 7.8 Hz, 1H), 6.42 (s, 1H), 6.38 (s, 1H), 6.28 (d, $J$ = 7.6 Hz, 1H), 6.12 (d, $J$ = 7.4 Hz, 1H), 4.31 (t, $J$ = 6.3 Hz, 1H), 4.12 (t, J = 5.7 Hz, 1H), 3.35-3.46 (m, 2H), 3.08 (s, 1H), 2.85-2.75 (m, 1H), 2.58 (m, 3H), 2.02 (t, $J$ =12.2 Hz, 2H), 1.45-1.60 (s, 2H), 1.47 (m, 2H), 1.29 (m, 2H), 1.17-1.30 (m, 2H). $^{13}C$ NMR (151 MHz, DMSO-$d_6$): $\delta$ 178.1 (1C), 172.5 (1C), 168.3 (1C), 167.0 (1C), 164.1 (1C), 163.2 (1C), 153.7 (1C), 153.6 (1C), 149.6 (1C), 149.4 (1C), 149.4 (1C), 145.0 (1C), 143.4 (1C), 142.2 (1C), 139.0 (1C), 138.3 (1C), 130.9 (2C), 130.8 (1C), 130.1 (1C), 125.4 (2C), 125.0 (1C),

124.8 (2C), 124.0 (1C), 123.3 (1C), 121.8 (1C), 120.7 (1C), 120.4 (1C), 118.9 (1C), 113.4 (1C), 61.5 (1C), 59.7 (1C), 55.9 (1C), 47.6 (1C), 40.5 (1C), 35.8 (1C), 35.5 (1C), 32.0 (1C), 28.7 (1C), 28.5 (1C), 25.7 (1C). HRMS (ES+): calcd. for $C_{43}H_{44}IrN_8O_2S$ 929.2937; found 929.2933 $[M-PF_6^-]^+$.

A.3. *Complex (**I-b**)*

From [(4-Me-ppy)zIrCl]z (199 mg, 0.18 mmol), (**I**) (180 mg, 0.35 mmol) and $Ag_2O$ (81 mg, 0.35 mmol) in 5 mL of MeOH. Eluent $CH_2Cl_2$ and MeOH (10:1). Yellow solid (106 mg, 27% yield). Anal. Calcd. For $C_{45}H_{48}F_6IrN_8O_2PS$: C, 49.04; H, 4.39; N, 10.17. Found: C, 48.85; H; 4.56; N, 10.27. $^1H$ NMR (400 MHz, MeOD): $\delta$ 8.27 (d, *J* = 2.2 Hz, 1H), 8.18-8.05 (m, 3H), 8.01 (d, *J* = 8.1 Hz, 1H), 7.94 (d, *J* = 5.8 Hz, 1H), 7.88-7.77 (m, 3H), 7.70 (dd, *J* = 9.7, 8.0 Hz, 2H), 7.62 (d, *J* = 5.9 Hz, 1H), 7.50 (d, *J* = 2.2 Hz, 1H), 7.28 (ddd, *J* = 7.1, 5.6, 1.5 Hz, 1H), 7.04 (m, 2H), 6.85 (dd, *J* = 8.2, 1.4 Hz, 1H), 6.80 (dd, *J* = 8.1, 1.6 Hz, 1H), 6.25 (s, 1H), 6.06 (s, 1H), 4.53-4.48 (m, 1H), 4.33-4.28 (m, 1H), 3.68-3.48 (m, 2H), 3.24-3.16 (m, 1H), 2.92 (dd, *J* = 12.8 Hz, 5.0 Hz, 1H), 2.73-2.63 (m, 3H), 2.18 (t, *J* = 7.2 Hz, 2H), 2.07 (s, 6H), 1.80-1.70 (m, 1H), 1.70-1.49 (m, 4H), 1.47-1.37 (m, 2H), 1.37-1.37 (m, 1H). $^{13}C$ NMR (101 MHz, MeOD): $\delta$ 179.5 (1C), 174.5 (1C), 169.0 (1C), 167.4 (1C), 164.7 (1C), 163.7 (1C), 153.5 (1C), 152.7 (1C), 149.6 (1C), 149.1 (1C), 148.6 (1C), 141.9 (1C), 141.3 (1C), 140.4 (1C), 140.4 (1C), 140.0 (1C), 137.9 (1C), 137.2 (1C), 131.4 (1C), 131.4 (1C), 124.6 (1C), 124.6 (1C), 123.8 (1C), 123.7 (2C), 123.2 (1C), 122.3 (1C), 122.2 (1C), 119.5 (1C), 119.3 (1C), 117.6 (1C), 112.3 (1C), 62.0 (1C), 60.2 (1C), 55.7 (1C), 47.4 (1C), 39.7 (1C), 35.8 (1C), 35.2 (1C), 31.5 (1C), 28.4 (1C), 28.1 (1C), 25.4 (1C), 20.5 (2C). HRMS (ES+): calcd. for $C_{45}H_{48}IrN_8O_2S$ 957.3290; found 957.3246 $[M-PF_6^-]^+$.

A.4. *Complex (**I-c**)*

From [(4-COOMe-ppy)zIrCl]z (156.5 mg, 0.12 mmol), (**I**) (121 mg, 0.24 mmol) and $Ag_2O$ (55.6 mg, 0.24 mmol) in 5 mL of DMF. Eluent $CH_2Cl_2$ and MeOH (10:1). Yellow solid (156 mg, 55% yield). Anal. Calcd. For $C_{47}H_{48}F_6IrN_8O_6PS$: C, 47.43; H, 4.07; N, 9.41. Found: C, 47.23; H, 4.12; N, 9.38. $^1H$ NMR (400 MHz, MeOD): $\delta$ 8.33 (d, *J* = 2.2 Hz, 1H), 8.30 (d, *J* = 8.3 Hz, 1H), 8.26-8.12 (m, 3H), 8.07 (d, *J* = 5.7 Hz, 1H), 8.04-7.96 (m, 2H), 7.96-7.85 (m, 2H), 7.81-7.74 (m, 2H), 7.64 (dd, *J* = 8.2, 2.4 Hz, 1H), 7.59 (dd, *J* = 8.2, 2.1 Hz, 1H), 7.55 (d, *J* = 2.2 Hz, 1H), 7.33 (ddd, *J* = 7.1, 5.6, 1.2 Hz, 1H), 7.24 (m, 2H), 7.04 (d, *J* = 1.7 Hz, 1H), 6.88 (d, *J* = 1.7 Hz, 1H), 4.64-4.46 (m, 1H), 4.35-4.23 (m, 1H), 3.72 (s, 6H), 3.64-3.49 (m, 2H), 3.21 (m, 1H), 2.93 (ddd, *J* = 12.7, 5.0, 1.5 Hz, 1H), 2.71 (dd, *J* = 12.7, 3.3 Hz, 1H), 2.70-2.54 (m, 2H), 2.16 (t, *J* = 7.3 Hz, 2H), 1.79-1.52 (m, 5H), 1.52-1.39 (m, 2H), 1.34-1.20 (m, 1H). $^{13}C$ NMR (101 MHz, MeOD): $\delta$ 177.9 (1C), 174.5 (1C), 167.3 (2C), 167.1 (1C), 165.9 (1C), 164.6 (1C), 162.3 (1C), 153.5 (1C), 153.4 (1C), 149.7 (1C), 149.4 (1C), 149.3 (1C), 147.8 (1C), 147.7 (1C), 141.7 (1C), 138.6 (1C), 137.9 (1C), 131.3 (2C), 130.7 (1C), 130.3 (1C), 125.2 (1C), 124.5 (1C), 124.4 (1C), 124.3 (2C), 124.1 (1C), 123.9 (1C), 122.7 (1C), 121.2 (1C), 121.0 (1C), 117.9 (1C), 112.7 (1C), 62.0 (1C), 60.2 (1C), 55.7 (1C), 51.0 (2C), 47.4 (1C), 39.7 (1C), 35.7 (1C), 35.2 (1C), 31.4 (1C), 28.3 (1C), 28.1 (1C), 25.4 (1C). HRMS (ES+): calcd. for $C_{47}H_{48}IrN_8O_6S$ 1045.3047; found 1045.3037 $[M-PF_6^-]^+$.

A.5. *Complex (**I-d**)*

From [(3-COOMe-ppy)zIrCl]z (189 mg, 0.15 mmol), (**I**) (146 mg, 0.29 mmol) and $Ag_2O$ (67.2 mg, 0.29 mmol) in 5 mL of DMF. Eluent $CH_2Cl_2$ and MeOH (10:1). Yellow solid (75 mg, 17% yield). Anal. Calcd. For $C_{47}H_{48}F_6IrN_8O_6PS$: C, 47.43; H, 4.07; N, 9.41. Found: C, 47.25; H, 4.04; N, 9.55. $^1H$ NMR (400 MHz, MeOD): $\delta$8.54-8.44 (m, 2H), 8.38-8.30 (m, 2H), 8.24 (d, *J* = 7.9 Hz, 2H), 8.22-8.14 (m, 1H), 8.07-7.93 (m, 3H), 7.77-7.69 (m, 2H), 7.60-7.52 (m, 2H), 7.52-7.43 (m, 1H), 7.36-7.27 (m, 1H), 7.21 (m, 2H), 6.56 (dd, *J* = 8.1, 1.9 Hz, 1H), 6.36 (d, *J* = 7.8 Hz, 1H), 4.51 (dd, *J* = 7.9, 4.9 Hz, 1H), 4.31 (dd, *J* = 7.9, 4.5 Hz, 1H), 3.87-3.82 (m, 6H), 3.65-3.46 (m, 2H), 3.21 (m, 1H), 2.92 (dd, *J* = 12.7, 5.0 Hz, 1H), 2.78-2.57 (m, 3H), 2.15 (t, *J* = 7.2 Hz, 2H), 1.79-1.54 (m, 4H), 1.54-1.25 (m, 4H). $^{13}C$ NMR (101 MHz, MeOD): $\delta$ 177.8 (1C), 174.5 (1C), 171.4 (1C), 167.8 (2C), 167.5 (1C), 166.1 (1C), 164.7 (1C), 158.0 (1C), 153.4 (1C), 153.3 (1C), 149.5 (1C), 149.3 (1C), 145.3 (1C), 143.8 (1C), 141.9 (1C), 138.9 (1C), 138.2 (1C), 131.0 (2C), 130.7 (1C), 130.1 (1C), 125.3 (1C), 125.2 (1C), 125.1 (1C), 124.9 (1C), 124.2 (1C), 124.1 (1C), 123.9 (1C), 123.7 (1C), 120.5 (1C), 120.3 (1C), 118.0 (1C), 112.8 (1C), 62.0 (1C), 60.2 (1C), 55.7 (1C), 51.1 (2C), 47.9 (1C), 39.7 (1C), 35.7 (1C), 35.2 (1C), 31.4 (1C), 28.4 (1C), 28.1 (1C), 25.4 (1C). HRMS (ES+): calcd. for $C_{47}H_{48}IrN_8O_6S$ 1045.3047; found 1045.3040 $[M-PF_6^-]^+$.

A.6. *Complexes (**I-e**) and (**I-f**)*

From [(p-3-COOMe-ppy)zIrCl]z (215 mg, 0.17 mmol), (**I**) (169 mg, 0.33 mmol) and $Ag_2O$ (76.5 mg, 0.33 mmol) in 5 mL of DMF. Eluent $CH_2Cl_2$ and MeOH (10:1) and $CH_2Cl_2$ and MeOH (5:1). Yellow solids ((**I-e**): 133 mg, 17% yield, ((**I-f**): 128 mg, 17%).

(**I-e**): Anal. Calcd. For $C_{47}H_{48}F_6IrN_8O_6PS$: C, 47.43; H, 4.07; N, 9.41. Found: C, 47.34; H; 4.18; N, 9.51. $^1H$ NMR (400 MHz, MeOD): $\delta$ 8.47 (m, 1H), 8.42-8.34 (m, 3H), 8.29-8.19 (m, 4H), 8.15 (m, 1H), 7.99-7.90 (m, 2H), 7.84

(d, *J* = 5.3, 1H), 7.61 (d, *J* = 2.2 Hz, 1H), 7.40 (ddd, *J* = 7.1, 5.6, 1.5 Hz, 1H), 7.10 (dd, *J* = 7.5, 1.3 Hz, 1H), 7.04 (m, 2H), 6.95 (dd, *J* = 7.3, 1.2 Hz, 1H), 6.46 (d, *J* = 7.8 Hz, 1H), 6.28 (dd, *J* = 7.5, 1.3 Hz, 1H), 4.51 (m, 1H), 4.31 (m, 1H), 3.89 (s, 3H), 3.82 (s, 3H), 3.69-3.45 (m, 2H), 3.20 (m, 1H), 2.92 (d, *J* =12.7 Hz, 1H), 2.82-2.66 (m, 3H), 2.17 (t, *J* = 4.3 Hz, 2H), 1.78-1.68 (m, 1H), 1.68-1.52 (m, 4H), 1.52-1.36 (m, 3H). $^{13}$C NMR (101 MHz, MeOD): $\delta$ 178.2 (1C), 174.6 (1C), 172.5 (1C), 171.0 (1C), 164.7 (1C), 164.6 (1C), 163.5 (2C), 154.1 (1C), 153.6 (1C), 150.8 (1C), 150.0 (1C), 143.4 (1C), 142.0 (1C), 141.8 (1C), 138.7 (1C), 138.6 (1C), 137.8 (1C), 131.7 (1C), 131.3 (1C), 131.0 (2C), 126.4 (2C), 125.9 (1C), 125.0 (1C), 124.2 (1C), 124.0 (1C), 123.3 (1C), 121.8 (1C), 119.6 (1C), 119.3 (1C), 118.2 (1C), 112.8 (1C), 62.0 (1C), 60.2 (1C), 55.7 (1C), 52.0 (1C), 51.8 (1C), 47.5 (1C), 39.7 (1C), 35.8 (1C), 35.2 (1C), 31.4 (1C), 28.3 (1C), 28.1 (1C), 25.4 (1C). HRMS (ES$^+$): calcd. for $C_{47}H_{48}IrN_8O_6S$ 1045.3047; found 1045.3060 [M-$PF_6^-$]$^+$.

(**I-f**): Anal. Calcd. For $C_{45}H_{44}F_6IrN_8O_6PS$: C, 46.51; H, 3.82; N, 9.64. Found: C, 46.33; H; 3.90; N, 9.55. $^1$H NMR (400 MHz, MeOD): $\delta$ 8.55 (d, *J* = 1.7 Hz, 1H), 8.37-8.29 (m, 2H), 8.25 (d, *J* = 2.3 Hz, 1H), 8.22 (m, 1H), 8.19-8.06 (m, 4H), 7.86 (ddd, *J* = 8.5, 4.4, 2.8 Hz, 2H), 7.79 (m, 1H), 7.42 (d, *J* = 2.2 Hz, 1H), 7.27 (ddd, *J* = 7.1, 5.6, 1.4 Hz, 1H), 7.04 (dd, *J* = 7.5, 1.3 Hz, 1H), 7.00-6.91 (m, 2H), 6.84 (dd, *J* = 7.4, 1.4 Hz, 1H), 6.42 (dd, *J* = 7.8, 1.2 Hz, 1H), 6.26 (dd, *J* = 7.4, 1.3 Hz, 1H), 4.47 (dd, *J* = 7.9, 4.8 Hz, 1H), 4.28 (ddd, *J* = 7.8, 4.5, 3.1 Hz, 1H), 3.64-3.41 (m, 2H), 3.17 (m, 1H), 2.89 (ddd, *J* = 12.8, 9.9, 5.0 Hz, 1H), 2.83-2.62 (m, 3H), 2.17 (t, *J* = 7.4 Hz, 2H), 1.77-1.50 (m, 6H), 1.40 (m, 2H). $^{13}$C NMR (101 MHz, MeOD): $\delta$ 178.9 (1C), 174.6 (1C), 169.8 (1C), 169.1 (1C), 168.9 (1C), 168.4 (1C), 164.7 (1C), 164.1 (1C), 154.6 (1C), 153.7 (1C), 150.3 (1C), 149.7 (1C), 149.5 (1C), 144.1 (1C), 142.6 (1C), 141.1 (1C), 138.5 (1C), 137.7 (1C), 133.3 (1C), 132.5 (1C), 131.1 (2C), 130.5 (1C), 130.0 (1C), 125.1 (2C), 123.6 (2C), 122.7 (1C), 121.2 (1C), 118.8 (1C), 118.4 (1C), 117.7 (1C), 112.6 (1C), 62.0 (1C), 60.2 (1C), 55.6 (1C), 47.5 (1C), 39.6 (1C), 36.0 (1C), 35.3 (1C), 31.3 (1C), 28.3 (1C), 28.0 (1C), 25.4 (1C). HRMS (ES$^+$): calcd. for $C_{45}H_{44}IrN_8O_6S$ 1017.2812; found 1017.2709 [M-$PF_6^-$]$^+$.

A.7. *Complex (**I-g**)*

From [(pq)zIrCl]z (178.1 mg, 0.14 mmol), (**I**) (143 mg, 0.28 mmol) and $Ag_2O$ (64.9 mg, 0.28 mmol) in 5 mL of MeOH. Eluent $CH_2Cl_2$ and MeOH (20:1). Orange solid (94 mg, 42% yield). Anal. Calcd. For $C_{51}H_{48}F_6IrN_8O_2PS$: C, 52.17; H, 4.12; N, 9.54. Found: C, 52.27; H; 4.36; N, 9.67. $^1$H NMR (400 MHz, MeOD): $\delta$ 8.48 (m, 2H), 8.33-8.29 (m, 2H), 8.18-8.11 (m, 1H), 8.08-8.00 (m, 2H), 7.95-7.89 (m, 2H), 7.85-7.77 (m, 3H), 7.71 (d, *J* = 8.8 Hz, 1H), 7.52-7.35 (m, 4H), 7.23-7.14 (m, 2H), 7.03 (m, 3H), 6.77 (dd, *J* = 7.4, 1.2 Hz, 1H), 6.70 (dd, *J* = 7.0, 1.6 Hz, 1H), 6.63 (dd, *J* = 7.9, 1.3 Hz, 1H), 6.23 (dd, *J* = 7.4, 1.3 Hz, 1H), 4.45 (m, 1H), 4.28 (dd, *J* = 7.9, 3.9 Hz, 1H), 3.67 (m, 1H), 3.47 (ddd, *J* = 13.2, 10.5, 5.6 Hz, 1H), 3.16 (m, 1H), 2.86 (ddd, *J* = 15.7, 12.7, 5.0 Hz, 2H), 2.74 (ddd, *J* = 13.8, 8.1, 6.2 Hz, 1H), 2.65 (d, *J* = 12.7 Hz, 1H), 2.20 (t, *J* = 7.2 Hz, 2H), 1.78-1.50 (m, 5H), 1.42 (m, 2H), 1.23 (m, 1H). $^{13}$C NMR (101 MHz, MeOD): $\delta$ 179.8 (1C), 174.6 (1C), 171.6 (1C), 170.7 (1C), 164.7 (1C), 164.0 (1C), 152.6 (1C), 149.8 (1C),148.3 (1C), 147.0 (2C), 146.8 (1C), 144.8 (1C), 141.4 (1C), 140.2 (1C), 139.5 (1C), 132.5 (2C), 131.0 (1C), 130.7 (2C), 130.6 (1C), 129.2 (2C), 128.0 (2C), 127.1 (1C), 127.0 (1C), 126.6 (2C), 126.3 (1C), 124.5 (1C), 123.7 (1C), 123.5 (1C), 123.3 (1C), 121.6 (1C), 118.0 (1C), 117.7 (1C), 117.5 (1C), 112.1 (1C), 62.0 (1C), 60.2 (1C), 55.7 (1C), 47.5 (1C), 39.7 (1C), 36.0 (1C), 35.3 (1C), 30.7 (1C), 28.4 (1C), 28.1 (1C), 25.4 (1C). HRMS (ES$^+$): calcd. for $C_{51}H_{48}IrN_8O_2S$ 1029.3250; found 1029.3259 [M-$PF_6^-$]$^+$.

A.8. *Complex (**I-h**)*

From [(piq)zIrCl]z (184.5 mg, 0.15 mmol), (**I**) (114 mg, 0.29 mmol) and $Ag_2O$ (67.2 mg, 0.29 mmol) in 5 mL of MeOH. Eluent $CH_2Cl_2$ and MeOH (20:1). Orange solid (147 mg, 43% yield). Anal. Calcd. For $C_{51}H_{48}F_6IrN_8O_2PS$: C, 52.17; H, 4.12; N, 9.54. Found: C, 52.25; H; 4.27; N, 9.69. $^1$H NMR (400 MHz, MeOD): $\delta$ 9.04 (m, 1H), 8.95 (d, *J* = 8.2 Hz, 1H), 8.42 (d, *J* = 8.1 Hz, 1H), 8.36 (d, *J* = 2.2 Hz, 1H), 8.28 (d, *J* = 8.0 Hz, 1H), 8.23 (d, *J* = 8.3 Hz, 1H), 8.14 (ddd, *J* = 8.4, 7.5, 1.7 Hz, 1H), 8.06-7.99 (m, 2H), 7.91-7.74 (m, 5H), 7.60 (m, 1H), 7.55 (dd, *J* = 6.5, 1.2 Hz, 1H), 7.53-7.43 (m, 3H), 7.28 (ddd, *J* = 7.3, 5.7, 1.2 Hz, 1H), 7.12 (m, 2H), 6.94 (dd, *J* = 7.4, 1.2 Hz, 1H), 6.82 (m, 1H), 6.40 (dd, *J* = 7.8, 1.5 Hz, 1H), 6.26 (dd, *J* = 7.5, 1.3 Hz, 1H), 4.47 (m, 1H), 4.25 (ddd, *J* = 11.1, 7.9, 4.5 Hz, 1H), 3.59-3.48 (m, 1H), 3.46-3.36 (m, 1H), 3.21-3.02 (m, 1H), 2.87 (ddd, *J* = 16.6, 12.8, 5.0 Hz, 1H), 2.69 (ddd, *J* = 16.8, 10.7, 4.9 Hz, 3H), 2.16-2.05 (m, 2H), 1.75-1.42 (m, 5H), 1.35 (m, 3H). $^{13}$C NMR (101 MHz, MeOD): $\delta$ 179.9 (1C), 174.5 (1C), 170.1 (1C), 167.8 (1C), 165.6 (1C), 164.6 (1C), 153.5 (1C), 152.5 (1C), 149.4 (1C), 146.0 (1C), 144.7 (1C), 144.4 (1C), 141.4 (1C), 140.8 (1C), 137.0 (1C), 136.6 (1C), 131.7 (1C), 131.4 (1C), 131.3 (1C), 131.2 (1C), 130.7 (1C), 130.4 (2C), 130.0 (1C), 128.7 (2C), 127.4 (1C), 127.2 (1C), 126.9 (1C), 126.3 (3C), 123.8 (2C), 122.4 (1C), 122.1 (1C), 121.2 (1C), 121.0 (1C), 117.8 (1C), 112.5 (1C), 62.0 (1C), 60.2 (1C), 55.7 (1C), 47.5 (1C), 39.6 (1C), 35.8 (1C), 35.2 (1C), 31.4 (1C), 28.5 (1C), 28.1 (1C), 25.4 (1C). HRMS (ES$^+$): calcd. for $C_{51}H_{48}IrN_8O_2S$ 1029.3250; found 1029.3257 [M-$PF_6^-$]$^+$.

A.9. *Complex (**I-i**)*

From [(bzq)zIrCl]z (245.3 mg, 0.21 mmol), (**I**) (216.5 mg, 0.42 mmol) and $Ag_2O$ (97.3 mg, 0.42 mmol) in 5 mL

of MeOH. Eluent $CH_2Cl_2$ and MeOH (20:1). Orange solid (189 mg, 40% yield). Anal. Calcd. For $C_{47}H_{44}F_6IrN_8O_2PS$: C, 50.31; H, 3.95; N, 9.99. Found: C, 50.33; H, 4.02; N, 9.84. $^1H$ NMR (400 MHz, MeOD): $\delta$ 8.47-8.40 (m, 3H), 8.30 (d, *J* = 1.95 Hz, 1H), 8.18 (d, *J* = 8.3 Hz, 1H), 8.11-8.04 (m, 2H), 7.86 (dd, *J* = 8.8, 2.5 Hz, 2H), 7.78 (dd, *J* = 8.9, 1.1 Hz, 1H), 7.76-7.70 (m, 2H), 7.51 (m, 2H), 7.48-7.44 (m, 2H), 7.41 (d, *J* = 7.8 Hz, 1H), 7.18-7.12 (m, 2H), 7.07 (dd, *J* = 7.6, 1.7 Hz, 1H), 6.38 (d, *J* = 7.3 Hz, 1H), 6.25 (d, *J* = 6.91 Hz, 1H), 4.48 (dd, *J* = 7.4, 4.5 Hz, 1H), 4.28 (dd, *J* = 7.9, 4.5 Hz, 1H), 3.56-3.31 (m, 2H), 3.22-3.10 (m, 1H), 2.90 (dt, *J* = 12.8, 5.2 Hz, 1H), 2.72-2.63 (m, 1H), 2.35 (dd, *J* = 13.9, 6.9 Hz, 1H), 2.23 (dd, *J* = 13.9, 7.2 Hz, 1H), 2.10 (t, *J* = 7.0, 2H), 1.77-1.49 (m, 4H), 1.44-1.31 (m, 2H), 1.30-1.17 (m, 1H), 0.83-0.71 (m, 1H). $^{13}C$ NMR (101 MHz, MeOD): $\delta$ 178.2 (1C), 174.3 (1C), 164.7 (1C), 160.8 (1C), 158.1 (1C), 156.9 (1C), 153.9 (1C), 152.1 (1C), 150.1 (1C), 148.1 (1C), 145.4 (1C), 141.4 (1C), 141.0 (1C), 140.1 (1C), 137.2 (1C), 136.3 (1C), 134.6 (1C), 134.4 (1C), 129.8 (1C), 129.7 (1C), 129.6 (1C), 129.2 (1C), 128.4 (1C), 127.9 (1C), 127.6 (1C), 127.3 (1C), 123.8 (2C), 123.6 (1C), 123.5 (1C), 122.6 (1C), 121.8 (1C), 120.7 (1C), 119.1 (1C), 117.7 (1C), 112.4 (1C), 62.0 (1C), 60.2 (1C), 55.7 (1C), 47.6 (1C), 39.7 (1C), 35.5 (1C), 35.2 (1C), 31.2 (1C), 28.4 (1C), 28.1 (1C), 25.4 (1C). HRMS ($ES^+$): calcd. for $C_{47}H_{44}IrN_8O_2S$ 977.2937; found 977.2927 $[M-PF_6^-]^+$.

A.10. *Complex (**I-j**)*

From [(thpy)zIrCl]z (195 mg, 0.15 mmol), (**I**) (153 mg, 0.30 mmol) and $Ag_2O$ (69.5 mg, 0.30 mmol) in 5 mL of MeOH. Eluent $CH_2Cl_2$ and MeOH (20:1). Orange solid (164 mg, 46% yield). Anal. Calcd. For $C_{47}H_{44}F_6IrN_8O_2PS_3$: C, 47.59; H, 3.74; N, 9.45. Found: C, 47.39; H, 3.69; N, 9.34. $^1H$ NMR (400 MHz, MeOD): $\delta$ 8.32 (d, *J* = 2.2 Hz, 1H), 8.18 (d, *J* = 8.5 Hz, 1H), 8.04 (ddd, *J* = 8.5, 7.6, 1.7 Hz, 1H), 7.97-7.84 (m, 3H), 7.82-7.75 (m, 1H), 7.75-7.66 (m, 5H), 7.53 (d, *J* = 2.2 Hz, 1H), 7.18-7.06 (m, 3H), 6.98 (m, 2H), 6.89 (m, 1H), 6.81 (dd, *J* = 8.1, 7.1 Hz, 1H), 6.15 (m, 1H), 5.96 (d, *J* = 8.2 Hz, 1H), 4.48 (dd, *J* = 8.0, 4.8 Hz, 1H), 4.28 (ddd, *J* = 8.0, 4.5, 1.6 Hz, 1H), 3.48 (d, *J* = 7.2 Hz, 2H), 3.18 (m, 1H), 2.91 (dd, *J* = 12.7, 4.8 Hz, 1H), 2.69 (dd, *J* = 12.7, 2.7 Hz, 1H), 2.49-2.31 (m, 2H), 2.11 (t, *J* = 7.2 Hz, 2H), 1.78-1.48 (m, 4H), 1.37 (m, 4H). $^{13}C$ NMR (101 MHz, MeOD): $\delta$ 174.4 (1C), 173.6 (1C), 165.4 (1C), 164.6 (1C), 164.5 (1C), 162.3 (1C), 153.7 (1C), 153.4 (1C), 149.8 (2C), 146.5 (1C), 146.2 (1C), 145.5 (1C), 143.6 (1C), 142.6 (1C), 142.0 (1C), 139.6 (1C), 138.7 (1C), 138.6 (1C), 134.2 (1C), 125.7 (1C), 125.3 (1C), 125.0 (2C), 124.3 (1C), 124.2 (1C), 124.0 (1C), 123.8 (1C), 123.0 (1C), 122.7 (1C), 122.0 (1C), 120.4 (1C), 119.8 (1C), 119.4.0 (1C), 118.1 (1C), 112.7 (1C), 62.0 (1C), 60.2 (1C), 55.7 (1C), 47.6 (1C), 39.7 (1C), 35.6 (1C), 35.1 (1C), 31.4 (1C), 28.4 (1C), 28.1 (1C), 25.4 (1C). HRMS ($ES^+$): calcd. for $C_{47}H_{44}IrN_8O_2S_3$ 1041.2379; found 1041.2366 $[M-PF_6^-]^+$.

A.11. *Complex (**I-k**)*

From [(pbt)zIrCl]z (213.9 mg, 0.17 mmol), (**I**) (168 mg, 0.33 mmol) and $Ag_2O$ (76.5 mg, 0.33 mmol) in 5 mL of MeOH. Eluent $CH_2Cl_2$ and MeOH (20:1). Yellow solid (142 mg, 36% yield). Anal. Calcd. For $C_{47}H_{44}F_6IrN_8O_2PS_3$: C, 47.59; H, 3.74; N, 9.45. Found: C, 47.66; H, 3.75; N, 9.37. $^1H$ NMR (400 MHz, MeOD): $\delta$ 8.42 (d, *J* = 2.2 Hz, 1H), 8.22 (d, *J* = 8.4 Hz, 1H), 8.13 (m, H, 1H), 8.08 (ddd, *J* = 8.2, 3.2, 1.2 Hz, 1H), 8.01 (dd, *J* = 8.0, 1.7 Hz, 1H), 7.90-7.82 (m, 3H), 7.68 (d, *J* = 2.2 Hz, 1H), 7.48-7.34 (m, 3H), 7.30 (ddd, *J* = 7.2, 5.6, 1.2 Hz, 1H), 7.14 (ddd, *J* = 8.5, 7.2, 1.2 Hz, 1H), 7.01 (dd, *J* = 7.5, 1.2 Hz, 1H), 6.95 (dd, *J* = 7.5, 1.0 Hz, 1H), 6.89 (dd, *J* = 7.4, 1.3 Hz, 1H), 6.76 (dd, *J* = 7.5, 1.4 Hz, 1H), 6.44 (ddd, *J* = 8.9, 7.7, 1.7 Hz, 2H), 6.37 (d, *J* = 7.9 Hz, 1H), 6.18 (d, *J* = 8.4 Hz, 1H), 4.46 (dd, *J* = 8.2, 4.1 Hz, 1H), 4.28 (dd, *J* = 7.5, 4.4 Hz, 1H), 3.69-3.51 (m, 2H), 3.17 (m, 1H), 2.86 (ddd, *J* = 12.7, 11.0, 4.9 Hz, 1H), 2.80-2.61 (m, 3H), 2.17 (t, *J* = 7.2 Hz, 2H), 1.79-1.51 (m, 5H), 1.39 (m, 3H). $^{13}C$ NMR (101 MHz, MeOD): $\delta$ 183.2 (1C), 180.0 (1C), 175.6 (1C), 174.5 (1C), 164.6 (1C), 163.7 (1C), 153.9 (1C), 150.0 (1C), 149.7 (1C), 149.3 (1C), 149.0 (1C), 142.1 (1C), 140.8 (1C), 139.3 (1C), 132.3 (1C), 132.1 (1C), 132.0 (1C), 131.5 (2C), 128.0 (1C), 127.7 (1C), 126.5 (1C), 126.2 (2C), 126.0 (1C), 124.3 (1C), 124.1 (1C), 123.9 (2C), 123.6 (1C), 122.2 (1C), 118.0 (1C), 118,0 (2C), 117.2 (1C), 112.5 (1C), 62.0 (1C), 60.2 (1C), 55.8 (1C), 47.6 (1C), 39.8 (1C), 36.0 (1C), 35.2 (1C), 31.2 (1C), 28.4 (1C), 28.1 (1C), 25.4 (1C). HRMS ($ES^+$): calcd. for $C_{47}H_{44}IrN_8O_2S_3$ 1041.2379; found 1041.2385 $[M-PF_6^-]^+$.

A.12. *Complex (**I-l**)*

From [(2,4-F-ppy)zIrCl]z (218.9 mg, 0.18 mmol), (**I**) (184.5 mg, 0.36 mmol) and $Ag_2O$ (83.4 mg, 0.36 mmol) in 5 mL of MeOH. Eluent $CH_2Cl_2$ and MeOH (20:1). Yellow solid (187 mg, 45% yield). Anal. Calcd. For $C_{43}H_{40}F_{10}IrN_8O_2PS$: C, 45.06; H, 3.52; N, 9.78. Found: C, 45.17; H, 3.63; N, 9.68. $^1H$ NMR (400 MHz, MeOD): $\delta$ 8.43-8.38 (m, 2H, H), 8.36-8.28 (m, 2H), 8.23 (dd, *J* = 8.4, 7.9 Hz, 1H), 8.07-8.02 (m, 1H), 8.01-7.92 (m, 2H), 7.84-7.80 (m, 1H), 7.77-7.71 (m, 1H), 7.64 (dd, *J* = 6.5, 1.9 Hz, 1H), 7.41 (ddd, *J* = 7.1, 5.6, 1.2 Hz, 1H), 7.19 (m, 2H), 6.62 (m, 2H), 5.84 (dd, *J* = 8.7, 2.4 Hz, 1H), 5.68 (dd, *J* = 7.9, 2.3 Hz, 1H), 4.49 (dd, *J* = 8.2, 4.1 Hz, 1H), 4.32 (td, *J* = 7.5, 4.4 Hz, 1H), 3.73-3.60 (m, 2H), 3.21 (m, 1H), 2.93 (ddd, *J* = 12.7, 11.0, 4.9 Hz, 1H), 2.82 (m, 2H), 2.71 (dd, *J* = 12.8, 3.9 Hz, 1H), 2.20 (t, *J* = 7.2 Hz, 2H), 1.81-1.36 (m, 5H), 1.39 (m, 3H). $^{13}C$ NMR (101 MHz, MeOD): $\delta$ 176.4 (1C), 174.6 (1C), 167.4 (1C), 164.4 (dd, $J_{C-F}$ = 230, 11 Hz, 1C), 164.8 (d, $J_{C-F}$ = 7 Hz, 1C), 164.7 (1C), 163.6 (d, $J_{C-F}$ = 7 Hz, 1C), 163.5 (dd, $J_{C-F}$ = 235, 12 Hz,

1C), 161.8 (dd, $J_{C-F}$ = 239, 11 Hz, 1C), 161.5 (dd, $J_{C-F}$ = 240, 12 Hz, 1C), 153.6 (1C), 153.4 (1C), 153.0 (d, $J_{C-F}$ = 7 Hz, 1C), 149.6 (1C), 149.4 (1C), 142.1 (1C), 139.2 (1C), 138.6 (1C), 127.9 (1C), 127.1 (1C), 124.5 (1C), 124.4 (1C), 124.2 (1C), 123.5 (2C), 123.5 (1C), 118.2 (1C), 113.0 (1C), 112.7 (d, $J_{C-F}$ = 21 Hz, 1C), 112.5 (d, $J_{C-F}$ = 21 Hz, 1C), 99.0 (t, $J_{C-F}$ = 27 Hz, 1C), 97.5 (t, $J_{C-F}$ = 27 Hz, 1C), 62.0 (2C), 60.2 (1C), 55.7 (1C), 47.7 (1C), 39.8 (1C), 35.8 (1C), 35.3 (1C), 31.4 (1C), 28.4 (1C), 28.1 (1C), 25.4 (1C). HRMS ($ES^+$): calcd. for $C_{43}H_{40}F_4IrN_8O_2S$ 1001.2560; found 1101.2551 $[M-PF_6^-]^+$.

A.13. *Complex (**II-a**)*

From [(ppy)zIrCl]z (172 mg, 0.16 mmol), (**II**) (200 mg, 0.32 mmol) and $Ag_2O$ (74 mg, 0.32 mmol) in 5 mL of MeCN. Eluent $CH_2Cl_2$ and MeOH (10:1). Yellow solid (116 mg, 31% yield). Anal. Calcd. For $C_{55}H_{58}F_6IrN_6OPS$: C, 55.59; H, 4.92; N, 7.07. Found: C, 55.52; H, 4.81; N, 7.08. $^1$H NMR (400 MHz, $CDCl_3$): $\delta$ 8.04-7.86 (m, 6H), 7.80-7.72 (m, 2H), 7.71-7.66 (m, 3H), 7.55-7.46 (m, 2H), 7.42-7.28 (m, 2H), 7.28-7.19 (m, 2H), 7.16 (ddd, *J* = 7.1, 5.6, 1.4 Hz, 1H), 7.09 (ddd, *J* = 7.4, 5.8, 1.5 Hz, 1H), 7.08-6.98 (m, 2H), 6.95 (dd, *J* = 7.3, 1.3 Hz, 1H), 6.85 (dd, *J* = 7.5, 1.3 Hz, 1H), 6.76 (dd, *J* = 7.4, 1.4 Hz, 1H), 6.43 (t, *J* = 6.0 Hz, 1H), 6.39 (dd, *J* = 7.7, 1.2 Hz, 1H), 6.22 (dd, *J* = 7.4, 1.3 Hz, 1H), 5.33-5.22 (m, 1H), 5.09 (m, 2H), 3.67-3.51 (m, 4H), 2.97 (m, 2H), 2.06 (m, 4H), 1.99 (m, 4H), 1.69 (m, 4H), 1.67-1.54 (m, 9H), 1.47-1.33 (m, 1H). $^{13}$C NMR (101 MHz, $CDCl_3$) $\delta$ 179.0 (1C), 168.6 (1C), 168.3 (1C), 167.3 (1C),163.2 (1C), 153.3 (2C), 149.8 (1C), 148.7 (2C), 144.3 (1C), 142.6 (1C), 141.7 (1C), 141.0 (1C), 138.0 (1C), 137.3 (1C), 136.5 (1C,), 135.4 (1C), 135.0 (1C), 131.1 (1C), 131.1 (2C), 130.9 (1C), 130.6 (1C), 130.4 (2C), 128.7 (1C), 126.3 (1C), 124.8 (2C), 124.3 (2C), 124.0 (2C), 123.7 (1C), 123.1 (2C), 121.6 (1C), 119.8 (2C), 118.5 (1C), 117.9 (1C), 112.8 (1C), 48.1 (1C), 39.7 (2C), 36.7 (1C), 32.6 (1C), 31.8 (1C), 26.6 (2C), 25.7 (1C), 17.7 (1C), 16.1 (2C). HRMS ($ES^+$): calcd. for $C_{55}H_{58}IrN_6OS$ 1043.4022; found 1043.4005 $[M-PF_6^-]^+$.

A.14. *Complex (**II-b**)*

From [(4-Me-ppy)zIrCl]z (90 mg, 0.08 mmol), (**II**) (100 mg, 0.16 mmol) and $Ag_2O$ (37 mg, 0.16 mmol) in 5 mL of MeCN. Eluent $CH_2Cl_2$ and MeOH (30:1). Yellow solid (101 mg, 52% yield). Anal. Calcd. For $C_{57}H_{62}F_6IrN_6OPS$: C, 56.28; H, 5.14; N, 6.91. Found: C, 56.13; H, 5.21; N, 7.05. $^1$H NMR (400 MHz, $CDCl_3$): $\delta$ 8.00 (ddd, *J* = 8.0, 7.4, 1.8 Hz, 1H), 7.96-7.90 (m, 3H), 7.89-7.80 (m, 2H), 7.72 (m, 3H), 7.57 (m, 2H), 7.53 (dd, *J* = 7.7, 1.5 Hz, 1H), 7.48 (dd, *J* = 5.9, 1.5 Hz, 1H), 7.40-7.30 (m, 2H), 7.27-7.20 (m, 2H), 7.17 (ddd, *J* = 7.0, 5.6, 1.3 Hz, 1H), 7.03 (ddd, *J* = 7.3, 5.8, 1.5 Hz, 1H), 6.96 (ddd, *J* = 7.3, 5.8, 1.4 Hz, 1H), 6.84 (dd, *J* = 8.0, 1.7 Hz, 1H), 6.70 (dd, *J* = 8.1, 1.7 Hz, 1H), 6.48 (t, *J* = 6.0 Hz, 1H), 6.21 (d, *J* = 1.7 Hz, 1H), 6.02 (d, *J* = 1.7 Hz, 1H), 5.33-5.17 (m, 1H), 5.10 (m, 2H), 3.55 (m, 4H), 3.00 (m, 2H), 2.08 (m, 6H), 2.04-1.89 (m, 8H), 1.69 (d, *J* = 1.5 Hz, 3H), 1.65-1.52 (m, 10H), 1.43 (m, 1H). $^{13}$C NMR (101 MHz, $CDCl_3$): $\delta$ 179.2 (1C), 168.6 (1C), 168.4 (1C), 167.4 (1C), 163.5 (1C), 153.2 (1C), 153.0 (1C), 149.8 (1C), 148.9 (1C), 148.6 (1C), 141.6 (2C), 140.9 (2C), 140.5 (1C), 140.1 (1C), 137.9 (1C), 137.1 (1C), 136.5 (1C), 135.4 (1C), 135.0 (1C), 131.9 (1C), 131.7 (1C), 131.4 (1C), 130.4 (2C), 128.6 (1C), 126.2 (1C), 124.8 (2C), 124.3 (1C), 124.2 (1C), 123.9 (2C), 123.8 (2C), 122.9 (1C), 122.4 (1C), 119.5 (2C), 118.5 (1C), 117.8 (1C), 112.6 (1C), 48.0 (1C), 39.7 (2C), 36.7 (1C), 32.6 (1C), 31.8 (1C), 26.8 (2C), 25.7 (1C), 21.9 (2C), 17.7 (1C), 16.11 (2C). HRMS ($ES^+$): calcd. for $C_{57}H_{62}IrN_6OS$ 1071.4335; found 1071.4344 $[M-PF_6^-]^+$.

A.15. *Complex (**II-c**)*

From [(4-COOMe-ppy)zIrCl]z (137 mg, 0.11 mmol), (**II**) (130 mg, 0.21 mmol) and $Ag_2O$ (48.7 mg, 0.21 mmol) in 5 mL of MeCN. Eluent $CH_2Cl_2$ and MeOH (20:1). Orange solid (115 mg, 42% yield). Anal. Calcd. For $C_{59}H_{62}F_6IrN_6O_5PS$: C, 54.33; H, 4.79; N, 6.44. Found: C, 54.18; H, 4.77; N, 6.23. $^1$H NMR (400 MHz, MeOD): $\delta$ 8.35 (d, *J* = 2.2 Hz, 1H), 8.24 (m, 2H), 8.19-8.07 (m, 3H), 7.95 (m, 2H), 7.88 (d, *J* = 8.2 Hz, 1H), 7.79 (m, 3H), 7.55 (m, 2H), 7.49 (dd, *J* = 8.2, 1.7 Hz, 1H), 7.43 (dd, *J* = 7.9, 1.3 Hz, 1H), 7.34 (m, 3H), 7.24 (m, 3H), 7.08 (d, *J* = 1.7 Hz, 1H), 6.91 (d, *J* = 1.7 Hz, 1H), 5.25 (t, *J* = 6.4 Hz, 1H), 5.07 (m, 2H), 3.66 (m, 8H), 3.55 (d, *J* = 7.7 Hz, 2H), 2.84 (m, 2H), 2.09-1.88 (m, 8H), 1.65 (d, *J* = 1.5 Hz, 3H), 1.62-1.51 (m, 10H), 1.42 (m, 1H). $^{13}$C NMR (101 MHz, MeOD): $\delta$ 177.9 (1C), 170.0 (1C), 167.3 (2C), 167.0 (1C), 165.9 (1C), 162.3 (1C), 153.5 (2C), 149.7 (1C), 149.4 (2C), 147.8 (2C), 141.8 (1C), 140.2 (1C), 138.7 (1C), 138.0 (1C), 137.7 (1C), 134.9 (2C), 131.3 (2C), 130.8 (1C), 130.3 (1C), 130.1 (1C), 129.9 (1C), 127.2 (1C), 125.7 (1C), 125.3 (1C), 124.6 (1C), 124.5 (1C), 124.4 (2C), 124.1 (1C), 124.0 (2C), 123.7 (2C), 122.8 (1C), 121.3 (1C), 121.1 (1C), 118.9 (1C), 118.0 (1C), 112.8 (1C), 51.2 (2C), 48.1 (1C), 39.4 (1C), 39.3 (1C), 36.2 (1C), 32.0 (1C), 31.5 (1C), 26.4 (1C), 26.0 (1C), 24.6 (1C), 16.5 (1C), 15.0 (2C). HRMS ($ES^+$): calcd. for $C_{59}H_{62}IrN_6O_5S$ 1159.4123; found 1159.4130 $[M-PF_6^-]^+$.

A.16. *Complex (**II-d**)*

From [(3-COOMe-ppy)zlrCl]z (137 mg, 0.11 mmol), (**II**) (130 mg, 0.21 mmol) and $Ag_2O$ (48.7 mg, 0.21 mmol) in 5 mL of MeCN. Eluent $CH_2Cl_2$ and MeOH (30:1). Yellow solid was filtered and dried under vacuum (57.2 mg, 21% yield). Anal. Calcd. For $C_{59}H_{62}F_6IrN_6O_5PS$: C, 54.33; H, 4.79; N, 6.44. Found: C, 54.59; H, 4.60; N, 6.35. $^1H$ NMR (400 MHz, $CDCl_3$): $\delta$ 8.43-8.33 (m, 2H), 8.18-7.96 (m, 6H), 7.85 (m, 2H), 7.66-7.61 (m, 1H), 7.58 (m, 2H), 7.51 (dd, *J* = 7.6, 1.4 Hz, 1H), 7.45 (dd, *J* = 8.1, 1.8 Hz, 1H), 7.40-7.30 (m, 3H), 7.22 (m, 3H), 7.14 (ddd, *J* = 7.4, 5.8, 1.4 Hz, 1H), 6.64 (m, 1H), 6.49 (d, *J* = 8.1 Hz, 1H), 6.29 (d, *J* = 7.8 Hz, 1H), 5.31-5.16 (m, 1H), 5.13-4.97 (m, 2H), 3.88 (s, 3H), 3.84 (s, 3H), 3.60 (m, 2H), 3.51 (d, *J* = 7.6 Hz, 2H), 2.97 (m, 2H), 2.00 (m, 8H), 1.68 (d, *J* = 1.5 Hz, 3H), 1.62 (m, 10H), 1.51-1.40 (m, 1H). $^{13}C$ NMR (101 MHz, $CDCl_3$): $\delta$ 177.7 (1C), 171.0 (1C), 168.4 (1C), 167.5 (1C), 167.4 (2C), 166.1 (1C), 157.5 (1C), 153.6 (1C), 153.2 (1C), 149.5 (1C), 149.1 (1C), 144.8 (1C), 143.2 (1C), 142.2 (1C), 140.9 (1C), 138.6 (1C), 138.0 (1C), 136.2 (1C), 135.4 (1C), 134.9 (1C), 131.4 (2C), 131.2 (2C), 130.8 (1C), 130.6 (1C), 130.5 (1C), 128.6 (1C), 126.3 (1C), 125.5 (2C), 125.3 (1C), 124.3 (4C), 124.0 (2C), 123.7 (1C), 120.3 (2C), 118.5 (1C), 118.2 (1C), 113.3 (1C), 52.0 (2C), 48.1 (1C), 40.0 (2C), 36.6 (1C), 32.5 (1C), 31.8 (1C), 26.6 (2C), 25.7 (1C), 17.7 (1C), 16.1 (2C). HRMS ($ES^+$): calcd. for $C_{59}H_{62}IrN_6O_5S$ 1159.4123; found 1159.4109 $[M-PF_6^-]^+$.

A.17. *Complex (**II-e**)*

From [(p-3-COOMe-ppy$_2$IrCl]$_2$ (137 mg, 0.11 mmol), (**II**) (130 mg, 0.21 mmol) and $Ag_2O$ (48.7 mg, 0.21 mmol) in 5 mL of MeCN. Eluent $CH_2Cl_2$ and MeOH (30:1). Orange solid (105 mg, 38% yield). Anal. Calcd. For $C_{59}H_{62}F_6IrN_6O_5PS$: C, 54.33; H, 4.79; N, 6.44. Found: C, 54.19; H, 4.90; N, 6.53. $^1H$ NMR (400 MHz, MeOD): $\delta$ 8.51 (d, *J* = 1.9 Hz, 1H), 8.42-8.30 (m, 3H), 8.28 (d, *J* = 8.5 Hz, 2H), 8.22 (m, 2H), 8.16 (d, *J* = 1.8 Hz, 1H), 7.93 (m, 2H), 7.84 (dd, *J* = 5.6, 1.6 Hz, 1H), 7.64 (d, *J* = 2.2 Hz, 1H), 7.45 (dd, *J* = 7.9, 1.2 Hz, 1H), 7.42-7.31 (m, 3H), 7.26 (dd, *J* = 7.4, 1.2 Hz, 1H), 7.08 (dd, *J* = 7.5, 1.3 Hz, 1H), 7.01 (m, 2H), 6.90 (dd, *J* = 7.5, 1.4 Hz, 1H), 6.48 (dd, *J* = 7.7, 1.2 Hz, 1H), 6.29 (dd, *J* = 7.4, 1.3 Hz, 1H), 5.23 (m, 1H), 5.05 (m, 2H), 3.83 (m, 6H), 3.78-3.69 (m, 1H), 3.73-3.56 (m, 1H), 3.56 (d, *J* = 7.6 Hz, 2H), 2.99 (m, 2H), 1.97 (m, 8H), 1.74 (m, 1H), 1.64 (d, *J* = 1.5 Hz, 3H), 1.55 (m, 10H). $^{13}C$ NMR (101 MHz, MeOD): $\delta$ 178.2 (1C), 172.5 (1C), 171.0 (1C), 170.1 (1C), 164.6 (1C), 163.5 (2C), 154.2 (1C), 153.5 (1C), 150.4 (1C), 149.7 (2C), 143.4 (1C), 142.0 (1C), 141.8 (1C), 140.2 (1C), 138.7 (1C), 137.9 (2C), 134.9 (1C), 134.9 (1C), 131.8 (1C), 131.3 (1C), 131.0 (2C), 130.8 (1C), 130.2 (1C), 129.9 (1C), 127.2 (1C), 126.5 (2C), 125.9 (1C), 125.8 (1C), 125.0 (1C), 124.3 (2C), 124.0 (1C), 123.6 (1C), 123.4 (1C), 121.9 (1C), 119.7 (1C), 119.4 (1C), 118.9 (1C), 118.2 (1C), 112.9 (1C), 52.0 (2C), 47.5 (1C), 39.4 (2C), 36.2 (1C), 32.0 (1C), 31.4 (1C), 26.4 (2C), 24.6 (1C), 16.5 (1C), 15.0 (2C). HRMS ($ES^+$): calcd. for $C_{59}H_{62}IrN_6O_5S$ 1159.4123; found 1159.4119 $[M-PF_6^-]^+$.

A.18. *Complex (**II-g**)*

From [(pq)zlrCl]z (133.6 mg, 0.11 mmol), (**II**) (130 mg, 0.21 mmol) and $Ag_2O$ (48.7 mg, 0.21 mmol) in 5 mL of MeCN. Eluent $CH_2Cl_2$ and MeOH (10:1). Orange solid (115 mg, 43% yield). Anal. Calcd. For $C_{63}H_{62}F_6IrN_6OPS$: C, 58.73; H, 4.85; N, 6.52. Found: C, 58.62; H, 4.87; N, 6.40. $^1H$ NMR (400 MHz, $CDCl_3$): $\delta$ 8.40 (d, *J* = 8.9 Hz, 1H), 8.30 (d, *J* = 8.8 Hz, 1H), 8.18 (d, *J* = 8.8 Hz, 1H), 8.13-8.03 (m, 2H), 7.93 (d, *J* = 7.9 Hz, 1H), 7.84 (dd, *J* = 7.9, 1.7 Hz, 1H), 7.80-7.73 (m, 2H), 7.68 (dd, *J* = 8.0, 1.7 Hz, 1H), 7.62 (d, *J* = 8.7 Hz, 1H), 7.58-7.48 (m, 3H), 7.40-7.29 (m, 5H), 7.24-7.16 (m, 2H), 7.15-7.03 (m, 3H), 7.00-6.93 (m, 2H), 6.82 (dd, *J* = 7.4, 1.2 Hz, 1H), 6.68-6.57 (m, 2H), 6.51 (t, *J* = 6.1 Hz, 1H), 6.30 (dd, *J* = 7.6, 1.2 Hz, 1H), 5.34-5.22 (m, 1H), 5.09 (m, 2H), 3.66 (m, 1H), 3.55 (d, *J* = 7.7 Hz, 2H), 3.45 (m, 1H), 3.23 (m, 1H), 2.98 (m, 1H), 2.02 (m, 8H), 1.68 (m, 4H), 1.64-1.54 (m, 9H), 1.41-1.33 (m, 1H). $^{13}C$ NMR (101 MHz, $CDCl_3$): $\delta$ 179.6 (1C), 171.3 (1C), 170.7 (1C), 168.4 (1C), 164.0 (1C), 152.4 (1C), 149.4 (1C), 148.3 (1C), 147.1 (2C), 146.7 (1C), 144.7 (1C), 141.5 (1C), 140.9 (1C), 140.1 (1 C), 139.5 (1C), 136.2 (1C), 135.4 (2C), 133.2 (1C), 132.8 (1C), 131.5 (1C), 131.4 (1C), 131.2 (1C), 131.1 (2C), 130.5 (1C), 130.0 (1C), 129.2 (2C), 128.5 (1C), 127.8 (2C), 127.5 (1C), 127.0 (1C), 126.9 (1C), 126.7 (1C), 126.4 (1C), 126.0 (1C), 124.7 (1C), 124.3 (1C), 123.7 (2C), 123.5 (2C), 122.0 (1C), 118.5 (1C), 118.1 (1C), 117.8 (1C), 117.4 (1C), 112.3 (1C), 47.2 (1C), 39.7 (2C), 36.7 (1C), 32.4 (1C), 31.3 (1C), 26.8 (2C), 25.7 (1C), 17.7 (1C), 16.1 (2C). HRMS ($ES^+$): calcd. for $C_{63}H_{62}IrN_6OS$ 1143.4335; found 1143.4332 $[M-PF_6^-]^+$.

A.19. *Complex (**II-h**)*

From [(piq)$_2$IrCl]$_2$ (102 mg, 0.08 mmol), (**II**) (100 mg, 0.16 mmol) and AgzO (37 mg, 0.16 mmol) in 5 mL of MeCN. Eluent $CH_2Cl_2$ and MeOH (10:1). Yellow solid (85 mg, 41% yield). Anal. Calcd. For $C_{63}H_{62}F_6IrN_6OPS$: C, 58.73; H, 4.85; N, 6.52. Found: C, 58.58; H, 4.88; N, 6.39. $^1H$ NMR (400 MHz, $CDCl_3$): $\delta$ 8.94 (d, *J* = 7.6 Hz, 1H), 8.88 (d, *J* = 7.5 Hz, 1H), 8.29 (d, *J* = 8.1 Hz, 1H), 8.21 (d, *J* = 8.0 Hz, 1H), 8.11-8.04 (m, 3H), 7.92 (m, 2H), 7.80-7.70 (m, 6H), 7.50-7.29 (m, 8H), 7.20 (m, 1H), 7.17-7.10 (m, 2H), 7.05-6.93 (m, 2H), 6.77 (m, 1H), 6.36 (d, *J* = 7.7 Hz, 1H), 6.26 (d, *J* = 7.4 Hz, 1H), 5.20 (t, *J* = 7.7 Hz, 1H), 5.08 (m, 2H), 3.58-3.33 (m, 4H), 3.07-2.96 (m, 2H), 2.08-1.89 (m, 8H), 1.68-1.49

(m, 12H), 1.34-1.24 (m, 2H). $^{13}C$ NMR (101 MHz, $CDCl_3$): $\delta$ 179.6 (1C), 170.2 (1C), 168.3 (1C), 167.9 (1C), 165.5 (1C), 153.2 (1C), 152.3 (1C), 149.5 (1C), 146.1 (1C), 145.0 (1C), 144.36 (1C), 141.7 (1C), 140.8 (2C), 136.9 (2C,), 136.5 (1C), 134.7 (1C), 131.6 (4C), 131.4 (2C), 131.0 (1C), 130.9 (2C), 130.5 (2C), 130.3 (1C), 128.7-128.6 (3C), 127.6 (1C), 127.5 (1C), 127.3 (1C), 126.7 (1C), 126.4 (2C), 126.3 (1C), 124.3 (1C), 124.0 (1C), 123.9 (1C), 123.7 (1C), 122.8 (1C), 122.7 (1C), 121.6 (1C), 121.5 (1C), 118.5 (1C), 118.2 (1C), 113.0 (1C), 47.9 (1C), 39.6 (2C), 36.7 (1C), 32.7 (1C), 31.7 (1C), 26.7 (1C), 26.4 (1C), 25.7 (1C), 17.7 (1C), 16.1 (2C). HRMS ($ES^+$): calcd. for $C_{63}H_{62}IrN_6OS$ 1143.4338; found 1143.4335 $[M-PF_6^-]^+$.

A.20. *Complex (**II-i**)*

From $[(bzq)_2IrCl]_2$ (93.5 mg, 0.08 mmol), (**II**) (100 mg, 0.16 mmol) and AgzO (37 mg, 0.16 mmol) in 5 mL of MeCN. Eluent $CH_2Cl_2$ and MeOH (10:1). Yellow solid (101 mg, 51% yield). Anal. Calcd. For $C_{59}H_{58}F_6IrN_6OPS$: C, 57.32; H, 4.73; N, 6.80. Found: C, 57.24; H, 4.75; N, 6.69. $^1H$ NMR (400 MHz, $CDCl_3$): $\delta$ 8.36 (dd, *J* = 5.4, 1.3 Hz, 1H), 8.25 (m, 2H), 7.94-7.86 (m, 4H), 7.79 (d, *J* = 8.8 Hz, 1H), 7.73 (d, *J* = 8.8 Hz, 1H), 7.66 (dd, *J* = 5.5, 1.3 Hz, 1H), 7.61 (m, 2H), 7.53-7.48 (m, 1H), 7.47-7.40 (m, 3H), 7.37-7.30 (m, 2H), 7.26 (d, J = 7.9 Hz, 1H), 7.22 (m, 1H), 7.18-7.11 (m, 2H), 7.05-6.93 (m, 2H), 6.39 (dd, *J* = 7.3, 0.8 Hz, 1H), 6.25 (dd, *J* = 7.0, 1.0 Hz, 1H), 6.15 (t, J = 6.0 Hz, 1H), 5.24 (m, 1H), 5.09 (m, 2H), 3.57-3.35 (m, 4H), 2.61 (m, 2H), 2.13-1.93 (m, 8H), 1.74-1.66 (m, 3H), 1.63-1.52 (m, 9H), 1.41-1.32 (m, 1H), 0.80-0.66 (m, 1H). $^{13}C$ NMR (101 MHz, $CDCl_3$): $\delta$ 178.0 (1C,), 168.2 (1C), 160.6 (1C), 158.1 (1C), 156.9 (1C), 153.5 (1C), 152.2 (1C), 150.2 (1C), 147.8 (1C), 145.3 (1C), 141.7 (1C), 141.1 (1C), 140.9 (1C), 140.0 (1C), 137.1 (1C), 136.4 (2C), 135.4 (1C), 134.9 (1C), 134.4 (1C), 134.1 (1C), 131.4 (1C), 130.5 (2C), 130.1 (3C), 130.0 (1C), 128.7 (1C), 128.6 (1C), 128.4 (1C), 127.5 (1C), 127.1 (1C), 126.2 (1C), 124.3 (1C), 123.8 (5C), 123.1 (1C), 122.1 (1C), 121.1 (1C), 119.5 (1C), 118.5 (1C), 117.8 (1C), 112.7 (1C), 48.1 (1C), 39.7 (2C), 36.4 (1C), 32.5 (1C), 31.6 (1C), 26.8 (2C), 25.7 (1C), 17.7 (1C), 16.1 (2C). HRMS ($ES^+$): calcd. for $C_{59}H_{58}IrN_6OS$ 1091.4022; found 1091.4025 $[M-PF_6^-]^+$.

A.21. *Complex (**II-j**)*

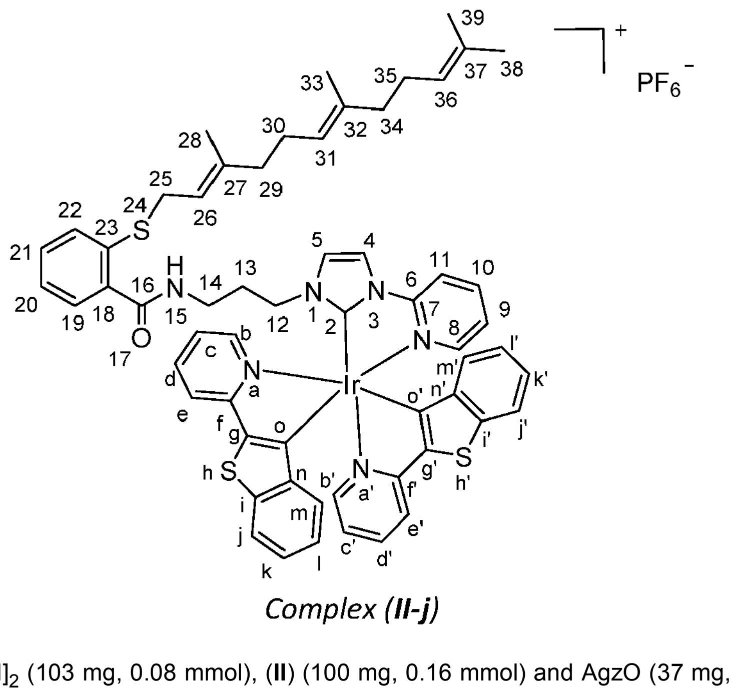

*Complex (**II-j**)*

From $[(thpy)_2IrCl]_2$ (103 mg, 0.08 mmol), (**II**) (100 mg, 0.16 mmol) and AgzO (37 mg, 0.16 mmol) in 5 mL of MeCN. Eluent $CH_2Cl_2$ and MeOH (20:1). Yellow solid (63 mg, 30% yield). Anal. Calcd. For $C_{59}H_{58}F_6IrN_6OPS_3$: C, 54.49; H, 4.50; N, 6.46. Found: C, 54.32; H, 4.67; N, 6.38. $^1H$ NMR (400 MHz, MeOD) $^1H$ NMR (400 MHz, $CDCl_3$): $\delta$ 8.14-7.99 (m, 3H), 7.89-7.76 (m, 6H), 7.66-7.57 (m, 3H), 7.41-7.30 (m, 2H), 7.26 (m, 2H), 7.21-7.08 (m, 3H), 7.04 (m, 1H), 6.94 (m, 1H), 6.86 (m, 2H), 6.21-6.11 (m, 2H), 5.92 (dd, *J* = 8.2, 3.8 Hz, 1H), 5.25 (t, *J* = 7.7 Hz, 1H), 5.09 (m, 2H), 3.58-3.33 (m), 2.78-2.48 (m, 2H), 2.02 (m, 8H), 1.70-1.67 (s, 3H), 1.65-1.51 (m, 9H), 1.31-1.25 (m, 2H). $^{13}C$ NMR (101 MHz, $CDCl_3$): $\delta$ 177.1 (1C), 168.4 (1C), 165.4 (2C), 164.5 (1C), 153.8 (1C), 153.3 (1C), 150.5 (2C), 149.7 (1C), 146.6 (1C), 146.2 (1C), 145.5 (1C), 143.7 (1C), 142.5 (1C), 139.3 (2C), 138.8 (1C), 138.4 (1C), 134.4 (1C), 134.2 (1C), 133.3 (1C), 132.1 (1C), 129.7 (1C), 126.6 (1C), 126.1 (1C), 125.7 (1C), 125.6 (2C), 125.2 (3C), 124.7 (1C), 124.4 (1C), 124.3 (2C),

124.0 (1C), 123.4 (1C), 123.1 (2C), 122.3 (1C), 120.6 (1C), 120.5 (1C), 119.9 (1C), 119.5 (1C), 119.0 (1C), 113.7 (1C), 47.9 (1C), 40.5 (2C), 38.3 (1C), 32.6 (1C), 31.8 (1C), 29.7 (2C), 29.4 (1C), 22.7 (1C), 14.1 (2C). HRMS (ES+): calcd. for $C_{59}H_{58}IrN_6OS_3$ 1155.3463; found 1155.3479 $[M-PF_6^-]^+$.

A.22. *Complex (**II-k**)*

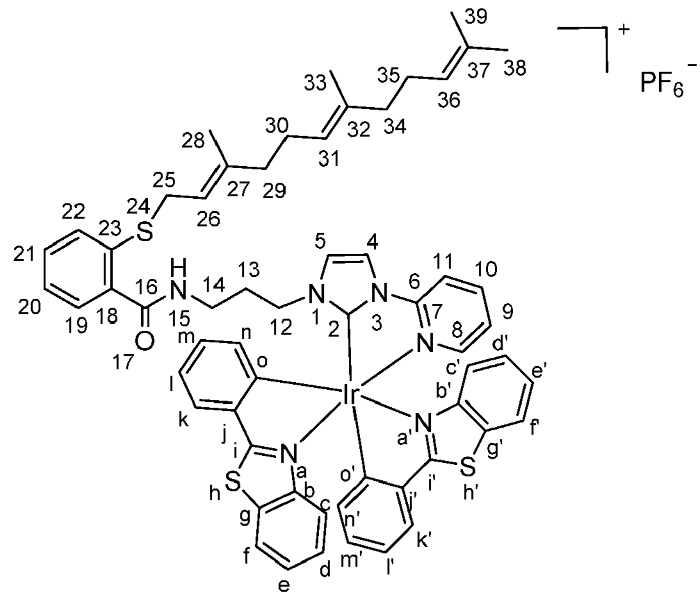

*Complex (**II-k**)*

From [(pbt)zIrCl]z (155.6 mg, 0.12 mmol), (**II**) (150 mg, 0.24 mmol) and $Ag_2O$ (55.6 mg, 0.24 mmol) in 5 mL of MeCN. Eluent $CH_2Cl_2$ and MeOH (20:1). Yellow solid (97 mg, 31% yield). Anal. Calcd. For $C_{59}H_{58}F_6IrN_6OPS_3$: C, 54.49; H, 4.50; N, 6.46. Found: C, 54.36; H, 4.62; N, 6.35. $^1H$ NMR (400 MHz, $CDCl_3$): $\delta$ 8.11 (m, 3H), 7.93-7.84 (m, 2H), 7.84-7.74 (m, 3H), 7.56-7.29 (m, 7H), 7.26-7.18 (m, 2H), 7.17-7.03 (m, 2H), 7.01-6.89 (m, 2H), 6.79-6.69 (m, 1H), 6.55 (s, 1H), 6.38 (m, 3H), 6.06 (d, *J* = 8.4 Hz, 1H), 5.29-5.19 (m, 1H), 5.16-5.02 (m, 2H), 3.67-3.54 (m, 2H), 3.50 (d, *J* = 7.6 Hz, 2H), 3.05-2.95 (m, 2H), 2.11-1.89 (m, 8H), 1.77-1.65 (m, 4H), 1.65-1.43 (m, 10H). $^{13}C$ NMR (101 MHz, $CDCl_3$): $\delta$ 182.7 (1C), 179.6 (1C), 175.5 (1C), 168.4 (1C), 163.6 (1C), 153.8 (1C), 150.1 (1C), 149.7 (1C), 149.1 (2C), 142.3 (1C), 140.6 (1C), 139.1 (1C), 132.4 (3C), 131.8 (1C), 131.0 (2C), 130.4 (1C), 128.7 (1C), 128.3 (1C), 126.7 (1C), 126.4 (2C), 126.1 (1C), 124.3 (1C), 124.0 (1C), 123.7 (2C), 123.5 (2C), 122.5 (1C), 118.4 (2C), 117.6 (1C), 113.0 (1C), 48.0 (1C), 39.7 (2C), 36.6 (1C), 32.7 (1C), 31.5 (1C), 26.8 (2C), 25.7 (1C), 17.7 (1C), 16.1 (2C). HRMS (ES+): calcd. for $C_{59}H_{58}IrN_6OS_3$ 1155.3463; found 1155.3483 $[M-PF_6^-]^+$.

A.23. *Complex (**II-l**)*

From [(2,4-F-ppy)zIrCl]z (146 mg, 0.12 mmol), (**II**) (150 mg, 0.24 mmol) and $Ag_2O$ (55.6 mg, 0.24 mmol) in 5 mL of MeCN. Eluent $CH_2Cl_2$ and MeOH (10:1). Yellow solid (108 mg, 36% yield). Anal. Calcd. For $C_{55}H_{54}F_{10}IrN_6OPS$: C, 52.42; H, 4.32; N, 6.67. Found: C, 52.32; H, 4.45; N, 6.53. $^1H$ NMR (400 MHz, $CDCl_3$) $\delta$ 8.38-8.32 (m, 1H), 8.32-8.27 (m, 1H), 8.18-8.06 (m, 2H), 8.04 (d, *J* = 2.2 Hz, 1H), 7.94 (dd, *J* = 6.0, 1.6 Hz, 1H), 7.82 (m, 2H), 7.71 (dd, *J* = 5.6, 1.2 Hz, 1H), 7.60-7.50 (m, 2H), 7.40-7.31 (m, 3H), 7.30-7.21 (m, 2H), 7.17 (ddd, *J* = 7.4, 5.8, 1.4 Hz, 1H), 7.09 (ddd, *J* = 7.4, 5.9, 1.4 Hz, 1H), 6.72 (t, *J* = 6.0 Hz, 1H), 6.56 (ddd, *J* = 12.6, 8.9, 2.4 Hz, 1H), 6.47 (ddd, *J* = 12.4, 9.0, 2.4 Hz, 1H), 5.79 (dd, *J* = 8.5, 2.3 Hz, 1H), 5.63 (dd, *J* = 7.7, 2.4 Hz, 1H), 5.25 (t, *J* = 7.1 Hz, 1H), 5.14-5.04 (m, 2H), 3.66 (d, *J* = 6.7, Hz, 2H), 3.52 (d, *J* = 7.7 Hz, 2H), 3.12 (dd, *J* = 13.3, 6.7 Hz, 2H), 2.01 (m, 8H), 1.69 (d, *J* = 1.4 Hz, 3H), 1.63-1.51 (m, 11H). $^{13}C$ NMR (101 MHz, $CDCl_3$): $\delta$ 176.4 (1C), 168.4 (1C), 167.4 (1C), 164.9 (d, $J_{C-F}$ = 7 Hz, 1C), 164.3 (dd, $J_{C-F}$ = 259, 11 Hz, 1C), 163.8 (d, $J_{C-F}$ = 7 Hz, 1C), 163.5 (dd, $J_{C-F}$ = 258, 11 Hz, 1C), 161.7 (dd, $J_{C-F}$ = 263, 11 Hz, 1C), 161.4 (dd, $J_{C-F}$ = 261, 13 Hz, 1C), 153.5 (1C), 153.1 (1C), 152.4 (d, $J_{C-F}$ = 7 Hz, 1C), 149.4 (1C), 149.0 (1C), 142.4 (1C), 140.9 (1C), 138.9 (1C), 138.3 (1C), 136.2 (1C), 135.4 (1C), 134.8 (1C), 131.4 (1C), 130.8 (1C), 130.5 (1C), 128.8 (1C), 127.9 (1C), 126.9 (1C), 126.4 (1C), 124.8 (1C), 124.4 (1C), 124.4 (1C), 124.3 (1C), 124.0 (1C), 123.8 (1C), 123.7 (1C), 123.5 (1C), 118.5 (1C), 118.3 (1C), 113.5 (1C), 113.1 (d, $J_{C-F}$ = 17 Hz, 1C), 113.0 (d, $J_{C-F}$ = 17 Hz, 1C), 99.7 (t, $J_{C-F}$ = 27 Hz,

1C), 98.2 (t, $J_{C-F}$ = 27 Hz, 1C), 48.2 (1C), 39.7 (1C), 39.6 (1C), 36.7 (1C), 32.7 (1C), 31.9 (1C), 26.7 (1C), 26.4 (1C), 25.7 (1C), 17.7 (1C), 16.1 (1C), 16.0 (1C). HRMS ($ES^+$): calcd. for $C_{55}H_{54}IrN_6OS$ 1115.3645; found 1115.3660 $[M-PF_6^-]^+$.

B. Synthesis of complexes (**III-a-b**), (**III-d-e**), (**III-g-l**) and (**IV-a**) to (**IV-l**).

The iridium(III) dimers $[(C^\wedge N)_2IrCl]_2$were prepared according to literature procedures [6,7].

B.1. *General protocol for the synthesis of complexes (**III-a-b**),* (**III-d-e**), *(**III-g-l**) and (**IV-a**) to (**IV-l**)*

A mixture of two equivalents of (**III**) or (IV), two equivalents of silver oxide and one equivalent of $[(C^\wedge N)_2IrCl]_2$ in MeOH for (**III**) or MeCN for (IV) (10 mL) was refluxed for 12 h. After cooling down to room temperature, an aqueous solution of $NH_4PF_6$ (200 mg in 10 mL deionized water) was slowly added into the reaction mixture under stirring, resulting in a yellow suspension. The suspension was then filtered and the resulting precipitate was washed with deionized water and dried under vacuum at 70°C for 12 h. The crude product was purified by column chromatography ($SiO_2$) with $CH_2Cl_2$/MeOH (10:1) as the eluent, yielding a solid.

B.2. *Complex (**III-a**)*

From (**III**) (0.100 g, 0.167 mmol), silver oxide (0.039 g, 0.167 mmol) and [(ppy)zIrCl]z (0.089 g, 0.083 mmol). Yellow solid (0.041 g, 21%). Anal. Calcd. For $C_{45}H_{46}F_6IrN_8O_8P$: C, 46.43; H, 3.98; N, 9.63. Found: C, 46.21; H, 4.02; N, 9.51. $^1H$ NMR (600 MHz, MeOD + $CD_2Cl_2$): $\delta$ 8.25 (d, *J* = 2.2 Hz, 1H), 8.19-8.14 (m, 2H), 8.12 (dd, *J* = 13.7, 6.2 Hz, 1H), 8.08 (d, *J* = 4.4 Hz, 1H), 8.07 (s, 1H), 7.90 (dd, *J* = 4.5, 2.9 Hz, 1H), 7.88 (d, *J* = 7.9 Hz, 1H), 7.81 (d, *J* = 7.7 Hz, 2H), 7.79-7.73 (m, 1H), 7.65 (dd, *J* = 8.8, 5.1 Hz, 1H), 7.42-7.35 (m, 1H), 7.27 (t, *J* = 6.5 Hz, 1H), 7.11 (ddd, *J* = 6.0, 4.4, 2.7 Hz, 1H), 7.10-7.06 (m, 1H), 7.05-7.01 (m, 1H), 6.97-6.91 (m, 2H), 6.85-6.80 (m, 1H), 6.45 (dd, *J* = 7.5, 4.4 Hz, 1H), 6.23 (d, *J* = 7.4 Hz, 1H), 4.41-4.28 (m, 1H), 4.19-4.06 (m, 1H), 3.81 (m, 2H), 3.23-2.79 (m, 2H), 2.70-2.35 (m, 2H), 2.32-2.06 (m, 4H), 1.96-1.65 (m, 2H), 1.54-1.42 (m, 2H), 1.42-1.30 (m, 2H). $^{13}C$ NMR (151 MHz, MeOD): $\delta$ 179.4 (1C), 179.3 (1C), 175.5 (1C), 175.0 (1C), 170.3 (1C), 168.8 (1C), 167.5 (1C), 163.0 (1C), 158.2 (1C), 153.6 (1C), 153.4 (1C), 149.4 (1C), 148.7 (1C), 148.6 (1C), 144.6 (1C), 142.8 (1C), 141.4 (1C), 138.9 (1C), 137.4 (1C), 130.9 (1C), 130.7 (1C), 130.2 (1C), 129.8 (1C), 127.8 (1C), 124.6 (1C), 124.6 (1C), 123.9 (1C), 123.8 (1C), 123.7 (1C), 122.7 (1C), 121.3 (1C), 119.7 (1C), 119.7 (1C), 117.7 (1C), 117.5 (1C), 56.6 (1C), 55.3 (1C), 45.8 (1C), 38.7 (1C), 36.6 (1C), 34.9 (1C), 30.5 (1C), 28.5 (1C), 25.5 (1C), 21.3 (1C). HRMS ($ES^+$): calcd. for $C_{45}H_{44}N_8O_7Ir$ 1001.2965; found 1001.2946 $[M-PF_6^--H_2O]^+$.

B.3. *Complex (**III-b**)*

From (**III**) (0.100 g, 0.167 mmol), silver oxide (0.039 g, 0.167 mmol) and [(4-Me-ppy)zIrCl]z (0.094 g, 0.083 mmol). Yellow solid (0.038 g, 19%). Anal. Calcd. For $C_{47}H_{50}F_6IrN_8O_8P$: C, 47.35; H, 4.23; N, 9.40. Found: C, 47.21; H, 4.23; N, 9.26. $^1H$ NMR (400 MHz, MeOD): $\delta$ 8.25 (d, *J* = 2.2 Hz, 1H), 8.12-8.03 (m, 3H), 7.99 (m, 2H), 7.9-7.80 (m, 2H), 7.78 (d, *J* = 5.3 Hz, 1H), 7.70-7.63 (m, 2H), 7.60 (d, *J* = 5.4 Hz, 1H), 7.35 (d, *J* = 2.2 Hz, 1H), 7.28 (ddd, *J* = 7.0, 5.5, 1.3 Hz, 1H), 7.04 (dd, *J* = 14.2, 7.0 Hz, 2H), 6.86 (d, *J* = 8.0 Hz, 1H), 6.77 (d, *J* = 7.8 Hz, 1H), 6.25 (s, 1H), 6.04 (s, 1H), 4.30-4.15 (m, 2H), 3.94-3.70 (m, 2H), 3.15-3.03 (m, 2H), 2.49-2.34 (m, 2H), 2.13 (t, *J* = 6.2 Hz, 2H), 2.05 (s, 3H), 2.04 (s, 3H), 1.98-1.87 (m, 2H), 1.85-1.58 (m, 2H), 1.51-1.42 (m, 2H), 1.42-1.32 (m, 2H). $^{13}C$ NMR (151 MHz, MeOD): $\delta$ 179.6 (1C), 176.2 (1C), 175.8 (2C), 170.2 (1C), 168.8 (1C), 167.4 (1C), 163.7 (1C), 158.8 (1C), 153.5 (1C), 153.1 (1C), 149.5 (1C), 148.9 (1C), 148.6 (1C), 142.0 (1C), 141.3 (1C), 140.4 (1C), 140.3 (1C), 140.0 (1C), 137.9 (1C), 137.2 (1C), 131.5 (1C), 131.4 (1C), 124.6 (2C), 123.8 (1C), 123.7 (1C), 123.7 (1C), 123.3 (1C), 122.3 (1C), 122.2 (1C), 119.4 (2C), 117.5 (1C), 112.4 (1C), 53.4 (1C), 53.1 (1C), 45.7 (1C), 38.8 (1C), 36.6 (1C), 32.1 (1C), 30.2 (1C), 28.5 (1C), 28.1 (1C), 22.8 (1C), 20.5 (2C). HRMS ($ES^+$): calcd. for $C_{47}H_{50}N_8O_8Ir$ 1047.3384; found 1047.3405 $[M-PF_6^-]^+$.

B.4. *Complex (**III-d**)*

From (**III**) (0.100 g, 0.167 mmol), silver oxide (0.039 g, 0.167 mmol) and [(3-COOMe-ppy)zIrCl]z (0.108 g, 0.083 mmol). Yellow solid (0.082 g, 39%). Anal. Calcd. For $C_{49}H_{50}F_6IrN_8O_{12}P$: C, 45.97; H, 3.94; N, 8.75. Found: C, 46.09; H, 4.01; N, 8.83. $^1H$ NMR (600 MHz, MeOD): $\delta$ 8.50-8.43 (m, 2H), 8.34-8.29 (m, 2H), 8.26-8.19 (m, 2H), 8.18-8.14 (m, 1H), 8.13-8.06 (m, 1H), 8.03-7.93 (m, 2H), 7.74-7.68 (m, 2H), 7.59-7.53 (m, 1H), 7.46-7.41 (m, 2H), 7.33-7.26 (m, 1H), 7.24-7.16 (m, 2H), 6.61-6.53 (m, 1H), 6.38-6.30 (m, 1H), 4.65-4.05 (m, 2H), 3.86 (s, 2H), 3.84-3.70 (m, 3H), 3.37 (s, 3H), 3.13-2.98 (m, 2H), 2.67-1.58 (m, 8H), 1.48-1.40 (m, 2H), 1.40-1.28 (m, 2H). $^{13}C$ NMR (151 MHz, MeOD): $\delta$ 178.0 (1C), 175.8 (2C), 171.5 (1C), 169.9-169.8 (2C), 167.8 (2C), 167.4 (1C), 166.2 (1C), 158.7 (1C), 157.8 (1C), 153.4 (2C), 149.2-149.4 (2C), 145.3 (1C), 143.6 (1C), 141.8 (1C), 138.8 (1C), 138.2 (1C), 131.1(2C), 130.6 (1C), 130.1(1C), 125.0 (3C), 124.9 (1C), 124.1-124.1 (2C), 123.8 (1C), 123.6 (1C), 120.4 (2C), 117.9 (1C), 112.9 (1C), 54.0 (1C), 53.2 (1C),

51.1 (1C), 48.5 (1C), 45.9 (1C), 38.7 (1C), 36.2 (1C), 32.5 (1C), 30.2 (1C), 28.4 (1C), 28.1 (1C), 22.6 (1C). HRMS ($ES^+$): calcd. for $C_{49}H_{50}N_8O_{12}Ir$ 1135.3154; found 1135.3181 $[M-PF_6^-]^+$.

B.S. *Complex (**III-e**)*

From (**III**) (0.100 g, 0.167 mmol), silver oxide (0.039 g, 0.167 mmol) and [(p-3-COOMe-ppy)zIrCl]z (0.108 g, 0.083 mmol). Yellow solid (0.092 g, 43%). Anal. Calcd. For $C_{49}H_{50}F_6IrN_8O_{12}P$: C, 45.97; H, 3.94; N, 8.75. Found: C, 45.88; H, 3.75; N, 8.57. $^1H$ NMR (600 MHz, MeOD): $\delta$ 8.48-8.34 (m, 5H), 8.34-8.28 (m, 2H), 8.24-8.18 (m, 1H), 8.16-8.11 (m, 1H), 7.97-7.91 (m, 2H), 7.84-7.80 (m, 1H), 7.51-7.42 (m, 1H), 7.41-7.33 (m, 1H), 7.14-7.08 (m, 1H), 7.05-6.98 (m, 2H), 6.94-6.84 (m, 1H), 6.48-6.42 (m, 1H), 6.28-6.24 (m, 1H), 4.62-4.26 (m, 2H), 3.91 (s, 3H), 3.82 (s, 3H), 3.79-3.60 (m, 2H), 3.20-2.99 (m, 2H), 2.68-1.65 (m, 8H), 1.57-1.07 (m, 4H). $^{13}C$ NMR (151 MHz, MeOD): $\delta$ 179.2 (1C), 178.2 (1C), 177.6 (2C), 172.5 (1C), 171.0 (2C), 169.9 (1C), 164.8 (1C), 163.8 (2C), 154.3 (1C), 153.6 (1C), 152.3 (1C), 150.2 (1C), 149.7 (2C), 143.4 (1C), 142.0 (2C), 138.5 (1C), 137.8 (1C), 131.8 (1C), 131.1 (1C), 130.9 (1C), 130.7 (1C), 126.2 (2C), 125.9 (1C), 124.9 (1C), 124.2 (1C), 123.2 (1C), 121.8 (1C), 119.3 (1C), 118.2 (2C), 113.1 (1C), 60.8 (1C), 55.2 (1C), 51.8 (1C), 48.5 (1C), 45.8 (1C), 39.0 (1C), 36.9 (1C), 34.7 (1C), 32.7 (1C), 30.6 (1C), 28.4 (1C), 22.1 (1C). HRMS ($ES^+$): calcd. for $C_{49}H_{48}N_8O_{11}Ir$ 1117.3075; found 1117.3080 $[M-PF_6^--H_2O]^+$.

B.6. *Complex (**III-g**)*

From (**III**) (0.100 g, 0.167 mmol), silver oxide (0.039 g, 0.167 mmol) and [(pq)zIrCl]z (0.106 g, 0.083 mmol). Yellow solid (0.084 g, 40%). Anal. Calcd. For $C_{53}H_{50}F_6IrN_8O_8P$: C, 50.35; H, 3.99; N, 8.86. Found: C, 50.45; H, 4.09; N, 8.71. $^1H$ NMR (600 MHz, MeOD): $\delta$ 8.55-8.49 (m, 1H), 8.46-8.42 (m, 1H), 8.30 (d, *J* = 8.8 Hz, 1H), 8.24 (d, *J* = 8.8 Hz, 1H), 8.07 (dd, *J* = 8.4, 5.6 Hz, 1H), 8.03-7.98 (m, 2H), 7.94 - 7.91 (m, 2H), 7.81-7.76 (m, 3H), 7.74-7.66 (m, 1H), 7.46-7.40 (m, 3H), 7.36 (m, 1H), 7.19-7.13 (m, 2H), 7.06-6.96 (m, 3H), 6.76 (m, 1H), 6.71-6.65 (m, 1H), 6.63 (dd, *J* = 7.8, 2.2 Hz, 1H), 6.21 (dd, *J* = 7.6, 5.2 Hz, 1H), 4.63-4.07 (m, 2H), 3.92-3.64 (m, 2H), 3.13-2.99 (m, 2H), 2.64-1.60 (m, 8H), 1.48-1.32 (m, 4H). $^{13}C$ NMR (151 MHz, MeOD): $\delta$ 179.9 (1C), 177.8 (1C), 177.5 (2C), 171.6 (1C), 170.8 (1C), 169.8 (1C), 164.1 (1C), 152.7 (1C), 152.6 (1C), 149.4 (1C), 148.3 (1C), 147.1 (1C), 147.0 (1C), 146.8 (1C), 144.8 (1C), 141.4 (1C), 140.1 (1C), 139.7 (1C), 132.6 (1C), 132.4 (1C), 131.3 (1C), 130.7 (1C), 130.6 (1C), 130.5 (1C), 129.2 (1C), 129.1 (1C), 128.0 (1C), 127.9 (1C), 127.1 (1C), 126.8 (1C,), 126.7 (1C), 126.5 (1C), 126.4 (1C), 124.6 (1C), 123.7 (1C), 123.4 (1C), 123.2 (1C), 121.6 (1C), 117.9 (1C), 117.8 (1C), 117.5 (1C), 112.2 (1C), 55.2 (1C), 54.7 (1C), 45.1 (1C), 38.9 (1C), 35.6 (1C), 32.4 (1C), 31.9 (1C), 28.6 (1C), 22.7 (1C), 22.0 (1C). HRMS ($ES^+$): calcd. for $C_{53}H_{50}N_8O_8Ir$ 1119.3385; found 1119.3365 $[M-PF_6^-]^+$.

B.7. *Complex (**III-h**)*

From (**III**) (0.100 g, 0.167 mmol), silver oxide (0.039 g, 0.167 mmol) and $[(piq)_2IrCl]_2$ (0.106 g, 0.083 mmol). Yellow solid (0.078 g, 37%). Anal. Calcd. For $C_{53}H_{50}F_6IrN_8O_8P$: C, 50.35; H, 3.99; N, 8.86. Found: C, 50.41; H, 3.75; N, 8.49. $^1H$ NMR (400 MHz, MeOD): $\delta$ 9.03 (d, *J* = 7.0 Hz, 1H), 8.95 (d, *J* = 8.5 Hz, 1H), 8.38 (d, *J* = 8.2 Hz, 1H), 8.30 (m, 1H), 8.28 (d, *J* = 8.0 Hz, 1H), 8.25-8.20 (m, 1H), 8.13 (m, 1H), 8.02 (d, *J* = 7.7 Hz, 2H), 7.91-7.77 (m, 5H), 7.59 (d, *J* = 6.9 Hz, 1H), 7.54 (d, *J* = 6.2 Hz, 1H), 7.50 (dd, *J* = 6.6, 2.5 Hz, 1H), 7.47 (d, *J* = 6.5 Hz, 1H), 7.43-7.36 (m, 1H), 7.29-7.22 (m, 1H), 7.13 (dd, *J* = 7.8 Hz, 1H), 7.06 (dd, *J* = 7.8 Hz, 1H), 6.93 (dd, *J* = 7.5 Hz, 1H), 6.80 (dd, *J* = 7.5 Hz, 1H), 6.43 (d, *J* = 7.5 Hz, 1H), 6.20 (d, *J* = 7.5 Hz, 1H), 4.36-4.10 (m, 2H), 3.78-3.57 (m, 2H), 3.09-2.98 (m, 2H), 2.47-2.32 (m, 2H), 2.20-2.11 (m, 2H), 2.09-1.98 (m, 2H), 1.93-1.53 (m, 2H), 1.45-1.38 (m, 2H), 1.35-1.29 (m, 2H). $^{13}C$ NMR (151 MHz, MeOD): $\delta$ 179.9 (2C), 176.2 (1C), 175.8 (2C), 170.1 (1C), 170.07 (1C), 168.0 (1C), 165.6 (1C), 158.8 (1C), 153.5 (1C), 152.2 (1C), 149.3 (1C), 146.1 (1C), 144.8 (1C), 144.2 (1C), 141.4 (1C), 140.9 (1C), 137.0 (1C), 136.6 (1C), 131.6 (1C), 131.4 (2C), 131.1 (1C), 130.6 (1C), 130.4 (2C), 129.7 (1C), 128.6 (2C), 127.3 (1C), 127.2 (1C), 126.9 (1C), 126.4 (2C), 126.3 (1C), 123.9 (1C), 123.7 (1C), 122.4 (1C), 122.3 (1C), 121.1 (1C), 120.9 (1C), 117.3 (1C), 112.6 (1C), 53.0 (2C), 45.6 (1C), 38.6 (1C), 36.8 (1C), 32.1 (1C), 30.1 (1C), 28.3 (1C), 22.5 (1C). HRMS ($ES^+$): calcd. for $C_{53}H_{50}N_8O_8Ir$ 1119.3385; found 1119.3390 $[M-PF_6^-]^+$.

B.8. *Complex (**III-i**)*

From (**III**) (0.100 g, 0.167 mmol), silver oxide (0.039 g, 0.167 mmol) and [(bzq)zIrCl]z (0.097 g, 0.083 mmol). Yellow solid (0.075 g, 37%). Anal. Calcd. For $C_{49}H_{46}F_6IrN_8O_8P$: C, 48.55; H, 3.83; N, 9.24. Found: C, 48.57; H, 3.77; N, 9.36. $^1H$ NMR (400 MHz, MeOD): $\delta$ 8.56-8.43 (m, 3H), 8.37-8.19 (m, 2H), 8.14-8.05 (m, 2H), 7.88 (dd, *J* = 8.9, 4.0 Hz, 2H), 7.83-7.78 (m, 1H), 7.73 (m, 2H), 7.65-7.47 (m, 3H), 7.41 (dd, *J* = 7.7, 3.0 Hz, 1H), 7.39-7.31 (m, 1H), 7.17 (m, 2H), 7.07 (dd, *J* = 7.5, 3.6 Hz, 1H), 6.41 (d, J = 7.0 Hz, 1H), 6.23 (d, *J* = 7.0 Hz, 1H), 4.45-4.01 (m, 2H), 3.73-3.51 (m, 2H), 3.20-2.95 (m, 2H), 2.57-1.58 (m, 8H), 1.50-1.38 (m, 2H), 1.38-1.23 (m, 2H). $^{13}C$ NMR (151 MHz, MeOD): $\delta$ 178.3

(1C), 177.3 (1C), 175.3 (1C), 174.0 (1C), 170.2 (1C), 160.9 (1C), 158.0 (1C), 158.0 (1C), 156.9 (1C), 154.0 (1C), 152.4 (1C), 150.0 (1C), 148.1 (1C), 145.1 (1C), 141.4 (1C), 141.1 (1C), 139.9 (1C), 137.1 (1C), 136.3 (1C), 134.6 (1C), 134.4 (1C), 129.7 (1C), 129.6 (2C), 129.2 (1C), 128.3 (1C), 128.0 (1C), 127.6 (1C), 127.4 (1C,), 123.8, 123.7, 123.6, 123.5 (4C), 122.7 (1C), 121.7 (1C), 120.7 (1C), 119.1 (1C), 117.8 (1C), 112.6 (1C), 56.5 (1C), 54.8 (1C), 45.7 (1C), 38.8 (1C), 36.3 (1C), 32.1 (1C), 30.5 (1C), 28.3 (1C), 24.6 (1C), 22.5 (1C). HRMS (ES$^+$): calcd. for $C_{49}H_{46}N_8O_8Ir$ 1067.3071; found 1067.3074 [M-$PF_6^-$]$^+$.

B.9. *Complex (**III-j**)*

From (**III**) (0.100 g, 0.167 mmol), silver oxide (0.039 g, 0.167 mmol) and [(thpy)$_2$IrCl]$_2$ (0.108 g, 0.083 mmol). Yellow solid (0.098 g, 46%). Anal. Calcd. For $C_{49}H_{46}F_6IrN_8O_8PS_2$: C, 46.11; H, 3.63; N, 8.78. Found: C, 46.21; H, 3.79; N, 8.63. $^1$H NMR (400 MHz, MeOD): $\delta$ 8.39-8.36 (d, *J* = 2.4 Hz, 1H), 8.33-8.19 (m, 1H), 8.12-8.04 (m, 2H), 7.98-7.88 (m, 2H), 7.81 (d, *J* = 8.2 Hz, 1H), 7.76-7.68 (m, 5H), 7.44-7.37 (m, 1H), 7.18-7.10 (m, 3H), 7.10-6.95 (m, 2H), 6.89-6.84 (m, 1H), 6.81 (dd, *J* = 7.7 Hz, 1H), 6.21-6.15 (m, 1H), 5.98-5.94 (m, 1H), 4.63-4.57 (m, 1H), 4.26-4.04 (m, 1H), 3.82-3.65 (m, 2H), 3.09-2.91 (m, 2H), 2.69-1.48 (m, 8H), 1.45-1.37 (m, 2H), 1.31-1.29 (m, 2H). $^{13}$C NMR (151 MHz, MeOD): $\delta$ 177.7, 177.6, 177.4 (3C), 173.7 (1C), 169.9 (1C), 165.3 (1C), 164.6 (1C), 162.4 (1C), 154.3 (1C), 153.5 (1C), 152.6 (1C), 150.1, 149.6 (2C), 146.2 (2C), 145.4 (1C), 143.6 (1C), 142.6 (1C), 142.0 (1C), 139.5 (1C), 138.7 (2C), 134.1 (1C), 125.5 (1C), 125.2 (1C), 125.0 (2C), 124.2 (2C), 123.9 (1C), 123.7 (1C), 122.8 (1C), 122.6 (1C), 122.0 (1C), 120.4 (1C), 119.5 (1C), 119.4 (1C), 118.2 (1C), 113.1 (1C), 60.9 (1C), 55.3 (1C), 46.2 (1C), 38.7 (1C), 36.2 (1C), 32.6 (1C), 32.0 (1C), 28.3 (1C), 22.4 (1C), 22.1 (1C). HRMS (ES$^+$): calcd. for $C_{49}H_{46}N_8O_8S_2Ir$ 1131.2404; found 1131.2510 [M-$PF_6^-$]$^+$.

B.10. *Complex (**III-k**)*

From (**III**) (0.100 g, 0.167 mmol), silver oxide (0.039 g, 0.167 mmol) and [(pbt)$_2$IrCl]$_2$ (0.108 g, 0.083 mmol). Yellow solid (0.108 g, 51%). Anal. Calcd. For $C_{49}H_{46}F_6IrN_8O_8PS_2$: C, 46.11; H, 3.63; N, 8.78. Found: C, 46.22; H, 3.79; N, 8.63. $^1$H NMR (400 MHz, MeOD): $\delta$ 8.40 (d, *J* = 2.4 Hz, 1H), 8.24 (ddd, *J* = 7.7, 7.1, 3.9 Hz, 1H), 8.19- 8.14 (m, 1H), 8.11 (d, *J* = 8.1 Hz, 1H), 8.03 (dd, *J* = 8.1, 3.9 Hz, 1H), 7.91 (dd, *J* = 7.7, 3.3 Hz, 1H), 7.89-7.84 (m, 2H), 7.62-7.54 (m, 1H), 7.50-7.41 (m, 2H), 7.41-7.32 (m, 2H), 7.17-7.07 (m, 2H), 7.00 (dd, *J* = 7.5 Hz, 1H), 6.94 (dd, *J* = 7.4 Hz, 1H), 6.80 (dd, *J* = 7.5 Hz, 1H), 6.48-6.40 (m, 2H), 6.34 (d, J = 7.5 Hz, 1H), 6.17 (dd, *J* = 8.5, 5.4 Hz, 1H), 4.13 (m, 2H), 3.93-3.74 (m, 2H), 3.01 (m, 2H), 2.32 (m, 2H), 2.29-2.10 (m, 2H), 2.10-1.82 (m, 2H), 1.67 (m, 2H), 1.35-1.21 (m, 4H). $^{13}$C NMR (151 MHz, MeOD): $\delta$ 183.1 (1C), 180.2 (1C), 178.4 (1C), 175.8 (1C), 169.8 (3C), 163.8 (1C), 158.6 (1C), 154.0 (1C), 149.9 (1C), 149.7 (1C), 149.0 (2C), 142.0 (1C), 140.8 (1C), 139.3 (1C), 132.2 (1C), 132.1 (1C), 131.9 (1C), 131.6 (1C), 131.4 (1C), 131.3 (1C), 128.2 (1C), 127.5 (1C), 126.4 (1C), 126.1 (1C), 126.0 (1C), 125.7 (1C), 123.9 (1C), 123.7 (2C), 123.6 (1C), 123.5 (1C), 122.1 (1C), 118.1 (1C), 117.2 (2C), 112.6 (1C), 55.2 (2C), 45.8 (1C), 38.8 (1C), 35.9 (1C), 33.2 (1C), 33.0 (1C), 29.9 (1C), 28.6 (1C), 22.6 (1C). HRMS (ES$^+$): calcd. for $C_{49}H_{46}N_8O_8IrS_2$ 1131.2510; found 1131.2511 [M-$PF_6^-$]$^+$.

B.11. *Complex (**III-l**)*

From (**III**) (0.100 g, 0.167 mmol), silver oxide (0.039 g, 0.167 mmol) and [(2,4-F-ppy)zIrCl]z (0.101 g, 0.083 mmol). Yellow solid (0.115 g, 56%). Anal. Calcd. For $C_{45}H_{42}F_{10}IrN_8O_8P$: C, 43.73; H, 3.43; N, 9.07. Found: C, 43.63; H, 3.38; N, 8.94. $^1$H NMR (600 MHz, MeOD): $\delta$ 8.41 (d, *J* = 8.6 Hz, 1H), 8.37-8.31 (m, 2H), 8.29-8.18 (m, 2H), 8.12-8.06 (m, 1H), 7.96 (m, 2H), 7.82-7.78 (m, 1H), 7.72 (dd, *J* = 6.5, 2.9 Hz, 1H), 7.49 (m, 1H), 7.41-7.36 (m, 1H), 7.22-7.13 (m, 2H), 6.66-6.60 (m, 1H), 6.60-6.52 (m, 1H), 5.85 (dd, *J* = 8.3, 2.5 Hz, 1H), 5.65 (dd, *J* = 8.0, 1.9 Hz, 1H), 4.56-4.48 (m, 1H), 4.30-4.18 (m, 1H), 3.92-3.81 (m, 2H), 3.17-3.03 (m, 2H), 2.70-2.35 (m, 2H), 2.34-2.19 (m, 2H), 2.18-2.01 (m, 2H), 1.97-1.57 (m, 2H), 1.50-1.41 (m, 2H), 1.40-1.30 (m, 2H). $^{13}$C NMR (151 MHz, MeOD): 176.6 (1C), 175.8 (1C), 175.4 (1C), 175.1 (1C), 169.8 (1C), 168.1 (1C), 164.7 (d, $J_{C\text{-}F}$ = 6 Hz, 1C), 164.2 (dd, $J_{C\text{-}F}$ = 258, 11 Hz, 1C), 163.7 (d, $J_{C\text{-}F}$ = 6 Hz, 1C), 163.5 (dd, $J_{C\text{-}F}$ = 258, 11 Hz, 1C), 161.9 (dd, $J_{C\text{-}F}$ = 260, 12 Hz, 1C), 161.6 (dd, $J_{C\text{-}F}$ = 260, 12 Hz, 1C), 158.7 (1C), 153.7 (1C), 153.4 (1C), 152.7 (1C), 149.5 (1C), 149.4 (1C), 142.1 (1C), 139.1 (1C), 138.4 (1C), 127.9 (1C), 127.0 (1C), 124.5 (1C), 124.3 (1C), 124.0 (1C), 123.6 (2C), 123.4 (1C), 118.1 (1C), 113.0 (1C), 112.7 (d, $J_{C\text{-}F}$ = 18 Hz, 1C), 112.4 (d, $J_{C\text{-}F}$ = 18 Hz, 1C), 98.9 (t, $J_{C\text{-}F}$ = 27 Hz, 1C), 97.5 (t, $J_{C\text{-}F}$ = 27 Hz, 1C), 56.6 (1C), 52.9 (1C), 46.0 (1C), 38.7 (1C), 36.2 (1C), 32.1 (1C), 30.1 (1C), 28.3 (1C), 28.0 (1C), 22.6 (1C). HRMS (ES$^+$): calcd. for $C_{45}H_{40}N_8O_7F_4Ir$ 1073.2562; found 1073.2584 [M-$PF_6^-$-$H_2O$]$^+$.

B.12. *Complex (**IV-a**)*

From (IV) (0.06 g, 0.12 mmol), silver oxide (0.028 g, 0.12 mmol) and [(ppy)zIrCl]z (0.078 g, 0.06 mmol). Yellow solid (0.077 g, 61%). Anal. Calcd. For $C_{44}H_{38}F_6FeIrN_6OP$: C, 49.86; H, 3.61; N, 7.93. Found: C, 49.69; H, 3.47; N, 7.81.

$^{1}H$ NMR (400 MHz, $CDCl_3$): $\delta$ 8.95 (s, 1H), 8.87 (d, $J$ = 8.3 Hz, 1H), 8.39 (s, 1H), 8.23 (t, $J$ = 5.7 Hz, 1H), 8.02 (td, $J$ = 7.8, 1.7 Hz, 1H), 7.95 (d, $J$ = 8.2 Hz, 1H), 7.91-7.85 (m, 2H), 7.78-7.71 (m, 2H), 7.71-7.64 (m, 3H), 7.46 (d, $J$ = 5.8 Hz, 1H), 7.15-7.08 (m, 1H), 7.08-7.00 (m, 2H), 7.00-6.91 (m, 3H), 6.90-6.81 (m, 1H), 6.35 (d, $J$ = 7.7 Hz, 1H), 6.26 (d, $J$ = 7.3 Hz, 1H), 5.21 (d, $J$ = 1.2 Hz, 1H), 5.16 (d, $J$ = 1.3 Hz, 1H), 4.24 (dd, $J$ = 3.2 Hz, 2H), 4.14 (s, 5H), 3.75 (t, $J$ = 7.1 Hz, 2H), 3.11-2.90 (m, 2H), 1.60-1.43 (m, 2H). $^{13}C$ NMR (101 MHz, $CDCl_3$): $\delta$ 178.2 (1C), 170.9 (1C), 168.9 (1C), 167.7 (1C), 163.7 (1C), 153.9 (1C), 153.1 (1C), 149.5 (1C), 148.6 (1C), 148.4 (1C), 144.2 (1C), 142.3 (1C), 141.5 (1C), 137.7 (1C), 137.0 (1C), 131.1 (1C), 130.9 (2C), 130.6 (1C), 125.7 (1C), 124.9 (1C), 124.7 (1C), 124.0 (1C), 123.4 (1C), 123.0 (1C), 122.6 (1C), 121.9 (1C), 119.8 (1C), 119.6 (1C), 118.6 (1C), 114.0 (1C), 76.4 (1C), 70.3 (1C), 70.2 (1C), 69.7 (5C), 69.1 (2C), 47.8 (1C), 36.1 (1C), 32.1 (1C). HRMS ($ES^+$): calcd. for $C_{44}H_{38}N_6OFeIr$ 915.2086; found 915.2093 $[M-PF_6^-]^+$.

B.13. *Complex (**IV-b**)*

From (IV) (0.06 g, 0.12 mmol), silver oxide (0.028 g, 0.12 mmol) and [(4-Me-ppy)zIrCl]z (0.067 g, 0.06 mmol). Yellow solid (0.083 g, 63%). Anal. Calcd. For $C_{46}H_{42}F_6FeIrN_6OP$: C, 50.79; H, 3.89; N, 7.73. Found: C, 50.64; H, 3.71; N, 7.65. $^{1}H$ NMR (400 MHz, $CDCl_3$): $\delta$ 8.86 (d, $J$ = 2.1 Hz, 1H), 8.79 (d, $J$ = 8.3 Hz, 1H), 8.35 (d, $J$ = 1.9 Hz, 1H), 8.21 (t, $J$ = 5.5 Hz, 1H), 8.06-7.99 (m, 1H), 7.89 (d, $J$ = 8.3 Hz, 1H), 7.87-7.79 (m, 2H), 7.76-7.64 (m, 3H), 7.62-7.55 (m, 2H), 7.43 (dd, $J$ = 5.7, 1.5 Hz, 1H), 7.13 (dd, $J$ = 7.4, 5.5 Hz, 1H), 6.99 (ddd, $J$ = 7.4, 5.8, 1.5 Hz, 1H), 6.90 (ddd, $J$ = 7.4, 5.8, 1.4 Hz, 1H), 6.87-6.79 (m, 2H), 6.18-6.13 (m, 1H), 6.07-6.02 (m, 1H), 5.22 (d, $J$ = 1.4 Hz, 1H), 5.16 (d, $J$ = 1.3 Hz, 1H), 4.24 (dd, $J$ = 3.5 Hz, 2H), 4.14 (s, 5H), 3.75 (t, $J$ = 7.0 Hz, 2H), 3.14-2.92 (m, 2H), 2.12 (s, 3H), 2.09 (s, 3H), 1.62-1.43 (m, 2H). $^{13}C$ NMR (101 MHz, $CDCl_3$): $\delta$ 178.4 (1C), 170.9 (1C), 168.9 (1C), 167.8 (1C), 164.0 (1C), 153.9 (1C), 152.9 (1C), 149.6 (1C), 148.8 (1C), 148.3 (1C), 141.6 (1C), 141.3 (1C), 140.9 (1C), 140.8 (1C), 139.8 (1C), 137.6 (1C), 136.8 (1C), 132.0 (1C), 131.6 (1C|), 125.6 (1C), 124.8 (1C), 124.6 (1C), 124.1 (1C), 123.4 (1C), 123.4 (1C), 123.1 (1C), 122.0 (1C), 119.4 (1C), 119.3 (1C), 118.5 (1C), 113.8 (1C), 76.4 (1C), 70.3 (1C), 70.2 (1C), 69.7 (5C), 69.1 (1C), 69.1 (1C), 47.8 (1C), 36.1 (1C), 32.1 (1C), 21.9 (2C). HRMS ($ES^+$): calcd. for $C_{46}H_{42}N_6OFeIr$ 943.2402; found 943.2379 $[M-PF_6^-]^+$.

B.14. *Complex (**IV-c**)*

From (IV) (0.06 g, 0.12 mmol), silver oxide (0.028 g, 0.12 mmol) and $[(4\text{-COOMe-ppy})_2IrCl]_2$ (0.078 g, 0.06 mmol). Yellow solid (0.081 g, 57%). Anal. Calcd. For $C_{48}H_{42}F_6FeIrN_6O_5P$: C, 49.03; H, 3.60; N, 7.15. Found: C, 49.21; H, 3.53; N, 7.22. $^{1}H$ NMR (400 MHz, $CDCl_3$): $\delta$ 8.14-8.06 (m, 2H), 8.03 (d, $J$ = 2.3 Hz, 1H), 8.00-7.95 (m, 3H), 7.90-7.80 (m, 3H), 7.75-7.70 (m, 3H), 7.66 (dd, $J$ = 8.2, 1.7 Hz, 1H), 7.62 (dd, $J$ = 5.6, 1.2 Hz, 1H), 7.33 (d, $J$ = 2.2 Hz, 1H), 7.23-7.12 (m, 3H), 7.04 (d, $J$ = 1.6 Hz, 1H), 6.87 (d, $J$ = 1.6 Hz, 1H), 6.20 (t, $J$ = 6.1 Hz, 1H), 4.72 ( d, $J$ = 4.1 Hz, 2H), 4.32 (dd, $J$ = 1.9 Hz, 2H), 4.19-4.15 (m, 5H), 3.78 (s, 3H), 3.77 (s, 3H), 3.55-3.39 (m, 2H), 3.04-2.92 (m, 1H), 2.64-2.55 (m, 1H), 1.60-1.50 (m, 2H). $^{13}C$ NMR (101 MHz, $CDCl_3$): $\delta$ 177.8 (1C), 170.7 (1C), 167.4 (1C), 167.3 (1C), 167.3 (1C), 165.9 (1C), 162.2 (1C), 153.5 (1C), 153.4 (1C), 149.8 (1C), 149.3 (1C), 148.8 (1C), 147.2 (1C), 147.1 (1C), 141.8 (1C), 138.4 (1C), 137.8 (1C), 131.8 (1C), 131.4 (1C), 131.2 (1C), 130.8 (1C), 125.6 (1C), 124.8 (1C), 124.7 (1C), 124.5 (1C), 124.3 (1C), 124.3 (1C), 124.0 (1C), 123.2 (1C), 121.1 (2C), 118.1 (1C), 113.0 (1C), 75.7 (1C), 70.5 (2C), 69.8 (5C), 68.4 (1C), 68.1 (1C), 52.2 (1C), 52.0 (1C), 48.1 (1C), 36.1 (1C), 32.0 (1C). HRMS ($ES^+$): calcd. for $C_{48}H_{42}N_6O_5FeIr$ 1031.2195; found 1031.2220 $[M-PF_6^-]^+$.

B.14. *Complex (**IV-d**)*

From (IV) (0.06 g, 0.12 mmol), silver oxide (0.028 g, 0.12 mmol) and $[(3\text{-COOMe-ppy})_2IrCl]_2$ (0.078 g, 0.06 mmol). Yellow solid (0.045 g, 32%). Anal. Calcd. For $C_{48}H_{42}F_6FeIrN_6O_5P$: C, 49.03; H, 3.60; N, 7.15. Found: C, 49.17; H, 3.42; N, 7.28. $^{1}H$ NMR (400 MHz, $CDCl_3$): $\delta$ 8.39 (d, $J$ = 1.8 Hz, 1H), 8.35 (d, $J$ = 1.7 Hz, 1H), 8.17-7.92 (m, 6H), 7.85 (dd, $J$ = 7.9 Hz, 2H), 7.66 (d, $J$ = 5.6 Hz, 1H), 7.59 (dd, $J$ = 7.8, 1.6 Hz, 1H), 7.54 (d, $J$ = 5.6 Hz, 1H), 7.49 (dd, $J$ = 8.1, 1.7 Hz 1H), 7.35 (d, $J$ = 2.2 Hz, 1H), 7.26-7.15 (m, 2H), 7.11 (dd, $J$ = 6.3 Hz, 1H), 6.48 (d, $J$ = 7.9 Hz, 1H), 6.29 (d, $J$ = 7.8 Hz, 1H), 6.14 (t, $J$ = 6.0 Hz, 1H), 4.78 (s, 2H), 4.38 (s, 2H), 4.24 (s, 5H), 3.88 (s, 3H), 3.86 (s, 3H), 3.51 (t, $J$ = 6.9 Hz, 2H) 2.92-2.85 (m, 2H), 1.59-1.37 (m, 2H). $^{13}C$ NMR (101 MHz, $CDCl_3$): $\delta$ 177.8 (1C), 171.0 (1C), 170.8 (1C), 167.5 (1C), 167.5 (1C), 167.4 (1C), 166.3 (1C), 157.2 (1C), 153.6 (1C), 153.2 (1C), 149.6 (1C), 149.0 (1C), 144.8 (1C), 143.1 (1C), 142.0 (1C), 138.6 (1C), 137.9 (1C), 131.4 (1C), 131.2 (1C), 131.1 (1C), 130.9 (1C), 125.6 (1C), 125.6 (1C), 125.4 (1C), 125.3 (1C), 124.4 (1C), 124.1 (2C), 123.9 (1C), 120.4 (1C), 120.3 (1C), 118.0 (1C), 113.1 (1C), 76.1 (1C), 71.3 (2C), 70.4 (5C), 68.9 (2C), 52.0 (1C), 52.0 (1C), 48.1 (1C), 36.2 (1C), 31.7 (1C). HRMS ($ES^+$): calcd. for $C_{48}H_{42}N_6O_5FeIr$ 1031.2195; found 1031.2218 $[M-PF_6^-]^+$.

B.15. *Complex (**IV-e**)*

From (IV) (0.06 g, 0.12 mmol), silver oxide (0.028 g, 0.12 mmol) and [(p-3-COOMe-ppy)zIrCl]z (0.078 g, 0.06

mmol). Yellow solid (0.056 g, 40%). Anal. Calcd. For $C_{48}H_{42}F_6FeIrN_6O_5P$: C, 49.03; H, 3.60; N, 7.15. Found: C, 49.22; H, 3.64; N, 7.27. $^1H$ NMR (400 MHz, $CDCl_3$): $\delta$ 9.02 (d, $J$ = 2.3 Hz, 1H), 8.96 (d, $J$ = 8.4 Hz, 1H), 8.49 (d, $J$ = 2.2 Hz, 1H), 8.46 (d, $J$ = 1.9 Hz, 1H), 8.34-8.26 (m, 2H,), 8.20 (t, J = 5.6 Hz, 1H), 8.14 (ddd, $J$ = 8.7, 7.6, 1.7 Hz, 1H), 8.04 (d, $J$ = 2.0 Hz, 1H), 8.01 (d, J = 8.7 Hz, 1H), 7.97 (d, $J$ = 8.6 Hz, 1H), 7.80-7.74 (m, 2H), 7.71 (dd, $J$ = 5.5, 1.8 Hz, 1H), 7.26-7.15 (m, 1H), 7.14-6.99 (m, 3H), 6.97-6.91 (m, 1H), 6.38 (dd, $J$ = 7.8, 1.1 Hz, 1H), 6.26 (dd, $J$ = 7.5, 1.3 Hz, 1H), 5.22-5.07 (m, 2H), 4.21 (dd, $J$ = 2.0 Hz, 2H), 4.13 (s, 5H), 3.93-3.78 (m, 7H), 3.56-3.46 (m, 1H), 3.15-3.06 (m, 2H), 1.77-1.65 (m, 2H). $^{13}C$ NMR (101 MHz, $CDCl_3$): $\delta$ 176.7 (1C), 172.5 (1C), 171.1 (1C), 171.0 (1C), 165.1 (1C), 163.6 (1C), 163.2 (1C), 154.5 (1C), 153.9 (1C), 150.4 (1C), 149.9 (1C), 149.6 (1C), 143.0 (1C), 142.0 (1C), 141.1 (1C), 138.7 (1C), 137.8 (1C), 132.3 (1C), 132.0 (1C), 131.4 (1C), 131.1 (1C), 126.5 (1C), 126.3 (1C), 126.0 (1C), 126.0 (1C), 124.7 (1C), 123.8 (1C), 123.5 (1C), 122.5 (1C), 119.3 (1C), 119.1 (2C), 114.5 (1C), 76.3 (1C), 70.2 (1C), 70.2 (1C), 69.6 (5C), 69.2 (1C), 69.0 (1C), 53.1 (1C), 52.8 (1C), 47.7 (1C), 36.0 (1C), 31.7 (1C). HRMS ($ES^+$): calcd. for $C_{48}H_{42}N_6O_5FeIr$ 1031.2195; found 1031.2192 $[M-PF_6^-]^+$.

B.16. *Complex **(IV-f)***

From (**IV**) (0.06 g, 0.12 mmol), silver oxide (0.028 g, 0.12 mmol) and [(p-3-COOH-ppy)zIrCl]z (0.078 g, 0.06 mmol). Yellow solid (0.052 g, 37%). Anal. Calcd. For $C_{46}H_{38}F_6FeIrN_6O_5P$: C, 48.13; H, 3.34; N, 7.32. Found: C, 48.33; H, 3.28; N, 7.25. $^1H$ NMR (400 MHz, MeOD): $\delta$ 8.65 (d, $J$ = 1.8 Hz, 1H), 8.37 (dd, $J$ = 8.4, 1.8 Hz, 1H), 8.32 (dd, $J$ = 8.4, 1.8 Hz, 1H), 8.27 (d, $J$ = 2.2 Hz, 1H), 8.23 (d, $J$ = 1.8 Hz, 1H), 8.20-8.06 (m, 4H), 7.88-7.82 (m, 2H), 7.81 (dd, $J$ = 5.6, 1.6 Hz, 1H), 7.44 (d, $J$ = 2.2 Hz, 1H), 7.28 (ddd, $J$ = 7.1, 5.6, 1.3 Hz, 1H), 7.03 (ddd, $J$ = 7.2, 5.3, 1.3 Hz, 1H), 6.99-6.90 (m, 2H), 6.77 (ddd, $J$ = 7.2, 5.3, 1.3 Hz, 1H), 6.42 (dd, $J$ = 7.7, 1.2 Hz, 1H), 6.27 (dd, $J$ = 7.4, 1.3 Hz, 1H), 4.96-4.90 (m, 1H), 4.81-4.72 (m, 1H), 4.42-4.36 (m, 2H), 4.13 (s, 5H), 3.71-3.58 (m, 1H), 3.53-3.43 (m, 1H), 3.23-3.10 (m, 1H), 2.96-2.85 (m, 1H), 1.81-1.56 (m, 2H). $^{13}C$ NMR (101 MHz, MeOD): $\delta$ 178.9 (1C), 172.1 (1C), 169.9 (1C), 169.1 (1C), 168.8 (1C), 168.4 (1C), 164.1 (1C), 154.8 (1C), 153.7 (1C), 150.2 (1C), 149.6 (1C), 149.6 (1C), 144.1 (1C), 142.5 (1C), 141.1 (1C), 138.5 (1C), 137.7 (1C), 133.4 (1C), 132.5 (1C), 131.0 (1C), 130.9 (1C), 130.5 (1C), 129.9 (1C), 125.1 (1C), 125.0 (1C), 124.0 (1C), 123.7 (1C), 122.7 (1C), 121.2 (1C), 118.8 (1C), 118.4 (1C), 117.6 (1C), 112.6 (1C), 74.9 (1C), 70.6 (1C), 70.3 (1C), 69.4 (5C), 68.3 (1C), 68.1 (1C), 47.5 (1C), 36.3 (1C), 31.7 (1C). HRMS ($ES^+$): calcd. for $C_{46}H_{38}N_6O_5FeIr$ 1003.1882; found 1003.1898 $[M-PF_6^-]^+$.

B.17. *Complex (**IV-g**)*

From (**IV**) (0.06 g, 0.12 mmol), silver oxide (0.028 g, 0.12 mmol) and [(pq)zIrCl]z (0.078 g, 0.06 mmol). Yellow solid (0.070 g, 50%). Anal. Calcd. For $C_{52}H_{42}F_6FeIrN_6OP$: C, 53.84; H, 3.65; N, 7.25. Found: C, 53.77; H, 3.89; N, 7.14. $^1H$ NMR (400 MHz, $CDCl_3$): $\delta$ 8.37 (d, $J$ = 2.1 Hz, 1H), 8.33-8.20 (m, 4H), 8.17 (d, $J$ = 2.1 Hz, 1H), 8.12 (d, $J$ = 8.7 Hz, 1H), 8.09-8.00 (m, 2H), 7.94-7.87 (m, 1H), 7.87-7.80 (m, 1H), 7.79-7.70 (m, 2H), 7.67-7.60 (m, 2H), 7.38-7.22 (m, 3H), 7.13-7.01 (m, 4H), 6.92 (ddd, $J$ = 8.7, 6.8, 1.5 Hz, 1H), 6.83 (ddd, $J$ = 8.5, 6.4, 1.2 Hz, 1H), 6.72 (ddd, $J$ = 8.4, 6.5, 1.3 Hz, 1H), 6.58 (dd, $J$ = 7.9, 1.2 Hz, 1H), 6.28 (dd, $J$ = 7.6, 1.2 Hz, 1H), 5.24-5.15 (m, 2H), 4.25-4.19 (m, 2H), 4.14 (s, 5H), 3.86-3.71 (m, 1H), 3.62-3.48 (m, 1H), 3.1-3.00 (m, 2H), 1.81-1.66 (m, 1H), 1.5-1.40 (m, 1H). $^{13}C$ NMR (101 MHz, $CDCl_3$): $\delta$ 178.5 (1C), 171.2 (1C), 170.9 (1C), 170.7 (1C), 164.5 (1C), 152.9 (1C), 149.5 (1C), 148.3 (1C), 147.1 (1C), 146.7 (1C), 146.7 (1C), 144.4 (1C), 141.1 (1C), 139.8 (1C), 139.2 (1C), 133.2 (1C), 132.6 (1C), 131.5 (1C), 131.1 (1C), 131.1 (1C), 130.9 (1C), 129.1 (1C), 128.9 (1C), 127.6 (1C), 127.5 (1C), 127.2 (1C), 126.9 (1C), 126.7 (1C), 126.6 (1C), 126.5 (1C), 125.2 (1C), 124.7 (1C), 123.3 (1C), 122.8 (1C), 122.1 (1C), 118.1 (1C), 117.7 (1C), 117.5 (1C), 113.5 (1C), 76.4 (1C), 70.2 (1C), 70.2 (1C), 69.6 (5C), 69.2 (1C), 69.0 (1C), 47.1 (1C), 36.3 (1C), 30.6 (1C). HRMS ($ES^+$): calcd. for $C_{52}H_{42}N_6OFeIr$ 1015.2404; found 1015.2403 $[M-PF_6^-]^+$.

B.18. *Complex (**IV-h**)*

From (**IV**) (0.06 g, 0.12 mmol), silver oxide (0.028 g, 0.12 mmol) and $[(piq)_2IrCl]_2$ (0.078 g, 0.06 mmol) in MeCN (10 mL). Yellow solid (0.048 g, 35%). Anal. Calcd. For $C_{52}H_{42}F_6FeIrN_6OP$: C, 53.84; H, 3.65; N, 7.25. Found: C, 53.77; H, 3.89; N, 7.14. $^1H$ NMR (400 MHz, MeOD): $\delta$ 9.08-9.01 (m, 1H), 8.99-8.92 (m, 1H), 8.43 (d, $J$ = 8.1 Hz, 1H), 8.37 (d, $J$ = 2.2 Hz, 1H), 8.29 (d, $J$ = 7.9 Hz, 1H), 8.24 (d, $J$ = 8.4 Hz, 1H), 8.22-8.11 (m, 1H), 8.05-7.97 (m, 2H), 7.88-7.80 (m, 5H), 7.62 (dd, $J$ = 5.6, 1.6 Hz, 1H), 7.58-7.50 (m, 3H), 7.46 (d, $J$ = 6.5 Hz, 1H), 7.33-7.25 (m, 1H), 7.25-7.07 (m, 2H), 6.95 (ddd, $J$ = 8.3, 6.3, 1.1 Hz, 1H), 6.88-6.80 (ddd, $J$ = 8.4, 6.3, 1.1 Hz, 1H), 6.41 (dd, $J$ = 7.7, 1.2 Hz, 1H), 6.25 (dd, $J$ = 7.5, 1.3 Hz, 1H), 4.71 (dd, $J$ = 1.9 Hz, 2H), 4.38 (dd, $J$ = 2.0 Hz, 2H), 4.08 (s, 5H), 3.67-3.41 (m, 2H), 2.95-2.72 (m, 2H), 1.60-1.39 (m, 2H). $^{13}C$ NMR (101 MHz, MeOD): $\delta$ 180.0 (1C), 171.9 (1C), 170.2 (1C), 167.9 (1C), 165.6 (1C), 153.5 (1C), 152.5 (1C), 149.4 (1C), 146.0 (1C), 144.7 (1C), 144.4 (1C), 141.4 (1C), 140.8 (1C), 136.9 (1C), 136.6 (1C), 131.7 (1C), 131.4 (1C), 131.3 (1C), 131.1 (1C), 130.7 (1C), 130.5 (1C), 130.4 (1C), 129.9 (1C), 128.7 (1C), 128.6 (1C), 127.4 (1C), 127.2 (1C), 126.9 (1C), 126.4 (1C), 126.4 (1C), 126.3 (1C), 123.8 (1C), 123.8 (1C), 122.5 (1C), 122.2 (1C), 121.2

(1C), 121.0 (1C), 117.8 (1C), 112.5 (1C), 75.0 (1C), 70.4 (1C), 70.4 (1C), 69.4 (5C), 68.0 (1C), 67.9 (1C), 47.2 (1C), 36.1 (1C), 31.8 (1C). HRMS ($ES^+$): calcd. for $C_{52}H_{42}N_6OFeIr$ 1015.2404; found 1015.2426 $[M-PF_6^-]^+$.

B.19. *Complex **(IV-i)***

From (**IV**) (0.06 g, 0.12 mmol), silver oxide (0.028 g, 0.12 mmol) and [(bzq)zIrCl]z (0.078 g, 0.06 mmol) in MeCN (10 mL). Yellow solid (0.041 g, 31%). Anal. Calcd. For $C_{48}H_{38}F_6FeIrN_6OP$: C, 52.04; H, 3.46; N, 7.59. Found: C, 52.18; H, 3.34; N, 7.72. $^1H$ NMR (400 MHz, $CDCl_3$): $\delta$ 8.96 (s, 1H), 8.88 (d, *J* = 8.2 Hz, 1H), 8.35-8.29 (m, 1H), 8.29-8.21 (m, 3H), 8.02 (t, *J* = 5.3 Hz, 1H), 7.99-7.93 (m, 1H), 7.86-7.79 (m, 3H), 7.72-7.59 (m, 3H), 7.50-7.41 (m, 3H), 7.37 (dd, *J* = 8.0, 5.4 Hz, 1H), 7.19 (dd, *J* = 7.4 Hz, 1H), 7.10 (dd, *J* = 7.6 Hz, 1H), 6.99 (dd, *J* = 6.5 Hz, 1H), 6.38-6.28 (m, 2H), 5.18-5.09 (m, 2H), 4.23-4.17 (m, 2H), 4.10 (s, 5H), 3.59 (t, J = 7.0 Hz, 2H), 2.8-2.64 (m, 2H), 1.33-120 (m, 1H), 0.92-0.79 (m, 1H). $^{13}C$ NMR (101 MHz, $CDCl_3$): $\delta$ 177.1 (1C), 170.8 (1C), 161.1 (1C), 158.4 (1C), 157.2 (1C), 154.2 (1C), 152.0 (1C), 150.0 (1C), 147.1 (1C), 145.0 (1C), 141.5 (1C), 141.2 (1C), 139.9 (1C), 136.9 (1C), 136.1 (1C), 134.4 (1C), 134.3 (1C), 130.4 (1C), 130.3 (1C), 130.1 (1C), 129.8 (1C), 128.9 (1C), 128.3 (1C), 127.4 (1C), 127.1 (1C), 125.5 (1C), 123.6 (1C), 123.4 (1C), 123.4 (1C), 122.8 (1C), 121.5 (1C), 121.0 (1C), 119.8 (1C), 118.7 (1C), 114.0 (1C), 76.3 (1C), 70.2 (2C), 69.6 (5C), 69.1 (2C), 47.8 (1C), 35.9 (1C), 31.7 (1C). HRMS ($ES^+$): calcd. for $C_{48}H_{38}N_6OFeIr$ 963.2086; found 963.2069 $[M-PF_6^-]^+$.

B.20. *Complex **(IV-j)***

From (**IV**) (0.06 g, 0.12 mmol), silver oxide (0.028 g, 0.12 mmol) and [(thpy)zIrCl]z (0.078 g, 0.06 mmol) in MeCN (10 mL). Yellow solid (0.056 g, 40%). Anal. Calcd. For $C_{48}H_{38}F_6FeIrN_6OPS_2$: C, 49.19; H, 3.27; N, 7.17. Found: C, 49.34; H, 3.18; N, 7.09. $^1H$ NMR (400 MHz, $CDCl_3$): $\delta$ 9.24 (d, J = 2.2 Hz, 1H), 9.12 (d, *J* = 8.4 Hz, 1H), 8.38 (d, *J* = 2.1 Hz, 1H), 8.03 (t, *J* = 7.1 Hz, 1H), 7.85-7.72 (m, 6H), 7.72-7.64 (m, 2H), 7.61 (d, *J* = 8.0 Hz, 1H), 7.51 (d, *J* = 5.9 Hz, 1H), 7.21-7.13 (m, 2H), 7.09 (ddd, *J* = 7.4, 5.8 Hz, 1H), 6.97 (ddd, *J* = 7.4, 5.8, 1.5 Hz, 1H), 6.90-6.79 (m, 3H), 6.08 (d, *J* = 8.2 Hz, 1H), 5.95 (d, *J* = 8.1 Hz, 1H), 5.12-5.07 (m, 1H), 5.06-5.01 (m, 1H), 4.23-.17 (m, 2H), 4.08 (s, 5H), 3.74-3.55 (m, 2H), 2.84-2.61 (m, 2H), 1.69-1.54 (m, 1H), 1.32-1.20 (m, 1H). $^{13}C$ NMR (101 MHz, $CDCl_3$): $\delta$ 173.5 (1C), 171.7 (1C), 165.5 (1C), 164.5 (1C), 162.2 (1C), 153.4 (1C), 153.2 (1C), 150.7 (1C), 149.3 (1C), 146.4 (1C), 146.0 (1C), 145.4 (1C), 143.6 (1C), 142.3 (1C), 139.4 (1C), 138.7 (1C), 138.5 (1C), 134.1 (1C), 125.8 (1C), 125.6 (1C), 125.0 (3C), 124.7 (1C), 124.5 (1C), 124.3 (1C), 124.2 (1C), 123.3 (1C), 123.0 (1C), 121.9 (1C), 120.3 (1C), 120.0 (1C), 119.5 (1C), 118.3 (1C), 113.0 (1C), 74.9 (1C), 70.6 (2C), 69.6 (5C), 68.1 (1C), 68.0 (1C), 48.0 (1C), 36.1 (1C), 31.7 (1C). HRMS ($ES^+$): calcd. for $C_{48}H_{38}N_6OS_2FeIr$ 1027.1527; found 1027.1531 $[M-PF_6^-]^+$.

B.21. *Complex **(IV-k)***

From (**IV**) (0.06 g, 0.12 mmol), silver oxide (0.028 g, 0.12 mmol) and [(pbt)zIrCl]z (0.078 g, 0.06 mmol) in MeCN (10 mL). Yellow solid (0.092 g, 66%). Anal. Calcd. For $C_{48}H_{38}F_6FeIrN_6OPS_2$: C, 49.19; H, 3.27; N, 7.17. Found: C, 49.32; H, 3.14; N, 7.11. $^1H$ NMR (400 MHz, $CDCl_3$): $\delta$ 8.99 (s, 1H), 8.95-8.84 (m, 1H), 8.69 (s, 1H), 8.22-8.12 (m, 1H), 8.11-8.00 (m, 1H), 7.94-7.69 (m, 5H), 7.40-7.32 (m, 2H), 7.29-7.26 (m, 1H), 7.16 (dd, *J* = 6.4, 6.3 Hz, 1H), 7.12-7.01 (m, 3H), 6.94 (t, *J* = 7.4 Hz, 1H), 6.82 (t, *J* = 7.5 Hz, 1H), 6.41-6.35 (m, 3H), 6.05 (d, *J* = 8.3 Hz, 1H), 5.25-5.13 (m, 2H), 4.26-4.18 (m, 2H), 4.12 (s, 5H), 3.90-3.75 (m, 1H), 3.72-3.58 (m, 1H), 3.09-2.95 (m, 2H), 1.97-1.75 (m, 2H). $^{13}C$ NMR (101 MHz, $CDCl_3$): $\delta$ 182.6 (1C), 179.8 (1C), 174.5 (1C), 171.0 (1C), 164.2 (1C), 154.4 (1C), 150.0 (1C), 149.8 (1C), 149.1 (1C), 148.9 (1C), 141.8 (1C), 140.7 (1C), 138.9 (1C), 132.7 (1C), 132.5 (1C), 132.3 (1C), 132.1 (1C), 131.3 (1C), 131.0 (1C), 128.6 (1C), 127.9 (1C), 126.7 (1C), 126.3 (2C), 126.1 (1C), 125.8 (1C), 123.6 (1C), 123.5 (1C), 123.3 (1C), 123.3 (1C), 122.7 (1C), 118.6 (1C), 118.6 (1C), 117.5 (1C), 113.9 (1C), 76.3 (1C), 70.2 (2C), 69.6 (5C), 69.2 (1C), 69.1 (1C), 47.8 (1C), 36.2 (1C), 31.1 (1C). HRMS ($ES^+$): calcd. for $C_{48}H_{38}N_6OS_2FeIr$ 1027.1527; found 1027.1517 $[M-PF_6^-]^+$.

B.22. *Complex **(IV-l)***

From **(IV)** (0.06 g, 0.12 mmol), silver oxide (0.028 g, 0.12 mmol) and [(2,4-F-ppy)zIrCl]z (0.078 g, 0.06 mmol). Yellow solid (0.076 g, 56%). Anal. Calcd. For $C_{44}H_{34}F_{10}FeIrN_6OP$: C, 46.69; H, 3.03; N, 7.43. Found: C, 46.54; H, 3.25; N, 7.32. $^1H$ NMR (400 MHz, MeOD): $\delta$ 8.50-8.31 (m, 3H), 8.31-8.18 (m, 2H), 8.11-8.03 (m, 1H), 8.02-7.92 (m, 2H), 7.85-7.79 (m, 1H), 7.77-7.70 (m, 1H), 7.62 (d, *J* = 2.3 Hz, 1H), 7.45-7.40 (m, 1H), 7.27-7.11 (m, 2H), 6.73-6.61 (m, 2H), 5.83 (dd, *J* = 8.7, 2.5 Hz, 1H), 5.65 (dd, *J* = 8.0, 2.4 Hz, 1H), 4.80-4.71 (m, 2H), 4.42 (dd, *J* = 2.0 Hz, 2H), 4.17 (s, 5H), 3.85-3.57 (m, 2H), 3.10-2.81 (m, 2H), 1.64-1.51 (m, 2H).$^{13}C$ NMR (101 MHz, MeOD): $\delta$ 176.4 (1C), 172.0 (1C), 168.0 (1C), 164.7 (d, *J* = 7.0 Hz, 1C), 164.4 (dd, *J* = 257, 12 Hz, 1C), 163.5 (dd, *J* = 236, 13 Hz, 1C), 162.2 (d, *J* = 7 Hz, 1C), 161.6 (dd, *J* = 240, 11 Hz, 1C), 161.8 (dd, *J* = 260, 13 Hz, 1C), 153.6 (1C), 153.4 (1C), 153.0 (d, *J* = 7 Hz, 1C), 149.6 (1C), 149.5 (1C), 142.1 (1C), 139.1 (1C), 138.5 (1C), 127.9 (1C), 127.1 (1C), 124.5 (1C), 124.4 (1C), 124.2 (1C), 123.8

(1C), 123.5 (1C), 123.3 (1C), 118.2 (1C), 113.0 (1C), 112.7 (dd, *J* = 25, 3 Hz, 1C), 112.6 (dd, *J* = 25, 3 Hz, 1C), 99.0 (t, *J* = 27 Hz, 1C), 97.6 (t, *J* = 27 Hz, 1C), 75.1 (1C), 70.5 (2C), 69.5 (5C), 68.2 (1C), 68.1 (1C), 47.8 (1C), 36.1 (1C), 31.7 (1C). HRMS ($ES^+$): calcd. for $C_{44}H_{34}N_6OF_4FeIr$ 987.1709; found 987.1716 $[M-PF_6^-]^+$.

## II. Properties of the complexes according to the invention.

### II.1. Lipophilicity of vectorized iridium(III) complexes.

#### A. Lipophilicity measurement.

The octanol-water partition coefficients (P) of all the complexes were determined using a shake-flask method[8]. Water (100 mL, distilled after milli-Q purification) and n-octanol (100 mL, Sigma Aldrich, ACS spectrophotometric grade, ≥ 99%) were shaken together for 24 h to allow saturation of both phases. Stock solutions of the compounds (50 μM) were prepared in the aqueous phase. Aliquots (2 mL) of each of these stock solutions were then added to an equal volume of the n-octanol phase. The resultant biphasic solution was mixed for 15 min and then centrifuged (3000 tr/min, 5 min) to separate the phases. The concentrations of the complex in the organic and aqueous phases were then determined using UV absorbance spectroscopy (260 nm). The blank is the water saturated with n-octanol. Log P was defined as logarithm of the ratio of the concentrations of the complex in the organic and aqueous phases (values reported are the means of at least three separated determinations).

$$Log\ P = \frac{[Ir]_{(org)}}{[Ir]_{(aq)}}$$

#### B. Result on lipophilicity.

The lipophilicity, which refers to the partition coefficient of the compound in n-octanol/water, has been measured for all the vectorized iridium(III) complexes (Table 3). The Log *P* values have been determined by a classical shake-flask method [8]. The Log *P* values of iridium(III) complexes range from -0.78 to 1.26 for biotin vectorized iridium(III) complexes, 1.08 to 2.06 for FTS vectorized iridium(III) complexes, -0.52 to 0.88 for PSMA vectorized iridium(III) complexes, and 0.61 to 2.73 for Fc vectorized iridium(III) complexes. In a given family of vectorized iridium(III) complexes, the lipophilicity is mainly regulated by modification of the structures of the C^N ligands. As expected, the complexes containing deprotonated 3- or 4-COOMe substituted phenylpyridine ligands and p-3-COOH substituted phenylpyridine ligand are the most hydrophilic. In contrary, the complexes with extended π-conjugation ligands, in particular containing deprotonated 2-phenylquinoline, 1-phenylisoquinoline and 7,8-benzoquinoline ligands or containing F groups show lipophilic character, the most lipophilic one being (**II-l**) with a Log P value of 2.06.

Table 3 : Lipophilicity of vectorized iridium(III) complexes (**I-a**) to (**I-l**), (**II-a-e**) and (**II-g-l**), (**III-a-b**), (**III-d-e**) and (**III-g-l**) and (**IV-a**) and (**IV-l**).

| Entry | Complex | Log *P* | Entry | Complex | Log *P* |
|---|---|---|---|---|---|
| 1 | (I-a) | 0.09±0.07 | 24 | (III-a) | -0.52±0.04 |
| 2 | (I-b) | 0.43±0.01 | 25 | (III-b) | -0.14±0.04 |
| 3 | (I-c) | -0.78 ±0.01 | 26 | (III-d) | -0.52±0.01 |
| 4 | (I-d) | -0.10±0.01 | 27 | (III-e) | -033±0.03 |
| 5 | (I-e) | 0.25±0.04 | 28 | (III-g) | 0.33±0.01 |
| 6 | (I-f) | -0.68±0.07 | 29 | (III-h) | 0.88±0.02 |
| 7 | (I-g) | 1.26±0.04 | 30 | (III-i) | 0.60±0.01 |
| 8 | (I-h) | 0.87±0.02 | 31 | (III-j) | -0.38±0.03 |
| 9 | (I-i) | 0.60±0.06 | 32 | (III-k) | -0.14±0.01 |
| 10 | (I-j) | 0.24±0.05 | 33 | (III-l) | 0.29±0.05 |
| 11 | (I-k) | 0.87±0.03 | 34 | (IV-a) | 0.61±0.07 |
| 12 | (I-l) | 0.50±0.02 | 35 | (IV-b) | 1.63±0.02 |
| 13 | (II-a) | 1.70±0.08 | 36 | (IV-c) | 0.72±0.03 |
| 14 | (II-b) | 1.68 ±0.02 | 37 | (IV-d) | 0.77±0.01 |

(continued)

| (**II-g-l**), (**III-a-b**), (**III-d-e**) and (**III-g-l**) and (**IV-a**) and (**IV-l**). | | | | | |
|---|---|---|---|---|---|
| Entry | Complex | Log *P* | Entry | Complex | Log *P* |
| 15 | (II-c) | 1.08 ±0.01 | 38 | (IV-e) | 1.37±0.05 |
| 16 | (II-d) | 1.40±0.02 | 39 | (IV-f) | 1.08±0.03 |
| 17 | (II-e) | 1.30±0.02 | 40 | (IV-g) | 1.42±0.06 |
| 18 | (II-g) | 1.95±0.05 | 41 | (IV-h) | 0.81±0.07 |
| 19 | (II-h) | 2.06±0.07 | 42 | (IV-i) | 0.74±0.02 |
| 20 | (II-i) | 1.82±0.01 | 43 | (IV-j) | 1.07±0.07 |
| 21 | (II-j) | 1.94±0.06 | 44 | (IV-k) | 0.74±0.05 |
| 22 | (II-k) | 1.60±0.01 | 45 | (IV-l) | 1.14±0.02 |
| 23 | (**II-l**) | 2.06±0.06 | | | |

II.2. Photophysical properties of vectorized iridium(III) complexes.

A. Photophysical mesurement.

UV/Vis absorption spectra were recorded at RT on solutions contained in quartz cuvettes (optical pathlength 1 cm, Hellma®) with a PerkinElmer® UV/VIS/NIR spectrophotometer Lambda 950 and a PerkinElmer® detector UV/VIS/NIR Accessory 2D Detector Module.

Fluorescence and phosphorescence emission spectra were obtained with a HORIBA JOBIN YVON, fluromax-4 and spectrofluorometer.

Luminescence quantum yields were measured according to the method of Demas and Crosby [9], on solution samples at RT. The quantum yield is defined as:

$$\Phi_P = \Phi_R * \frac{(A_s)}{(A_r)} * \frac{(\eta_s)}{(\eta_r)^2}^2$$

Where $\Phi_P$ is the quantum yield of the sample, $\Phi_R$ is the know quantum yield of the reference standard. At the area subtended by the emission band (on a wavelength scale) and η is the refractive index of the solvent used for the preparation of the solution. $A_s$ (related to the sample) and $A_r$ (related to the reference) must be excitation wavelength. Different standards were selected depending on the spectral region of the interest: POPOP in hexane (Φ = 97%) and rhodamine B in ethanol (Φ = 65%).

Fluorescence lifetime measurements were performed by an Edinburgh FLS920 spectrofluorimeter equipped with a TCC900 card for data acquisition in time-correlated single-photon counting experiments (0.5 ns time resolution) with a D2 lamp and an LDH-P-C-405 pulsed diode laser. The estimated experimental error is 2 nm on the band maximum (5% on the lifetime, 10% on the fluorescence quantum yield).

B. Result on photophysical properties.

The absorption spectra of vectorized iridium(III) complexes were determined in $CH_2Cl_2$ or in $CH_2Cl_2$/MeOH (1:1) solutions at room temperature (Table 4). All the complexes displayed intense absorption bands below 300 nm, which can be assigned to spin-allowed $^1\pi \rightarrow \pi^*$ (C^N) electronic ligand-centered ($^1$LC) transitions [10-15]. The structureless bands at 300-360 nm were attributed both to phenyl-to-pyridine π-π* (C^N) ligand-to-ligand charge transfer ($^1$LLCT) and metal-to-ligand charge transfer dπ(Ir)→π*(C^N) with a predominant singlet spin multiplicity ($^1$MLCT)[9-14]. The weak and broad lowest-lying bands in the visible region over 360 nm corresponds to both singlet and triplet metal-to-ligand charge transfer dπ(Ir)→π*(C^N) ($^1$MLCT and $^3$MLCT) transitions [10-15]. In this area, the complexes bearing C^N ligands such as 2,4-F-ppy (complexes (**I-l**), (**II-l**), (**III-l**), (**IV-l**)) show maximum absorption ranging from 356-361 nm, 3-COOMe-ppy (complexes (**I-d**), (**II-d**), (**III-d**), (**IV-d**)) show maximum absorption around 370 nm, and ppy (complexes ((**I-a**), (**II-a**), (**III-a**), (**IV-a**)) and 4-Me-ppy (complexes (**I-b**), (**II-b**), (**III-b**), **(IV-b))** show maximum absorption around 380 nm. the complexes bearing C^N ligands such as 4-COOMe-ppy (complexes (**I-c**), (**II-c**), (**III-c**), **(IV-c)),** bzq (complexes (**I-i**), (**II-i**), (**III-i**), **(IV-i))** and pbt (complexes (**I-k**), (**II-k**), (**III-k**), **(IV-k))** show maximum absorption ranging from 398-413 nm. The complexes with C^N ligands such as p-3-COOMe-ppy (complexes (**I-e**), (**II-e**), (**III-e**), **(IV-e)),** pq (complexes (**I-**

**g**), (**II-g**), (**III-g**), **(IV-g)),** piq (complexes (**I-h**), (**II-h**), (**III-h**), **(IV-h)),** and thpy (complexes (**I-j**), (**II-j**), (**III-j**), **(IV-j))** exhibit a maximum ranging from 428-438 nm absorption in the visible region corresponding to the blue area. The relatively weak ferrocene-based bands that generally appear around 450 nm and correspond to spin allowed d-d transitions are masked by the MLCT ones.

Table 4 : Selected UV-visible properties of vectorized iridium(III) complexes (**I-a-e**) to (**I-g-l**), (**II-a-e**) and (**II-g-l**), **(III-a-b**), (**III-d-e**) and (**III-g-l**) and **(IV-a)** and (**IV-l**).

| Entry | Complex | $\lambda_{max}$ (nm)[a] $\varepsilon$ ($M^{-1}.cm^{-1}$) |
|---|---|---|
| 1 | (I-a) | 308 (sh) (16690), 380 (5560) |
| 2 | (I-b) | 314 (sh) (18240), 382 (6150) |
| 3 | (I-c) | 310 (sh) (16860), 358 (4090), 400 (3295) |
| 4 | (I-d) | 373 (4260) |
| 5 | (I-e) | 310 (sh) (19500), 365 (sh) (4690), 432 (4155) |
| 6 | (I-g) | 336 (19000), 437 (4600) |
| 7 | (I-h) | 337 (sh) (12520), 352 (sh) (11890), 380 (sh) (8100), 436 (4855) |
| 8 | (I-i) | 320 (13840), 410 (3920) |
| 9 | (I-j) | 324 (sh) (15070), 342 (sh) (13570), 430 (5960) |
| 10 | (I-k) | 321 (25835), 372 (sh) (7480), 406 (6130) |
| 11 | (I-l) | 300 (sh) (14510), 359 (4820) |
| 12 | (II-a) | 308 (sh) (14540), 380 (4505) |
| 13 | (II-b) | 306 (sh) (15875), 382 (4560) |
| 14 | (II-c) | 310 (sh) (21710), 355 (5105), 398 (3925) |
| 15 | (II-d) | 372 (4535) |
| 16 | (II-e) | 310 (sh) (24540), 363 (sh) (5975), 432 (5280) |
| 17 | (II-g) | 333 (20560), 438 (4735) |
| 18 | (II-h) | 332 (sh) (12380), 353 (sh) (11525), 380 (sh) (8040), 436 (4810) |
| 19 | (II-i) | 315 (16900), 412 (4615) |
| 20 | (II-j) | 324 (16000), 341 (sh) (14300), 430 (6205) |
| 21 | (II-k) | 321 (32250), 372 (sh) (9520), 406 (7855) |
| 22 | **(II-l)** | 300 (sh) (21325), 361 (6610) |
| 24 | (III-a) | 303 (sh) (37760), 380 (14610), 462 (sh) (570) |
| 25 | (III-b) | 310 (sh) (23310), 379 (7615) |
| 26 | (III-d) | 370 (13650), 452 (sh) (565) |
| 27 | (III-e) | 308 (sh) (35630), 363 (sh) (8950), 429 (8065) |
| 28 | (III-g) | 334 (18100), 436 (4280) |
| 29 | (III-h) | 336 (sh) (23695), 352 (sh) (22270), 378 (sh) (15520), 435 (9310) |
| 30 | (III-i) | 317 (19830), 412 (5510) |
| 31 | (III-j) | 324 (26900), 343 (sh)[b] (24560), 428 (10800) |
| 32 | (III-k) | 321 (39070), 370 (sh) (12560), 406 (10170) |
| 33 | (III-l) | 300 (sh) (17230), 360 (5740) |
| 34 | (IV-a) | 304 (sh) (25840), 381 (8080) |
| 35 | (IV-b) | 308 (sh) (27820), 379 (8620) |
| 36 | (IV-c) | 304 (sh) (28570), 353 (sh) (7050), 399 (5315), 427 (sh) (3660) |
| 37 | (IV-d) | 335 (sh) (9725), 372 (6540) |
| 38 | (IV-e) | 312 (sh) (27925), 363 (sh) (7290), 434 (6530) |
| 39 | (IV-f) | 304 (sh) (22560), 410 (4150) |
| 40 | (IV-g) | 334 (17135), 436 (4150) |

(continued)

| Entry | Complex | $\lambda_{max}$ (nm)[a] $\varepsilon$ ($M^{-1}.cm^{-1}$) |
|---|---|---|
| 41 | (IV-h) | 334 (sh) (19440), 351 (sh) (18355), 380 (12745), 435 (7870) |
| 42 | (IV-i) | 318 (25600), 413 (7410) |
| 43 | (IV-j) | 322 (15400), 342 (sh) (14040), 434 (6150) |
| 44 | (IV-k) | 321 (24910), 371 (sh) (7200), 407 (5940) |
| 45 | (IV-l) | 305 (sh) (21345), 356 (sh) (8645), 412 (3395) |

[a] UV-Visible spectra were recorded at room temperature in air-saturated $CH_2Cl_2$ solutions (50 $\mu$M) for complexes (**I-a-e**) and (**I-g-l**), (**II-a-e**) and (**II-g-l**) and (**IV-a-e**) and (**IV-g-l**), and in $CH_2Cl_2$/MeOH (1:1) solutions (50 $\mu$M) for complexes (**III-a-b**), (**III-d-e**) and (**III-g-l**) and (**IV-f**).
[b] (sh) for shoulder.

The luminescence spectra of vectorized iridium(III) complexes were determined in $CH_2Cl_2$ at room temperature (Table 3). When excited at 365 nm, biotin- or FTS-vectorized iridium complexes (**I-a**), (**II-a**), (**I-b**), (**II-b**), (**I-d**)**,** (**II-d** and (**I-l**), (**II-l**) exhibit blue-green phosphorescent emission with two maximum wavelengths ranging from 452-475 nm to 478-505 nm; biotin- or FTS-vectorized iridium complexes (**II-e**), (**I-c**), (**II-c**), (**II-i**) and (**I-k**), (**II-k**) display green to orange phosphorescent emission with two maximum wavelengths from 500-524 nm to 534-558 nm, except complexes (**I-e**) and (**I-i**) which show a broad band from 511 to 550 nm and from 522 to 567 nm, respectively. When excited at 470 nm, biotin- or FTS-vectorized iridium complexes (**II-h**) and (**I-j**), (**II-j**) display orange to red phosphorescent emission with two maximum wavelengths ranging from 586-593 nm to 626-633 nm and complexes (**I-g**), (**II-g**), (**I-h**) show a broad band from 593 to 652 nm and from 554 to 637 nm, respectively.

When excited at 365 nm, PSMA-vectorized iridium complex (**III-l**) displays blue phosphorescent emission with two maximum wavelengths at 453 to 479 nm, PSMA-vectorized complexes (**III-b**) and (**III-d**) exhibit blue-green phosphorescent emission with three maximum wavelengths ranging from 476 to 546 nm for (**III-b**) and one maximum wavelength at 498 nm for (**III-d**), PSMA-vectorized complexes (**III-a**) and (**III-e**) display green phosphorescent emission with one maximum wavelength ranging from 528 to 542 nm, and PSMA-vectorized complex (**III-k**) display green phosphorescent emission with two maximum wavelengths at 520 to 558 nm. When excited at 470 nm, PSMA-vectorized complex (**III-i**) displays green-yellow phosphorescent emission with one maximum wavelength at 548 nm, PSMA-vectorized complex (**III-j**) displays yellow-orange phosphorescent emission with two maximum wavelengths at 584 to 635 nm, and PSMA-vectorized complexes (**III-g**) and (**III-h**) exhibit orange phosphorescent emission with one maximum wavelength at 593 and 628 nm, respectively.

When excited at 365 nm, Fc-vectorized iridium complexes **(IV-a), (IV-b), (IV-d),** and (**IV-l**) exhibit blue-green phosphorescence emission with two maximum wavelengths ranging from 461-475 nm to 490-536 nm for **(IV-a),** (**I-Vb**), and **(IV-d)** and four maximum wavelengths at 452, 477, 515 and 547 nm for (**IV-l**), whereas Fc-vectorized iridium complexes **(IV-c), (IV-e), (IV-f), (IV-k)** display green phosphorescent emissions with two maximum wavelengths ranging from 445-518 nm to 536-558 nm for **(IV-c), (IV-f), (IV-k)** and with one maximum wavelength at 528 nm for complex (IV-e). When excited at 470 nm, Fc-vectorized complexes **(IV-g), (IV-h), (IV-i),** and **(IV-j)** show orange phosphorescent emission with two maximum wavelengths ranging from 574-615 nm to 626-668 nm for complexes (**IV-h**), (**IV-i**), and (**IV-j**) and one maximum wavelength at 579 nm for complex (IV-g).

In addition, the relative emission quantum yields ($\Phi_P$) of iridium complexes (**I-a-e**), (**I-i**), (**I-k**), (**II-a-e**), (**II-i**), (**II-k**), (**II-l**), (**III-a-b**), (**III-d-e**), (**III-k-l**) and **(IV-a)** to (**IV-l**) were measured at room temperature by using 1,4-bis(5-phenyloxazole-2-yl)benzene (POPOP) in hexane as a reference standard ($\Phi_P$ = 97%), and those of complexes (**I-g-h**), (**I-j**), (**II-g-h**), (**II-j**), (**III-g-j**) were measured by using rhodamine B in ethanol as a reference standard ($\Phi_P$ = 65%). The quantum yields of biotin vectorized complexes (**I-a-b**), (**I-d**)**,** (**I-g**), (**I-i-j**), (**I-l**) are low (below 1%); for the biotin vectorized complexes (**I-c**), (**I-e**), (**I-h**), (**I-k**), they are higher with values of 5.9, 3.7, 3.0, and 7.0% respectively (Table 5). The quantum yields of FTS-vectorized complexes (**II-a-b**), (**II-d**), (**II-g**), (**II-j**), (**II-l**) are below 1%; for the FTS-vectorized complexes (**II-c**), (**II-e**), (**II-h**), (**II-k**) they are higher with values of 4.6, 4.6, 3.0, and 6.1%, respectively (Table 2). The quantum yields of PSMA-vectorized complexes (**III-g**) and (**III-i**) were below 1%, while PSMA-vectorized complexes (**III-a**), (**III-e**), (**III-h**), (**III-j**), and (**III-k**) gave values as high as 9.6, 4.0, 3.0, 3.0, and 6.1%, respectively (Table 5). The quantum yields of all the Fc-vectorized complexes **(IV-a)** to (**IV-l**) are below 1% (Table 5).

The emission lifetime of all iridium complexes, which refer to the average time that the compound stays in its excited state before emitting a photon, are showed in Table 4. The emission lifetimes of biotin-vectorized complexes (**I-**

**a**) to (**I-l**) range from 138 ns to 411 ns, among them, the lifetime of biotin-vectorized complexes (**I-c**), (**I-e**), (**I-k**) are 411, 224, and 322 ns respectively. The emission lifetimes of FTS-vectorized complexes (**II-a**) to (**II-l**) range from 167 ns to 486 ns, among them, the lifetime of FTS-vectorized complexes **(II-c)** and (**II-k**) are 319 and 486 ns respectively. The emission lifetimes of PSMA-vectorized complexes (**III-a**) to (**III-l**) are in the range of 159-649 ns, among them, the lifetime of complexes (**III-a**) and (**III-k**) were 248 and 649 ns, respectively. The emission lifetimes of Fc-vectorized complexes **(IV-a)** to (**IV-l**) are in the range of 178-228 ns.

Table 5 : Photophysical properties of vectorized iridium(III) complexes (**I-a-e**) to (**I-g-l**), (**II-a-e**) and (**II-g-l**)**,** (**III-a-b**), (**III-d-e**) and (**III-g-l**) and **(IV-a)** to (**IV-l**).

| Complex | $\lambda_{em}$(nm)[a] | $\Phi_P$(%)[b] | $\tau_1$(ns) | Complex | $\lambda_{em}$(nm)[a] | $\Phi_P$( %)[b] | $\tau_1$(ns ) |
|---|---|---|---|---|---|---|---|
| (I-a) | 472,501 | 0.5 | 179 | (III-a) | 528 | 9.6 | 248 |
| (I-b) | 475,505 | 0.5 | 177 | (III-b) | 476,516,546 | 1.8 | 194 |
| (I-c) | 502, 535 | 5.9 | 411 | (III-d) | 498 | 2.1 | 219 |
| (I-d) | 461,490 | 0.7 | 184 | (III-e) | 542 | 4.0 | 208 |
| (I-e) | 511-550 | 3.7 | 224 | (III-g) | 593 | 0.1 | 191 |
| (I-g) | 593-652 | <0.1 | 203 | (III-h) | 628 | 3.0 | 215 |
| (I-h) | 589, 627 | 3.0 | 202 | (III-i) | 548 | 0.3 | 227 |
| (I-i) | 522-567 | <0.1 | 210 | (III-j) | 584,635 | 3.0 | 197 |
| (I-j) | 586, 633 | 1.0 | 214 | (III-k) | 520, 558 | 6.1 | 649 |
| (I-k) | 518, 555 | 7.0 | 322 | (III-l) | 453,479 | 1.7 | 159 |
| (I-l) | 453, 480 | 1.0 | 138 | (IV-a) | 473,499 | 0.04 | 197 |
| (II-a) | 472, 500 | 0.5 | 210 | (IV-b) | 475, 502 | 0.03 | 197 |
| (II-b) | 475, 503 | 0.4 | 167 | (IV-c) | 504, 536 | 0.04 | 195 |
| (II-c) | 500, 534 | 4.6 | 319 | (IV-d) | 463, 491 | 0.03 | 228 |
| (II-d) | 461,489 | 0.9 | 201 | (IV-e) | 528 | 0.17 | 200 |
| (II-e) | 514, 533 | 4.6 | 182 | (IV-f) | 445, 536 | 0.12 | 202 |
| (II-h) | 554-637 | <0.1 | 185 | (IV-g) | 579 | 0.02 | 214 |
| (II-i) | 588, 629 | 3.0 | 199 | (IV-h) | 588,626 | 0.07 | 178 |
| (II-j) | 524, 558 | 0.1 | 215 | (IV-i) | 615, 668 | 0.02 | 205 |
| (II-k) | 586, 632 | 1.0 | 206 | (IV-j) | 574, 648 | 0.01 | 196 |
| (**II-l**) | 517,556 | 6.1 | 486 | (IV-k) | 518, 558 | 0.08 | 212 |
| (II-h) | 452,478 | 0.8 | 209 | (IV-l) | 452,477, 515, 547 | 0.01 | 191 |

[a] $\lambda_{em}$ were measured in $CH_2Cl_2$ at room temperature ($\lambda_{exc}$ = 365 nm for complexes (**I-a-e**), (**I-i**), (**I-k**), (**II-a-e**), (**II-i**), (**II-k**), (**III-a-e**), (**III-k-l**) and (**IV-a-l**), 470 nm for complexes (**I-g-h**), (**I-j**), (**II-g-h**), (**II-j**), and (**III-g-j**).
[b] The emission phosphorescence quantum yields ($\Phi_p$) were determined at room temperature using POPOP in hexane ($\Phi_p$ = 97%) as the reference for complexes (**I-a-e**), (**I-i**), (**I-k**), (**II-a-e**), (**II-i**), (**II-k**), (**III-a-e**), (**III-k-l**) and (**IV-a-m**), and using Rhodamine B in ethanol ($\Phi_p$ = 65%) as the reference (**I-g-h**), (**I-j**), (**II-g-h**), (**II-j**), and (**III-g-j**).

II.3. Singlet oxygen ($^1O_2$) production of vectorized iridium(III) complexes. A. Singlet Oxygen Measurements.

The detection of $^1O_2$ generation of iridium complexes was measured with 9,10-anthracenediyl-bis(methylene)dimalonic acid (ABDA) as an $^1O_2$ indicator and $Ru(bpy)_3Cl_2$ as a standard according to a modified reported method [16,17]. Experiments were conducted using ABDA (50 μM) in phosphate buffered solution (PBS, pH 7.4), the respective iridium complex, i.e. (**I-e**), (**I-g**), (**I-h**), (**I-j**), (**II-e**), (**II-g**), (**II-h**), (**II-j**), (**III-i**), (**III-j**), (**III-k**), (**III-h**), **(IV-i), (IV-j), (IV-k), (IV-h)** and $Ru(bpy)_3Cl_2$ (5 μM) in a mixture of $H_2O/CH_3CN$ (50/50) with a ratio ABDA/complex of 1/10 and a final volume of 3 mL. ABDA oxidation was monitored by UV-vis spectrophotometry. The samples were irradiated with a blue custom-made LED plate (458 nm, 4.5 $mWcm^{-2}$). During irradiation UV-vis absorption spectra were recorded at 5, 10, 15, 20, 30 and 45 min. The $^1O_2$ generation was followed by monitoring the absorption spectral change at 380 nm of ABDA versus time.

B. Detection of singlet oxygen ($^1O_2$) production.

The ability of vectorized iridium complexes to produce singlet oxygen $^1O_2$ upon light irradiation (blue custom-made LED plate (458 nm, 4.5 $mWcm^{-2}$) was examined using the commonly applied singlet oxygen detection agent ABDA in PBS [16,17]. ABDA selectively reacts with $^1O_2$ to give an endoperoxide which loses the characteristic anthracene absorption bands at around 350 to 410 nm. In order to evaluate the singlet oxygen production, the decrease of the ABDA absorption band at 380 nm was plotted versus time at 5, 10, 15, 20, 30 and 45 min. Upon irradiation with blue light, complexes (**I-j**), (**III-h**), (**III-j**) and (IV-j) show fast depletion of the ABDA absorbance at 380 nm with a fast complete consumption of ABDA for complexes (**III-j**) and (IV-j) (Figure 1).

**III. Cytotoxicity and photodynamic of the complexes** according **to the invention.**

III.1. Material and Methods.

A. Cell culture.

Cells were grown in humidified incubators at 37°C in 5% $CO_2$. Murine MC3T3 osteoblast and NIH-3T3 fibroblast cells - used as non-tumor cell models - were cultured in α-MEM medium and DMEM medium supplemented with 10% fetal bovine serum (FBS) and 1% Penicillin/Streptomycin (P/S) antibiotics, respectively. Human T24 bladder, MGHU3 bladder, Scaber bladder, A549 lung, MCF7 breast, MiaPaCa2 pancreas cancer cells were cultured in DMEM medium supplemented with 10% FBS and 1% P/S antibiotics. Human ASPC-1 pancreas, PC-3 prostate, DU-145 prostate, C4-2B prostate and 22rV1 prostate cancer cells were cultured in RPMI medium supplemented with 10% FBS and 1% P/S antibiotics. Human HeLa cervix and HepG2 liver cancer cells were cultured EMEM medium supplemented with 10% FBS and 1% P/S antibiotics.

B. Cell Viability assay (MTT assay).

MTT reagent (3-(4,5-dimethylthiazol-2-yl)-2,5-diphenyltetrazolium bromide) was used to determine cell death as originally described by Mosmann [18]. Briefly, cells were seeded from 5,000 to 10,000 cells/well in 24-well plates (1 mL) depending on the cell type and allowed to attach overnight. The complexes were prepared in DMSO. The concentration of the complexes was calculated based on the elemental composition of the complexes determined by the elemental analyses. All cell viability experiments were carried out with a fixed concentration of 0.1% (v/v) DMSO. After 72 h of treatment, cells were incubated at 37°C and 5% $CO_2$ with 25 μL of MTT solution (5 mg/ml; Sigma-Aldrich) in 24-well plates for 3 to 4 h. After solubilization with 500 μL of DMSO, formazan was quantified spectrophotometrically with a microplate reader at 570 nm absorbance (ClarioSTAR, BMG LabTech). $GI_{50}$ values corresponding to the concentration that causes 50% inhibition of cell proliferation were calculated from dose-response curves obtained by nonlinear regression analysis (Prism 9.0, Graphpad Software). All the results were calculated from data obtained in at least three independent experiments performed in triplicate.

C. Treatment conditions.

Irradiation was conducted using an in-house device, a series of blue light LEDs selected according to the absorption spectra of the compounds at 458 nm wavelength with an emitted power of 9.6 Watts. The light fluence was measured at 458 nm with an optical power meter Thorlabs PM100A at different distances between the cell layer and the light source (5 and 10 cm). The data are presented in Figure 2A.

A series of cell viability experiments were performed to evaluate the cytotoxic effect of light itself in three cell line models (non-tumor NIH-3T3, PC3 prostate cancer and T24 bladder cancer). For this purpose, cells were irradiated or not at 458 nm for different times (5 and 10 min) at different distances between the cell layer and the light source (5 and 10 cm). Cell viability was measured after 72 h of incubation. As shown in Figure 2B, minimal toxicity was observed for a distance of 10 cm between the light source and the cells, with no significant difference in toxicity between 5 or 10 min of irradiation.

Based on these results, all further experiments were conducted with the following irradiation conditions: 10 min of irradiation at a distance of 10 cm. Two sets of experiments were conducted in parallel for each complex to be studied:

1. The cells were incubated with the different complexes (in the dark) for 72 h.
2. The cells were incubated for 8 h with the different complexes (in the dark), then irradiated for 10 min, followed by an incubation in the dark up to 72 h.

III.2. Cytotoxicity and photodynamic of the series of biotin-vectorized compounds.

Biotin is an essential B vitamin that acts as an important coenzyme in carbohydrate and lipid metabolism. With their rapid growth, cancer cells exhibit high metabolic activity, which is accompanied by a high utilization of essential vitamins. Consequently, receptors and/or transporters involved in the absorption of vitamins are overexpressed on the surface of cancer cells. For biotin, its cellular uptake is mediated by the sodium dependent multivitamin transporter also known as "SMVT" that is responsible for the translocation of hydrosoluble vitamins (pantothenic acid or Vit B5, Biotin or Vitamin B7 and $\alpha$-lipoic acid). A number of studies have taken advantage of the overexpression of SMVT in a number of cancer cells and the simple structure of biotin to design effective tumor-targeting molecules conjugated to biotin, notably chemotherapeutic agents that showed superior cellular uptake and increased specificity.

The cytotoxicity and photodynamic activities of the eleven biotin-vectorized compounds were first examined in two cell models (non-tumor fibroblastic NIH-3T3 and T24 bladder cancer). $GI_{50}$ values under dark and light irradiation conditions are summarized in Table 6. In the dark, the complexes showed no toxicity toward NIH-3T3 cells with $GI_{50}$ values ranging from 67 to over 250 $\mu$M), and showed moderate to no toxicity ($GI_{50}$ values ranging from 11.5 to 96 $\mu$M) against T24 cells. Complexes (**I-a**), (**I-b**), (**I-c**), (**I-d**), (**I-i**), and (**I-l**) did not show marked photosensitizing effects on tumor cells as shown in Table 7 (PI values ranging from 1.0 to 8.2, median = 1.4-fold, mean = 2.4-fold) in contrast to complexes (**I-e**), (**I-g**), (**I-h**), (**I-j**), and (**I-k**) (PI values ranging from 16.9 to 24.0, median = 19.3-fold, mean = 19.6-fold). Therefore, only complexes (**I-e**), (**I-g**), (**I-h**), (**I-j**), and (**I-k**) were evaluated in an additional non-tumor cell line (osteoblastic MC3T3) and five additional cancer cell lines (Scaber bladder, MGHU3 bladder, A549 lung, MCF7 breast and HeLa cervix). In the dark (Table 6), these complexes showed no toxicity toward non-tumor MC3T3 cells ($GI_{50}$ values ranging from 25.8 to 56.8 $\mu$M). Complex (**I-e**) showed no activity in the dark on all tumor cell lines tested ($GI_{50}$ values ranging from 96.4 to 221 $\mu$M) and its selectivity was low (SI values ranging from <1 *vs.* MC3T3 to >2.6 *vs.* NIH-3T3) (Table 6). In some cancer cell models (including Scaber and MGHU3 bladder, and HeLa cervix), complexes (**I-j**) and (**I-k**) had higher $GI_{50}$ values than in non-tumor MC3T3 cells (Table 6). Complexes (**I-g**) ($GI_{50}$ values ranging from 9.1 to 25.4 $\mu$M) and (**I-h**) ($GI_{50}$ values ranging from 11.9 to 26.7 $\mu$M) showed a better selectivity (SI values ranging from 3.8 *vs.* MC3T3 to 7.6 *vs* NIH-3T3 for I-g and from 2.6 *vs.* MC3T3 to 6.3 *vs* NIH-3T3 for I-h) in all cancer cell models (with the exception of HeLa cervix) compared with non-tumor control cells (Table 6). In non-tumor cells (Table 7), the photosensitizing effects were low for complexes (**I-e**), (**I-g**), and (**I-k**) (PI values ranging from 1.2 to 2.4, median = 1.8-fold, average = 1.6-fold) yet quite significant for complexes (**I-h**) and (**I-j**) (PI values ranging from 6.0 to 26.2, median = 9-fold, mean = 12.5-fold). In all cancer cells, photosensitizing effects were markedly increased with PI values ranging from 3.7 to 17.2, median = 6.2-fold, mean = 8.4-fold for complex (**I-e**), PI values ranging from 4.6 to 16.9, median = 6.0-fold, mean = 7.5-fold for complex (**I-g**), PI values ranging from 12 to 24.8, median = 19.3-fold, mean = 19.0-fold for complex (**I-h**), PI values ranging from 18.9 to 49, median = 24.9-fold, mean = 28.0-fold for complex (**I-j**), and PI values ranging from 7.8 to 19.3, median = 12.2-fold, mean = 12.9-fold for complex (**I-k**) (Table 7). In conclusion, complex (**I-g**) and complex (**I-k**) showed the most selective effects under irradiated conditions, while complex (**I-g**) showed the highest selectivity under both dark and irradiated conditions and complex (**I-k**) showed little toxicity against tumor cells under dark conditions (Table 6 and Figure 3).

Table 6 : Cytotoxicity ($GI_{50}$ values in μM) and selectivity index (SI) of the series of biotin-vectorized compounds (I).

| | | **I-a** | | **I-b** | | **I-c** | | **I-d** | | **I-e** | | **I-g** | | **I-h** | | **I-i** | | **I-j** | | **I-k** | | **I-l** | |
|---|---|---|---|---|---|---|---|---|---|---|---|---|---|---|---|---|---|---|---|---|---|---|---|
| | | Dark | Blue | Dark | Blue | Dark | Blue | Dark | Blue | Dark | Blue | Dark | Blue | Dark | Blue | Dark | Blue | Dark | Blue | Dark | Blue | Dark | Blue |
| Non Tumoral Cells | NIH-3T3 | >100 | 32.6 | >100 | >100 | >100 | >100 | >100 | >100 | > 250 | 135 | 110 | 61.7 | 101 | 10.4 | >100 | >100 | 207 | 7.9 | 75.8 | 31.1 | 66.6 | 55.2 |
| | MC3T3 | | | | | | | | | 56.8 | 41.1 | 55.2 | 31.4 | 42.2 | 5.0 | | | 27.7 | 4.6 | 25.8 | 19.2 | | |
| Bladder Cancer | **T24** | **11.5** | **1.4** | **13.1** | **8.3** | 26.9 | **24.5** | **26.3** | **26.9** | **96.4** | **5.6** | **18.8** | 1.1 | **13.9** | **0.58** | **50.2** | **35.4** | **14.7** | **0.71** | **18.7** | **0.97** | **13.9** | **8.4** |
| | *SI (vs NIH-3T3)* | *>8.7* | *23.3* | *>7.3* | *>12* | *>3.7* | *>4.1* | *>3.8* | *>3.7* | *>2.6* | *24.1* | *5.9* | *56.1* | *7.3* | *17.9* | *>2.0* | *>2.8* | *14.1* | *11.1* | *4.1* | *32.1* | *4.8* | *6.6* |
| | *SI (vs MC3T3)* | | | | | | | | | *0.6* | *7.3* | *3.0* | *28.5* | *3.0* | *8.6* | | | *1.9* | *6.5* | *1.4* | *19.8* | | |
| Bladder Cancer | **Scaber** | | | | | | | | | **>100** | **14.7** | **14.1** | **2.5** | **24.8** | **1.0** | | | **58.8** | **1.2** | **25.9** | **1.8** | | |
| | *SI (vs NIH-3T3)* | | | | | | | | | >2.5 | 8.2 | 7.8 | 24.7 | 4.1 | *10.4* | | | 3.5 | 6.6 | 2.9 | 17.3 | | |
| | *SI (vs MC3T3)* | | | | | | | | | <1 | 2.8 | 3.9 | 12.6 | 1.7 | 5.0 | | | <1 | 3.8 | 1.0 | 10.7 | | |
| Bladder Cancer | **MGHU3** | | | | | | | | | **>100** | **17.8** | **25.4** | **4.3** | **26.7** | **1.6** | | | **37.8** | **2.0** | **46.1** | **2.9** | | |
| | *SI (vs NIH-3T3)* | | | | | | | | | >2.5 | 7.6 | 4.3 | 14.3 | 3.8 | 6.5 | | | 5.5 | 4.0 | 1.6 | 10.7 | | |
| | *SI (vs MC3T3)* | | | | | | | | | <1 | 2.3 | 2.2 | 7.3 | 1.6 | 3.1 | | | <1 | 2.3 | 0.6 | 6.6 | | |
| Lung Cancer | **A549** | | | | | | | | | **107** | **21.6** | **9.1** | **1.5** | **13.1** | **0.60** | | | **15.4** | **0.62** | **12.2** | **1.2** | | |
| | *SI (vs NIH-3T3)* | | | | | | | | | >2.3 | 6.3 | 12.1 | 41.1 | 7.7 | 17.3 | | | 13.4 | 12.7 | 6.2 | 25.9 | | |
| | *SI (vs MC3T3)* | | | | | | | | | 0.5 | 1.9 | 6.1 | 20.9 | 3.2 | 8.3 | | | 1.8 | 7.4 | 2.1 | 16.0 | | |

(continued)

| | | I-a | | I-b | | I-c | | I-d | | I-e | | I-g | | I-h | | I-i | | I-j | | I-k | | I-l | |
|---|---|---|---|---|---|---|---|---|---|---|---|---|---|---|---|---|---|---|---|---|---|---|---|
| | | Dark | Blue | Dark | Blue | Dark | Blue | Dark | Blue | Dark | Blue | Dark | Blue | Dark | Blue | Dark | Blue | Dark | Blue | Dark | Blue | Dark | Blue |
| Breast Cancer | **MCF7** | | | | | | | | | **145** | **12.2** | **13.8** | **2.2** | **11.9** | **0.99** | | | **29.9** | **1.2** | **14.9** | **1.5** | | |
| | *SI (vs NIH-3T3)* | | | | | | | | | *>1.7* | *11.1* | *8.0* | *28.0* | *8.5* | *10.5* | | | *6.9* | *6.6* | *5.1* | *20.7* | | |
| | *SI (vs MC3T3)* | | | | | | | | | *0.4* | *3.4* | *4.0* | *14.3* | *3.5* | *5.1* | | | *<1* | *3.8* | *1.7* | *12.8* | | |
| Cervix Cancer | **HeLa** | | | | | | | | | **221** | **59.0** | **44.3** | **10.8** | **41.8** | **2.86** | | | **77.7** | **2.63** | **50.6** | **6.50** | | |
| | *SI (vs NIH-3T3)* | | | | | | | | | *>1.1* | *2.3* | *2.5* | *5.7* | *2.4* | *3.6* | | | *2.7* | *3.0* | *1.5* | *4.8* | | |
| | *SI (vs MC3T3)* | | | | | | | | | *0.3* | *0.7* | *1.2* | *2.9* | *1.0* | *1.7* | | | *0.4* | *1.7* | *0.5* | *3.0* | | |

Table 7 : Photosensitizing index (PI) of the series of biotin-vectorized complex.

| | Non Tumoral Cells | | Tumoral Cells | | | | | |
|---|---|---|---|---|---|---|---|---|
| | **NIH-3T3** | **MC3T3** | **T24** | **Scaber** | **MGHU3** | **A549** | **MCF7** | **HeLa** |
| **I-a** | | | | | | | | |
| Dark | >100 | | 11.5 | | | | | |
| Blue | 32.6 | | 1.4 | | | | | |
| *PI* | *>3.1* | | *8.2* | | | | | |
| **I-b** | | | | | | | | |
| Dark | >100 | | 13.7 | | | | | |
| Blue | >100 | | 8.3 | | | | | |
| *PI* | *1.0* | | *1.7* | | | | | |
| **I-c** | | | | | | | | |
| Dark | >100 | | 26.9 | | | | | |
| Blue | >100 | | 24.5 | | | | | |
| *PI* | *1.0* | | *1.1* | | | | | |
| **I-d** | | | | | | | | |
| Dark | >100 | | 26.3 | | | | | |
| Blue | >100 | | 26.9 | | | | | |
| *PI* | *1.0* | | *1.0* | | | | | |
| **I-e** | | | | | | | | |
| Dark | >250 | 56.8 | 96 | >100 | >100 | 107 | 145 | 221 |
| Blue | 135 | 41.1 | 5.6 | 14.7 | 17.8 | 21.6 | 12.2 | 59.0 |
| *PI* | *>1.9* | *1.4* | *17.2* | *>6.8* | *>5.6* | *5.0* | *11.9* | *3.7* |
| **I-g** | | | | | | | | |
| Dark | 110 | 55.2 | 18.6 | 14.1 | 25.4 | 9.1 | 13.8 | 44.3 |
| Blue | 61.7 | 31.4 | 1.1 | 2.5 | 4.3 | 1.5 | 2.2 | 10.8 |
| *PI* | *1.8* | *1.8* | *16.9* | *5.6* | *5.9* | *6.1* | *6.3* | *4.1* |
| **I-h** | | | | | | | | |
| Dark | 101 | 42.2 | 13.9 | 24.8 | 26.7 | 13.1 | 11.9 | 41.8 |
| Blue | 10.4 | 5.0 | 0.58 | 1.0 | 1.6 | 0.60 | 0.99 | 2.86 |
| *PI* | *9.7* | *8.4* | *24.0* | *24.8* | *16.7* | *21.8* | *12.0* | *14.6* |
| **I-i** | | | | | | | | |
| Dark | >100 | | 50.2 | | | | | |
| Blue | >100 | | 35.4 | | | | | |
| *PI* | *>1* | | *1.4* | | | | | |
| **I-j** | | | | | | | | |
| Dark | | | | | | | | |
| Blue | 207 | 27.7 | 14.7 | 58.8 | 37.8 | 15.4 | 29.9 | 77.7 |
| *PI* | 7.9 | 4.6 | 0.71 | 1.2 | 2.0 | 0.62 | 1.2 | 2.6 |
| | *26.2* | *6.0* | *20.7* | *49.0* | *18.9* | *24.8* | *24.9* | *29.9* |
| **I-k** | | | | | | | | |
| Dark | 75.8 | 25.8 | 18.7 | 25.9 | 46.1 | 12.2 | 14.9 | 50.6 |
| Blue | 31.1 | 19.2 | 0.97 | 1.80 | 2.9 | 1.2 | 1.5 | 6.5 |
| *PI* | *2.4* | *1.3* | *19.3* | *14.4* | *15.9* | *10.2* | *9.9* | *7.8* |

(continued)

| I-I | | |
|---|---|---|
| Dark | 66.6 | 13.9 |
| Blue | 55.2 | 8.4 |
| *PI* | *1.2* | *1.7* |

III.3. Cytotoxicity and photodynamic of the series of salarisib-vectorized complexes.

RAS proteins function as binary molecular switches that control intracellular signaling pathways involved in cellular processes such as proliferation, differentiation, or survival, in response to extracellular signals activating receptor tyrosine kinases. RAS proteins cycle between an inactive GDP-bound conformation and an active GTP-bound conformation. Mutations of *RAS* family genes (*K-RAS, N-RAS* and *H-RAS*) render the RAS protein constitutively active. Activating point mutations of *RAS* are not uncommon and comprise up to nearly one-third of all human cancers, including 90% of pancreatic ductal adenocarcinomas. The association of RAS proteins with membranes is absolutely required for their function. The RAS antagonist salirasib, also known as farnesylthiosalicylate (FTS), was designed to competitively inhibit the binding of GTP-bound RAS to its plasma membrane-anchorage domain (galectin), thereby inactivating RAS signaling by facilitating its degradation. Salirasib inhibits all isoforms of RAS and inhibits RAS-induced cell growth in several cell and animal models. Although salirasib did not show sufficient efficacy in Phase I and II clinical trials in humans, recent work has shown that it can be conjugated to improve its bioavailability. Thus, conjugated with heptamethine carbocyanine dyes, Salirasib has shown its interest as a tumor-targeting delivery system usable for tumor imaging in pancreatic animal models [19].

The cytotoxicity and photodynamic activities of the five salarisib-vectorized compounds were examined in two non-tumor cell models (NIH-3T3 and MC3T3) and four cancer cell lines carrying various Ras mutations (H-Ras-mutated T24 bladder, K-Ras-mutated MiaPaCa2 pancreas, K-Ras-mutated ASPC-1 pancreas, and K-Ras-mutated A549 lung). $GI_{50}$ values under dark and light irradiation conditions are summarized in Table 8. In the dark, complexes **(II-e)** and **(II-j)** showed moderate toxicity toward NIH-3T3 and MC3T3 cells with $GI_{50}$ values ranging from 7 to 12 $\mu$M, whereas the remaining compounds showed higher cytotoxicity ($GI_{50}$ values ranging from 4 to 5.8 $\mu$M for complexes **(II-g)** and **(II-k)** and $GI_{50}$ values ranging from 2 to 3.6 $\mu$M for complex **(II-k).** In cancer cells, specificity was moderate for complex **(II-e)** (SI values ranging from 2.2 to 6.1, median = 4.1-fold, mean = 4.1-fold), complex **(II-h)** (SI values ranging from 1.6 to 4.1, median = 2.7-fold, mean = 2.7-fold), complex **(II-j)** (SI values ranging from 0.6 to 3.6, median = 2.0-fold, mean = 2.0-fold), and complex **(II-k)** (SI values ranging from 2.0 to 4.7, median = 3.3-fold, mean = 3.1-fold) regardless of the cell type (Table 8). Compound complex **(II-g)** showed the highest selectivity toward cancer cells compared to non-tumor cells with SI values ranging from 5.8 to 14.4 (median = 10.3-fold, mean = 10.2-fold).

Under blue light, complex **(II-g)** was the only one to display zero photosensitizing effect on non-tumor as well as on tumor cells, as shown in Table 9 (PI values ranging from 0.8 to 1 and from to 0.7 to 1.6, respectively). For all other compounds, the photosensitizing effect was significantly higher on cancer cells than on non-tumor models (PI values ranging from 1.1 to 5.0 and from 4 to 43.6, respectively). Complexes **(II-h)** and **(II-j)** showed the highest photosensitizing effect on cancer cells (PI values ranging from 18.1 to 37.8 and from 22.3 to 43.6, respectively) with minor to moderate effect on non-tumor models (PI values ranging from 2.6 to 2.9 and from 4.8 to 5.0, respectively).

Collectively, complex **(II-h)** showed the most selective effects under irradiated conditions with a 25-fold increase in cell toxicity in cancer cells ($GI_{50}$ values ranging from 37 to 103 nM, mean = 66 nM) compared to non-tumor models ($GI_{50}$ values ranging from 1.37 to 1.85 $\mu$M, mean = 1.61 $\mu$M) as shown in Table 8 and Figure 4.

Table 8 : Cytotoxicity ($GI_{50}$ values in $\mu$M) and selectivity index (SI) of the salarisib-vectorized complexes.

| | | II-e | | II-g | | II-h | | II-j | | II-k | |
|---|---|---|---|---|---|---|---|---|---|---|---|
| | | Dark | Blue | Dark | Blue | Dark | Blue | Dark | Blue | Dark | Blue |
| Non Tumoral Cells | NIH-3T3 | 12 | 5.2 | 4.3 | 5.1 | 4.1 | 1.4 | 12 | 2.5 | 3.6 | 1.7 |
| | MC3T3 | 11.0 | 10.1 | 5.8 | 5.9 | 4.9 | 1.9 | 7.0 | 1.4 | 2.0 | 1.7 |
| Bladder Cancer | **T24** | **2.2** | **0.26** | **0.47** | **0.64** | **1.2** | **0.064** | **3.7** | **0.17** | **0.77** | **0.10** |
| | *SI (vs NIH-3T3)* | *5.5* | *20.0* | *9.1* | *8.0* | *3.4* | *21.9* | *3.2* | *15.1* | *4.7* | *16.3* |
| | *SI (vs MC3T3)* | *5.0* | *38.8* | *12.3* | *9.2* | *4.1* | *29.7* | *1.9* | *8.4* | *2.6* | *16.3* |
| Pancreatic Cancer | **MiaPaCa2** | **2.0** | **0.18** | **0.40** | **0.25** | **1.4** | **0.037** | **3.3** | **0.095** | **0.80** | **0.054** |

(continued)

| | | II-e | | II-g | | II-h | | II-j | | II-k | |
|---|---|---|---|---|---|---|---|---|---|---|---|
| | | Dark | Blue | Dark | Blue | Dark | Blue | Dark | Blue | Dark | Blue |
| | *SI (vs NIH-3T3)* | *6.1* | *29.7* | *10.7* | *20.7* | *3.0* | *37.8* | *3.6* | *26.3* | *4.5* | *31.5* |
| | *SI (vs MC3T3)* | *5.6* | *57.7* | *14.4* | *24.0* | *3.6* | *51.4* | *2.1* | *14.7* | *2.5* | *31.5* |
| Pancreatic Cancer | ASPC-1 | **5.1** | **1.3** | **0.74** | **0.47** | **3.0** | **0.10** | **10.9** | **0.25** | **1.00** | **0.15** |
| | *SI (vs NIH-3T3)* | *2.3* | *4.1* | *5.8* | *10.9* | *1.4* | *13.6* | *1.1* | *10.0* | *3.6* | *11.7* |
| | *SI (vs MC3T3)* | *2.2* | *7.9* | *7.8* | *12.6* | *1.6* | *18.4* | *0.6* | *5.6* | *2.0* | *11.7* |
| Lung Cancer | **A549** | **3.80** | **0.69** | **0.45** | **0.39** | **2.1** | **0.061** | **5.1** | **0.23** | **0.88** | **0.11** |
| | *SI (vs NIH-3T3)* | *3.2* | *7.5* | *9.6* | *13.2* | *1.9* | *23.0* | *2.3* | *11.0* | *4.1* | *15.7* |
| | *SI (vs MC3T3)* | *2.9* | *14.6* | *12.9* | *15.2* | *2.3* | *31.1* | *1.4* | *6.1* | *2.3* | *15.7* |

Table 9 : Photosensitizing index (PI) of the salarisib-vectorized complexes.

| | Non Tumoral Cells | | Tumoral Cells | | | |
|---|---|---|---|---|---|---|
| **II-e** | **NIH-3T3** | **MC3T3** | **T24** | **MiaPaCa2** | **ASPC-1** | **A549** |
| Dark | 12.0 | 11.0 | 2.2 | 2.0 | 5.1 | 3.8 |
| Blue | 5.2 | 10.1 | 0.26 | 0.18 | 1.3 | 0.69 |
| *PI* | *2.3* | *1.1* | *8.6* | *11.2* | *4.0* | *5.5* |
| **II-g** | | | | | | |
| Dark | 4.3 | 5.8 | 0.47 | 0.40 | 0.74 | 0.45 |
| Blue | 5.1 | 5.9 | 0.65 | 0.25 | 0.47 | 0.39 |
| *PI* | *0.8* | *1.0* | *0.7* | *1.6* | *1.6* | *1.2* |
| **II-h** | | | | | | |
| Dark | 4.1 | 4.9 | 1.2 | 1.40 | 3.00 | 2.10 |
| Blue | 1.4 | 1.9 | 0.064 | 0.037 | 0.10 | 0.061 |
| *PI* | *2.9* | *2.6* | *18.1* | *37.8* | *29.1* | *34.4* |
| **II-j** | | | | | | |
| Dark | 11.9 | 7.0 | 3.7 | 3.3 | 10.90 | 5.1 |
| Blue | 2.5 | 1.4 | 0.17 | 0.095 | 0.25 | 0.23 |
| *PI* | *4.8* | *5.0* | *22.3* | *35.1* | *43.6* | *22.3* |
| **II-k** | | | | | | |
| Dark | 3.6 | 2.0 | 0.77 | 0.80 | 1.00 | 0.88 |
| Blue | 1.7 | 1.7 | 0.11 | 0.054 | 0.15 | 0.11 |
| *PI* | *2.1* | *1.2* | *7.2* | *14.7* | *6.9* | *8.2* |

III.4. Cytotoxicity and photodynamic of the series of PSMA-vectorized complexes.

In what follows, the expression "PSMA-vectorized complexes" is to be understood "PSMA inhibitor-vectorized complexes" or "Lys-urea-Glu-vectorized complexes".

Prostate-specific membrane antigen (PSMA) is a transmembrane protein, which upon binding of a ligand is internalized into the cell. PSMA is mainly expressed in prostate tissue but can be found in lacrimal and salivary glands, kidneys, liver, spleen and small intestine. Its expression can also be detected in the neovasculature of some solid tumors including glioblastoma, thyroid cancer, gastric, breast, renal and colorectal cancers. Yet, its expression is dramatically increase (100- to 1,000-fold) on the cell membrane of nearly all prostatic cancer cells compared with normal non-target expression (on normal prostate epithelial cells, in the small intestine, renal tubular cells and the salivary glands), and increases in higher grades, metastatic, and castration-resistant cancers, potentially enabling PSMA to be used for

diagnosis and therapy for prostate cancer.

Several antibodies or small molecules targeting PSMA with very high affinity and specificity have been developed (i) for imaging, especially for biological recurrence or detection of oligometastases (e.g., PSMA small-molecule inhibitor HBED-CC, a class of urea-based PSMA inhibitors, labelled with $^{68}$Gallium for positron emission tomography (FDA Approved), or (ii) for the potential treatment of metastatic castration-resistant prostate cancer (e.g., ongoing phase III trial with $^{177}$Lutetium radiolabeled antibody HuJ591 or phase I with an anti-PSMA small-molecule drug conjugate with tubulysin B).

The cytotoxicity and photodynamic activities of the ten PSMA-vectorized complexes were examined in the non-tumor cell model (NIH-3T3) and two prostate cancer cell models : PC-3 (that does not express PSMA) and C4-2b expressing PSMA. $GI_{50}$ values under dark and light irradiation conditions are summarized in Table 10. In the dark, all compounds showed no toxicity toward NIH-3T3 cells with $GI_{50}$ values above 100 μM, and moderate to no toxicity in PC-3 ($GI_{50}$ values ranging from 9.6 to >100 μM) and C4-2b ($GI_{50}$ values ranging from 4.0 to >100 μM) cells. Compounds **(III-h), (III-I), (III-j),** and **(III-k)** showed the highest selectivity index in both PC-3 (SI values ranging from >1.7 to >10.4, median = 3.2-fold, mean = 4.6-fold) and C4-2b cells (SI values ranging from >6.7 to 25, median = 13.9-fold, mean = 14.9-fold), whereas compounds **(III-a), (III-b), (III-d), (III-e), (III-**g), and **(III-l)** no selectivity against PSMA-negative PC-3 cells and little selectivity (SI values ranging from 1 to 4.7, median = 2.2-fold, mean = 2.6-fold) against PSMA-positive C4-2b cells.

Complexes **(III-a), (III-b), (III-d),** and **(III-l)** did not show any photosensitizing effect on non-tumoral NIH-3T3 and on PC-3 and C4-2b tumor cells as shown in Table 11. Complexes **(III-e)** and **(III-g)** showed photosensitizing effect on non-tumoral NIH-3T3 and a small photosensitizing effect against PC-3 (PI values from >2.1 to >7.0) and C4-2b (PI values from >1.2 to >3.8) tumor cells in contrast to complexes **(III-h), (III-I), (III-j),** and **(III-k)** (PI values ranging from 4.6 to 31.2 in PC-3 cells and from 5.5 to 21.7 in C4-2b). Therefore, only complexes **(III-h), (III-I), (III-j),** and **(III-k)** were evaluated in an additional non-tumor cell line (osteoblastic MC3T3) and two additional prostate cancer cell lines (the PSMA-negative DU145 and the PSMA-positive 22Rv1).

Similar to NIH-3T3, complexes **(III-h), (III-I), (III-j),** and **(III-k)** showed low to no toxicity toward MC3T3 cells ($GI_{50}$ values ranging from 52.4 to > 250 μM). As shown in Table 10, SI values were higher in PSMA-negative cells (PC-3 and DU145) than in PSMA-positive cells (C4-2b and 22Rv1) when compared to both NIH-3T3 and MC3T3 cells in the dark with SI values ranging from >0.5 to 10.4-fold in PSMA-negative cells (median = 2.4-fold, mean = 4.1-fold) versus SI values ranging from 8 to >25.2-fold in PSMA-positive cells (median = 15-fold, mean = 16.8-fold). As shown in Table 11, under blue light, the photosensitizing effect on non-tumoral cells was moderate with PI values ranging from 1.8 to > 20-fold (median = 5.8-fold, mean = 6.9-fold), but much higher in cancer cells regardless of their PSMA status with PI values ranging from 4.6 to 146.4-fold (median = 20.1-fold, mean = 24.2-fold).

Overall, complexes **(III-h),** and **(III-k)** showed the most selective effects under irradiated conditions with a >70-fold increase in cell toxicity in PSMA-positive cancer cells ($GI_{50}$ values ranging from 140 to 410 nM, mean = 310 nM), and >14-fold increase in cell toxicity in PSMA-neagtive cancer cells ($GI_{50}$ values ranging from 0.679 to 2.2 μM, mean = 1.6 μM) compared to non-tumor models ($GI_{50}$ values ranging from 8.3 to 33.8 μM, mean = 22.8 μM) as shown in Table 10 and Figure 5.

Table 10 : Cytotoxicity ($GI_{50}$ values in $\mu M$) and selectivity index (SI) of the PSMA-vectorized complexes.

| | | **III-a** | | **III-b** | | **III-c** | | **III-d** | | **III-e** \| | | **III-g** | | **III-h** \| | | **III-i** | | **III-j** | | **III-k** | | **III-l** | |
|---|---|---|---|---|---|---|---|---|---|---|---|---|---|---|---|---|---|---|---|---|---|---|---|
| | | Dark | Blue | Dark | Blue | Dark | Blue | Dark | Blue | Dark | Blue | Dark | Blue | Dark | Blue | Dark | Blue | Dark | Blue | Dark | Blue | Dark | Blue |
| Non Tumoral Cell: | NIH-3T3 | >100 | >100 | >100 | >100 | | | >100 | >100 | >100 | >100 | >100 | >100 | >100 | 19.0 | >100 | 55.8 | >100 | 10.8 | >100 | 30.0 | >100 | >100 |
| | MC3T3 | | | | | | | | | | | | | 52.4 | 8.3 | 250 | 40.6 | >250 | 12.5 | 95.1 | 33.8 | | |
| Prostate Cancer | **PC-3** | **>100** | **>100** | **>100** | **>100** | | | **>100** | **>100** | **>100** | **14.3** | **100** | **48.7** | **21.2** | **0.68** | **57.2** | **8.7** | **58.2** | **1.9** | **9.6** | **2.1** | **>100** | **>100** |
| | *SI (vs NIH-3T3)* | *1.0* | *1.0* | *1.0* | *1.0* | | | *1.0* | *1.0* | *1.0* | *>7.0* | *>1.0* | *>2.1* | *>4.7* | *28.0* | *>1.7* | *6.4* | *>1.7* | *5.7* | *>10.4* | *14.3* | *1.0* | *1.0* |
| | *SI (vs MC3T3)* | | | | | | | | | | | | | *2.5* | *12.2* | *4.4* | *4.7* | *>4.3* | *6.6* | *9.9* | *16.1* | | |
| Prostate Cancer | **DU145** | | | | | | | | | | | | | **26.1** | **1.41** | **45.3** | **8.2** | **205** | **1.4** | **16.3** | **2.2** | | |
| | *SI (vs NIH-3T3)* | | | | | | | | | | | | | *>3.8* | *13.5* | *>2.2* | *6.8* | *>0.5* | *8.0* | *>6.1* | *13.8* | | |
| | *SI (vs MC3T3)* | | | | | | | | | | | | | *2.0* | *5.9* | *5.5* | *5.0* | *>1.2* | *9.3* | *5.8* | *15.5* | | |
| Prostate Cancer | **C4-2b** | | | | | | | | | **>100** | **26.4** | **21.3** | **17.6** | **4.0** | **0.31** | **14.8** | **2.7** | **11.3** | **0.52** | **5.3** | **0.41** | **44.7** | **>50** |
| | *SI (vs NIH-3T3)* | | | | | | | | | *1.0* | *>3.8* | *>4.7* | *>5.7* | *>25* | *61.9* | *>6.7* | *20.7* | *>8.8* | *20.7* | *>18.9* | *73.9* | *>2.2* | *>2.0* |
| | *SI (vs MC3T3)* | | | | | | | | | | | | | *13.0* | *27.0* | *16.9* | *15.1* | *>22.1* | *24.0* | *17.9* | *83.3* | | |
| Prostate Cancer | **22Rv1** | | | | | | | | | | | | | **4.1** | **0.14** | **12.5** | **2.0** | **9.9** | **0.28** | **5.1** | **0.39** | | |
| | *SI (vs NIH-3T3)* | | | | | | | | | | | | | *>25* | *131.9* | *>8* | *28.5* | *>10.1* | *38.0* | *>19.6* | *77.3* | | |
| | *SI (vs MC3T3)* | | | | | | | | | | | | | *12.9* | *57.6* | *20.0* | *20.7* | *>25.2* | *44.0* | *18.8* | *87.1* | | |

Table 11 : Photosensitizing index (PI) of the PSMA-vectorized complexes.

| | Non Tumoral Cells | | Tumoral Cells \| | | | |
|---|---|---|---|---|---|---|
| | **NIH-3T3** | **MC3T3** | **PC3** | **DU145** | **C4-2b** | **22Rv1** |
| **III-a** | | | | | | |
| Dark | >100 | | >100 | | | |
| Blue | >100 | | >100 | | | |
| *PI* | *1.0* | | *1.0* | | | |
| **III-b** | | | | | | |
| Dark | >100 | | >100 | | | |
| Blue | >100 | | >100 | | | |
| *PI* | *1.0* | | *1.0* | | | |
| **III-c** | | | | | | |
| Dark | | | | | | |
| Blue | | | | | | |
| *PI* | | | | | | |
| **III-d** | | | | | | |
| Dark | >100 | | >100 | | | |
| Blue | >100 | | >100 | | | |
| PI | *1.0* | | *1.0* | | | |
| **III-e** | | | | | | |
| Dark | >100 | | >100 | | >100 | |
| Blue | >100 | | 14.3 | | 26.4 | |
| *PI* | *1.0* | | *>7.0* | | *>3.8* | |
| **III-g** | | | | | | |
| Dark | >100 | | >100 | | 21.3 | |
| Blue | >100 | | 48.7 | | 17.6 | |
| *PI* | *1.0* | | *>2.1* | | *1.2* | |
| **III-h** | | | | | | |
| Dark | >100 | 52.4 | 21.2 | 26.1 | 4.0 | 4.1 |
| Blue | 19.0 | 8.3 | 0.68 | 1.41 | 0.31 | 0.14 |
| *PI* | *>5.3* | *6.3* | *31.2* | *18.5* | *12.9* | *29.3* |
| **III-i** | | | | | | |
| Dark | >100 | 250 | 57.2 | 45.3 | 14.8 | 12.5 |
| Blue | 55.8 | 40.6 | 8.72 | 8.16 | 2.69 | 1.96 |
| *PI* | *>1.8* | *6.2* | *6.6* | *5.6* | *5.5* | *6.4* |
| **III-j** | | | | | | |
| Dark | >100 | >250 | 58.2 | 205.0 | 11.3 | 9.9 |
| Blue | 10.8 | 12.5 | 1.94 | 1.35 | 0.52 | 0.28 |
| *PI* | *>9.3* | *>20* | *30.0* | *151.9* | *21.7* | *35.4* |
| **III-k** | | | | | | |
| Dark | >100 | 95.1 | 9.6 | 16.30 | 5.3 | 5.1 |
| Blue | 30.0 | 33.8 | 2.10 | 2.18 | 0.41 | 0.39 |
| *PI* | *>3.3* | *2.8* | *4.6* | *7.5* | *12.9* | *13.1* |
| **III-l** | | | | | | |
| Dark | >100 | | >100 | | 44.7 | |

(continued)

| | | | | | | |
|---|---|---|---|---|---|---|
| Blue | 30.0 | 33.8 | 2.10 | 2.18 | 0.41 | 0.39 |
| Blue | >100 | | >100 | | >50 | |
| *PI* | *1.0* | | *1.0* | | *<1* | |

III.5. Cytotoxicity and photodynamic of the series of ferrocene-vectorized complexes.

Ferrocene is the prototype of metallocenes with (i) low toxicity, (ii) excellent stability, and (iii) remarkable platform capabilities for the development of new organometallic compounds to treat a variety of diseases including cancer. The ability of ferrocene to induce ROS production via *in situ* oxidation (Fe(II)-Fe(III)) is the most important feature of ferrocene in its anticancer effects. Ferrocene has been shown to improve the profile of several anti-cancer agents including tamoxifen, nilutamide or taxanes.

The cytotoxicity and photodynamic activities of the twelve ferrocene-vectorized complexes were first examined in two cell models (non-tumor fibroblastic NIH-3T3 and PC3 protate cancer). $GI_{50}$ values obtained under dark and light irradiation conditions are summarized in Table 12. Under dark conditions, the complexes showed a minimal toxicity toward NIH-3T3 cells with $GI_{50}$ values ranging from 11.8 to 62.8 μM (mean = 26.8 μM, median = 25.8 μM), and significant to moderate toxicity ($GI_{50}$ values ranging from 850 nM to 21.6 μM, mean = 6.1 μM, median = 3.9 μM) against PC3 cells. Under irradiation conditions, complexes **(IV-h), (IV-i), (IV-j),** and **(IV-k)** showed the highest specificity towards PC3 cells (SI values ranging from 65.7 to 233.3, median = 80-fold, mean = 114.7-fold) as shown in Table 12. Complex **(IV-b)** showed a good specificity (SI value = 33.8) while the other compounds showed a lower specificity (SI values ranging from 2.8 to 13.7, median = 7.8-fold, average = 8.6-fold) as shown in Table 12. Based on these results, compounds **(IV-h), (IV-i), (IV-j),** and **(IV-k)** were further evaluated in an additional non-tumor cell line (osteoblastic MC3T3) and four additional cancer cell models (T24 bladder, A549 lung, MCF7 breast and MiaPaCa2 pancreatic). As for NIH3T3, the compounds showed limited toxicity in the dark against the non-tumor MC3T3 cell model with $GI_{50}$ values ranging from 10.0 to 17.1 μM (mean = 13.8 μM), and significant to moderate toxicity ($GI_{50}$ values ranging from 890 nM to 7.3 μM, mean = 3.8 μM) against the five tumor cell models. The photosensitizing effects of complexes **(IV-h), (IV-i), (IV-j),** and **(IV-k)** were low and similar in NIH-3T3 and MC3T3 non-tumoral cells with a mean PI value of 2.8-fold (Table 13). In tumor cells, complexes **(IV-h)** and **(IV-j)** showed high photosensitizing effect with PI values ranging from 13.0 to 24.6 (mean = 20.1-fold) and from 15.7 to 121.7 (mean = 39.8-fold), respectively. In contrast, complexes **(IV-i)** and **(IV-k)** showed very low photosensitizing effect in tumor cells (mean PI values of 1.6-fold and 1.8-fold, respectively).

Collectively, complexes **(IV-h)** and **(IV-j)** showed the most selective effects under irradiated conditions with 29-fold and 39-fold increases in cell toxicity in cancer cells ($GI_{50}$ values ranging from 58 to 277 nM, mean = 155 nM for complex **(IV-h);** and from 60 to 421 nM, mean = 228 nM for complex **(IV-j)),** respectively compared to non-tumor models ($GI_{50}$ values ranging from 4.44 to 4.46 μM, mean = 4.45 μM and from 5.1 to 11.6 μM, mean = 8.4 μM, respectively) as shown in Figure 6.

Table 12 : Cytotoxicity (GI50 values in μM) and selectivity index (SI) of the ferrocene-vectorized complexes.

| | | **IV-a** | | **IV-b** | | **IV-c** | | **IV-d** | | **IV-e** | | **IV-g** | | **IV-h** | | **IV-i** | | **IV-j** | | **IV-k** | | **IV-l** | | **IV-m** | |
|---|---|---|---|---|---|---|---|---|---|---|---|---|---|---|---|---|---|---|---|---|---|---|---|---|---|
| | | Dark | Blue | Dark | Blue | Dark | Blue | Dark | Blue | Dark | Blue | Dark | Blue | Dark | Blue | Dark | Blue | Dark | Blue | Dark | Blue | Dark | Blue | Dark | Blue |
| Non Tumoral Cell: | NIH-3T3 | 28.2 | 6.7 | 13.9 | 13.2 | 32.3 | 9.3 | 28.9 | 15.3 | 32.3 | 4.2 | 11.8 | 3.6 | 13.3 | 4.4 | 41.3 | 17.8 | 62.8 | 14.0 | 18.7 | 11.6 | 66.6 | 55.2 | 66.6 | 55.2 |
| | MC3T3 | | | | | | | | | | | | | 117.1 | 4.5 | 10.0 | 9.6 | 16.4 | 3.9 | 10.2 | 5.1 | | | | |
| Prostate Cancer | **PC3** | **2.9** | **2.4** | **0.85** | **0.39** | **14.6** | **0.87** | **21.6** | **1.3** | **9.9** | **0.54** | **1.5** | **0.26** | **1.3** | **0.058** | **5.9** | **0.27** | **7.3** | **0.060** | **1.1** | **0.14** | **13.9** | **8.4** | **13.9** | **8.4** |
| | *SI (vs NIH-3T3)* | *9.9* | *2.8* | *16.4* | *33.8* | *2.2* | *10.7* | *1.3* | *12.0* | *3.3* | *7.8* | *7.9* | *13.7* | *10.2* | *75.9* | *7.0* | *65.7* | *8.6* | *233.3* | *17.3* | *84.1* | *4.8* | *6.6* | *4.8* | *6.6* |
| | *SI (vs MC3T3)* | | | | | | | | | | | | | *13.2* | *77.6* | *1.7* | *35.4* | *2.2* | *65.0* | *9.4* | *37.0* | | | | |
| Bladder Cancer | **T24** | | | | | | | | | | | | | **1.3** | **0.10** | **1.8** | **0.35** | **2.8** | **0.13** | **0.89** | **0.19** | | | | |
| | *SI (vs NIH-3T3)* | | | | | | | | | | | | | *10.2* | *44.0* | *23.2* | *50.4* | *22.7* | *108.5* | *20.9* | *61.4* | | | | |
| | *SI (vs MC3T3)* | | | | | | | | | | | | | *13.2* | *45.0* | *5.6* | *27.2* | *5.9* | *30.2* | *11.4* | *27.0* | | | | |
| Lung Cancer | **A549** | | | | | | | | | | | | | **5.1** | **0.27** | **4.6** | **1.0** | **6.5** | **0.37** | **3.8** | **0.34** | | | | |
| | *SI (vs NIH-3T3)* | | | | | | | | | | | | | *2.6* | *16.3* | *9.0* | *17.8* | *9.7* | *37.8* | *4.9* | *34.2* | | | | |
| | *SI (vs MC3T3)* | | | | | | | | | | | | | *3.4* | *16.7* | *2.2* | *9.6* | *2.5* | *10.5* | *2.7* | *15.0* | | | | |
| Breast Cancer | **MCF7** | | | | | | | | | | | | | **6.0** | **0.28** | **5.3** | **0.62** | **6.6** | **0.42** | **5.8** | **0.62** | | | | |
| | *SI (vs NIH-3T3)* | | | | | | | | | | | | | *2.2* | *15.7* | *7.8* | *28.7* | *9.5* | *33.3* | *3.2* | *18.7* | | | | |
| | *SI (vs MC3T3)* | | | | | | | | | | | | | *2.9* | *16.1* | *1.9* | *15.5* | *2.5* | *9.3* | *1.8* | *8.2* | | | | |
| Pancreatic Cance | **MiapaCa 2** | | | | | | | | | | | | | **1.7** | **0.069** | **1.9** | **0.37** | **3.5** | **0.16** | **2.2** | **0.32** | | | | |
| | *SI (vs NIH-3T3)* | | | | | | | | | | | | | *7.8* | *63.8* | *21.7* | *48.1* | *17.9* | *87.5* | *8.5* | *36.3* | | | | |

(continued)

| | IV-a | | IV-b | | IV-c | | IV-d | | IV-e | | IV-g | | IV-h | | IV-i | | IV-j | | IV-k | | IV-l | | IV-m | |
|---|---|---|---|---|---|---|---|---|---|---|---|---|---|---|---|---|---|---|---|---|---|---|---|---|
| | Dark | Blue | Dark | Blue | Dark | Blue | Dark | Blue | Dark | Blue | Dark | Blue | Dark | Blue | Dark | Blue | Dark | Blue | Dark | Blue | Dark | Blue | Dark | Blue |
| *SI (vs MC3T3)* | | | | | | | | | | | | | *10.1* | *65.2* | *5.3* | *25.9* | *4.7* | *24.4* | *4.6* | *15.9* | | | | |

Table 13 : Photosensitizing index (PI) of the ferrocene-vectorized complexes.

| | Non Tumoral Cells | | Tumoral Cells | | | | |
|---|---|---|---|---|---|---|---|
| | **NIH-3T3** | **MC3T3** | **PC3** | **T24** | **A549** | **MCF7** | **MiaPaCa2** |
| **IV-a** | | | | | | | |
| Dark | 28.2 | | 2.9 | | | | |
| Blue | 6.7 | | 2.4 | | | | |
| *PI* | *4.2* | | *1.2* | | | | |
| **IV-b** | | | | | | | |
| Dark | 13.9 | | 0.85 | | | | |
| Blue | 13.2 | | 0.39 | | | | |
| *PI* | *1.1* | | 2.2 | | | | |
| **IV-c** | | | | | | | |
| Dark | 32.3 | | 14.6 | | | | |
| Blue | 9.3 | | 0.87 | | | | |
| *PI* | *3.5* | | *16.8* | | | | |
| **IV-d** | | | | | | | |
| Dark | 28.9 | | 21.6 | | | | |
| Blue | 15.3 | | 1.3 | | | | |
| *PI* | *1.9* | | *17.0* | | | | |
| **IV-e** | | | | | | | |
| Dark | 32.3 | | 9.9 | | | | |
| Blue | 4.2 | | 0.54 | | | | |
| *PI* | *7.7* | | *18.3* | | | | |
| **IV-g** | | | | | | | |
| Dark | 11.8 | | 1.5 | | | | |
| Blue | 3.6 | | 0.26 | | | | |
| *PI* | *3.3* | | *5.8* | | | | |
| **IV-h** | | | | | | | |
| Dark | 13.3 | 17.1 | 1.3 | 1.3 | 5.1 | 6.0 | 1.7 |
| Blue | 4.4 | 5 | 0.058 | 0.10 | 0.27 | 0.28 | 0.069 |
| *PI* | *3.0* | *3.8* | *22.4* | *13.0* | *18.9* | *21.4* | 24.6 |
| **IV-i** | | | | | | | |
| Dark | 41.3 | 10.0 | 5.9 | 1.8 | 4.6 | 5.3 | 1.9 |
| Blue | 17.8 | 10 | 0.27 | 0.35 | 1.00 | 0.62 | 0.37 |
| *PI* | *2.3* | *1.0* | 21.9 | 5.1 | 4.6 | 8.5 | 5.1 |
| **IV-j** | | | | | | | |
| Dark | 62.8 | 16.4 | 7.3 | 2.8 | 6.5 | 6.6 | 3.5 |
| Blue | 14.0 | 3.9 | 0.060 | 0.13 | 0.37 | 0.42 | 0.16 |
| *PI* | *4.5* | *4.2* | *121.7* | *21.5* | *17.6* | *15.7* | *21.9* |
| **IV-k** | | | | | | | |
| Dark | 18.7 | 11.6 | 1.1 | 0.89 | 3.8 | 5.8 | 2.2 |
| Blue | 11.6 | 5.1 | 0.14 | 0.19 | 0.34 | 0.62 | 0.32 |
| *PI* | 1.6 | 2.3 | 7.9 | *4.7* | *11.2* | *9.4* | *6.9* |

(continued)

| | | |
|---|---|---|
| **IV-l** | | |
| Dark | 23.4 | 1.6 |
| Blue | 4.3 | 0.5 |
| *PI* | *5.4* | 3.2 |
| **IV-m** | | |
| Dark | 15.1 | 4.9 |
| Blue | 14.3 | 4.5 |
| *PI* | *1.1* | 1.1 |

**References**

[1] Zhu et al, 2007, "Simple Copper Salt-Catalyzed N-Arylation of Nitrogen-Containing Heterocycles with Aryl and Heteroaryl Halides", J. Org. Chem., vol. 72, pages 8535-8538.

[2] Susumu et al, 2007, "Enhancing the Stability and Biological Functionalities of Quantum Dots via Compact Multifunctional Ligands", J. Am. Chem. Soc., vol. 129, pages 13987-13996.

[3] Porta et al, 2019, "Repositioning Salirasib as a New Antimalarial Agent", Med. Chem. Commun., vol. 10, pages 1599-1605.

[4] Maresca et al, 2009, "A Series of Halogenated Heterodimeric Inhibitors of Prostate Specific Membrane Antigen (PSMA) as Radiolabeled Probes for Targeting Prostate Cancer", J. Med. Chem., vol. 52, pages 347-357.

[5] Lindsay & Hauser, 1957, "Aminomethylation of Ferrocene to Form N,N-Dimethylaminomethylferrocene and Its Conversion to the Corresponding Alcohol and Aldehyde", C. R. J. Org. Chem., vol. 22, pages 355-358.

[6] Garces et al, 1988, "Synthesis, Structure, Electrochemistry, and Photophysics of Methyl-Substituted Phenylpyridine Ortho-Metalated Iridium(III) Complexes", Inorg. Chem, vol. 27, pages 3464-3471.

[7] King & Watts, 1987, "Dual Emission from an Ortho-Metalated Ir(III) Complex", J. Am. Chem. Soc, vol. 109, pages 1589-1590.

[8] Leo et al, 1971, "Partition coefficients and their uses", Chem. Rev., vol. 71, pages 525-616.

[9] Demas & Crosby, 1971, "The Measurement of Photoluminescence Quantum Yields", J. Phys. Chem., vol. 75, pages 991-1024.

[10] Wu et al, 2010, "Dynamics of the Excited States of [Ir(Ppy)2bpy]+ with Triple Phosphorescence", J. Phys. Chem. A, vol. 114, pages 10339-10344.

[11] Sauvageot et al, 2014, "Iridium(III) Dipyridylamine Complexes: Synthesis, Characterization and Catalytic Activities in Photoredox Reactions", Org. Chem. Front., vol. 1, pages 639-644.

[12] Lau et al, 2009, "Luminescent Cyclometalated Iridium(III) Polypyridine Indole Complexes-Synthesis, Photophysics, Electrochemistry, Protein-Binding Properties, Cytotoxicity, and Cellular Uptake", Inorg. Chem., vol. 48, pages 708-718.

[13] Li et al, 2012, "Cyclometalated Iridium(III)-Polyamine Complexes with Intense and Long-Lived Multicolor Phosphorescence: Synthesis, Crystal Structure, Photophysical Behavior, Cellular Uptake, and Transfection Properties", Chem. Eur. J., vol. 18, pages 13342-13354.

[14] Yang et al, 2010, "Iridium Metal Complexes Containing N-Heterocyclic Carbene Ligands for Blue-Light-Emitting Electrochemical Cells", Inorg. Chem., vol. 49, pages 9891-9901.

[15] Monti et al, 2013, "Charged Bis-Cyclometalated Iridium(III) Complexes with Carbene-Based Ancillary Ligands", Inorg. Chem., vol. 52, pages 10292-10305.

[16] Yang et al, 2020, "Electron Storage Capability and Singlet Oxygen Productivity of a RuII Photosensitizer Containing a Fused Naphthaloylenebenzene Moiety at the 1,10-Phenanthroline Ligand", Chem. Eur. J., vol. 26, pages 17027-17034.

[17] Feng et al, 2017, "Hyperbranched Phosphorescent Conjugated Polymer Dots with Iridium(III) Complex as the Core for Hypoxia Imaging and Photodynamic Therapy", ACS Appl. Mater. Interfaces, vol. 9, pages 28319-28330.

[18] Mosmann, 1983, "Rapid colorimetric assay for cellular growth and survival: application to proliferation and cytotoxicity assays", J Immunol Methods, vol. 65, pages 55-63.

[19] Zhao et al, 2020, "Mediated Imaging and Improved Targeting of Farnesylthiosalicylic Acid Delivery for Pancreatic Cancer via Conjugation with Near-Infrared Fluorescence Heptamethine Carbocyanine Dye", ACS Applied Biomaterials, vol. 2, pages 1129-1138.

## Claims

1. An iridium(III) complex of formula (I)

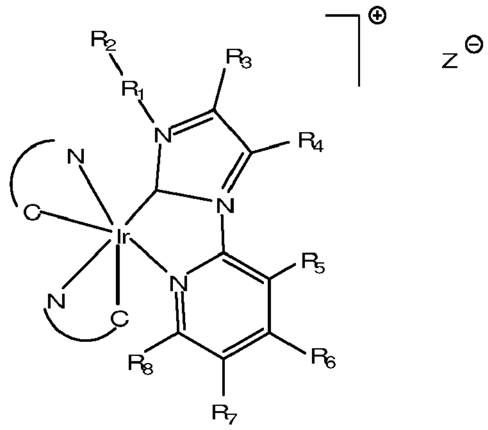

(I)

in which

$Z^-$ represents a halide anion, a nitrate anion ($-NO_3^-$) or a non-coordinating anion,
$-R_1-$ represents a covalent link or a spacer segment,
the radical $R_2$ represents a targeting entity or ferrocene,
the radicals $R_3$ and $R_4$, identical or different, represent a hydrogen atom, a halogen atom, an alkyl group optionally substituted, an aryl group optionally substituted, an alkylaryl group optionally substituted, an arylalkyl group optionally substituted, an ether group (-OR), an amine group (-NRR'), a borane group (-BRR'), a thioether group (-SR), and a phosphine group (-PRR');
the radicals $R_5$, $R_6$, $R_7$ and $R_8$, identical or different, represent a hydrogen atom, a halogen atom, a hydroxyl group or an ether group (-OR), a cyano group (-CN), a nitro group ($-NO_2$), a carboxylic group or a carboxylic acid ester group (-C(=O)OR), an alkyl group optionally substituted, an aryl group optionally substituted, an alkylaryl group optionally substituted, an arylalkyl group optionally substituted, an aldehyde group or a ketone group (-C(=O)R), an amine group (-NRR'), a cyanate group (-OC≡N), an isocyanate group (-N=C=O), a carboxamide group (-C(=O)NRR'), a boronic acid group ($-B(OR)_2$), a sulfhydryl group or a thioether group (-SR), an iminothiol group (-C(NR)SR') and a trimethylsilylacetylene group (-CCSiMe3);
the ligands

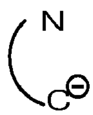

are either of formula (II) or of formula (III)

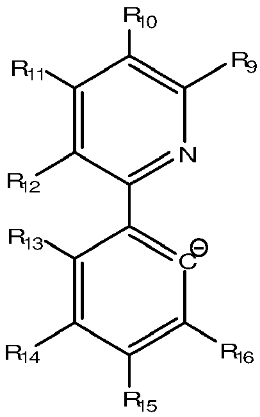
(II) or
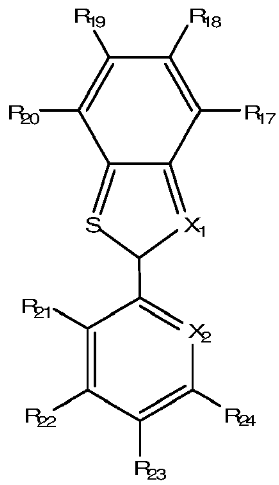
(III)

in which

($X_1$, $X_2$) represents either (N, C⊖) or (C⊖, N),
the radicals $R_9$ to $R_{24}$, identical or different, represent a hydrogen atom, a halogen atom, a cyano group (-CN), a nitro group ($-NO_2$), a carboxylic group or a carboxylic acid ester group (-COOR), an alkyl group optionally substituted, an aryl group optionally substituted, an alkylaryl group optionally substituted or an arylalkyl group optionally substituted;
the radicals $R_9$ and $R_{10}$, the radicals $R_{10}$ and $R_{11}$, the radicals $R_{11}$ and $R_{12}$, the radicals $R_{12}$ and $R_{13}$, the radicals $R_{13}$ and $R_{14}$, the radicals $R_{14}$ and $R_{15}$, the radicals $R_{15}$ and $R_{16}$, the radicals $R_{17}$ and $R_{18}$, the radicals $R_{18}$ and $R_{19}$, the radicals $R_{19}$ and $R_{20}$, the radicals $R_{21}$ and $R_{22}$, the radicals $R_{22}$ and $R_{23}$ and the radicals $R_{23}$ and $R_{24}$ may form with the carbons to which they are attached a conjugated system of delocalized electrons,
R and R', identical or different, representing a hydrogen atom or an alkyl group optionally substituted.

2. The iridium(III) complex according to claim 1, wherein said non-coordinating anion is selected from the group consisting of tetrafluoroborate ($BF_4^-$), hexafluorophosphate ($PF_6^-$), benzenesulfonate ($CH_5SO_3^-$), bis(trifluoromethanesulfonyl)imide ($(N[SO_2(CF_3)]_2)^-$), methanesulfonate (mesylate, $CH_3SO_3^-$), perchlorate ($ClO_4^-$), tetrachloroaluminate ($AlCl_4^-$), tetrakis[3,5-bis(trifluoromethyl)phenyl]borate ($B[3,5-(CF_3)_2C_6H_3]_4^-$), tetrakis (hexafluoroisopropyl)aluminate ($Al[OC(CF_3)_3]_4^-$), tetrakis(pentafluorophenyl) borate ($B[C_6F_5]_4^-$), p-toluene sulfonate (tosylate, $CH_3C_6H_4SO_2^-$) and trifluoromethanesulfonate (triflate, $CF_3SO_3^-$).

3. The iridium(III) complex according to claim 1 or 2, wherein said spacer segment is selected from the group consisting of an alkylene chain optionally substituted, an alkenylene chain optionally substituted, an alkynylene chain optionally substituted and an arylene chain optionally substituted.

4. The iridium(III) complex according to any one of claims 1 to 3, wherein said targeting entity is selected from the group consisting in PSMA (for "Prostate Specific Membrane Antigen") inhibitor such as any antibody specifically binding PSMA or the molecule Lys-urea-Glu; the cyclic tripeptide Arg-Gly-Asp (RGD); Herceptin; Trastuzumab; antibody specifically binding to the nectin 4 antigen; biotin and FTS (for "Farnesyl Thiosalicylic Acid").

5. The iridium(III) complex according to any one of claims 1 to 4, wherein said radicals $R_3$ and $R_4$ are any one of the combinations defined in the below Table 1 with R, R', R", R‴ and R⁗, identical or different, representing a hydrogen atom or an alkyl group optionally substituted.

Table 1

| Combination | $R_3$ | $R_4$ |
|---|---|---|
| 1 | H | H |
| 2 | Me | Me |

(continued)

| Combination | $R_3$ | $R_4$ |
|---|---|---|
| 3 | CRR’R" | H, SR"’, NR‴ R⁗, OR"’ |
| 4 | OR | H, OR’ |
| 5 | NR’R" | H, N R‴ R"" |
| 6 | SR | H, SR’ |
| 7 | PRR’ | H, PR″R‴ |
| 8 | F | H |
| 9 | BRR’ | H |
| 10 | H, SR"’, NR‴R⁗, OR"’ | CRR’R" |
| 11 | H, OR’, | OR |
| 12 | H, NR‴R⁗ | NR’R" |
| 13 | H, SR’ | SR |
| 14 | H, PR″R‴ | PRR’ |
| 15 | H | F |
| 16 | H | BRR’ |

**6.** The iridium(III) complex according to any one of claims 1 to 5, wherein said radicals $R_5$, $R_6$, $R_7$, and $R_8$ are any one of the combinations defined in the below Table 2 with R and R’, identical or different, representing a hydrogen atom or an alkyl group optionally substituted.

Table 2

| Combination | $R_5$ | $R_6$ | $R_7$ | $R_8$ |
|---|---|---|---|---|
| 20 | H | H | H | H |
| 21 | H | H | H | Me, CN, OMe, $CF_3$, C(O)R, $CH_2OH$, $CHF_2$, NHR, pyridine, COOR, $NR_2$, F, $B(OR)_2$ |
| 22 | H | H | Me, CN, OMe, $CF_3$, C (O)R, $CH_2OR$, $CHF_2$, NHR, pyridine, COOR, $NR_2$, F, $CH_2NHR$, $B(OR)_2$, $CH_2CN$, NCO, $NO_2$, | H |
| | | | CCSiMe3, SMe, C (NR)SR’ | |
| 23 | H | Me, Et, CN, OMe, $CF_3$, C (O)R, $CH_2OR$, $CRF_2$, NHR, pyridine, COOR, $NR_2$, F, $CH_2NHR$, $B(OR)_2$, $CH_2CN$, NCO, $NO_2$, CCSiMe3, SMe, C(NR)SR’, C(O)NHR | H | H |

(continued)

| Combination | $R_5$ | $R_6$ | $R_7$ | $R_8$ |
|---|---|---|---|---|
| 24 | Me, CN, OMe, $CF_3$, C(O) R, $CH_2OR$, $CHF_2$, NHR, pyridine, COOR, $NR_2$, F, $CH_2NHR$, $B(OR)_2$, $CH_2CN$, NCO, $NO_2$, CCSiMe3, SMe, C(NR) SR' | H | H | H |
| 25 | Me, $NH_2$, C(O)H | H | F | H |
| 26 | $NH_2$, $NO_2$ | H | Me | H |
| 27 | F, | H | COOR | H |
| 28 | Me, CN | H | $NO_2$ | H |
| 29 | C(O)R | H | F, $CF_3$, Ph | H |
| 30 | H | H | $NH_2$, COOR | $NH_2$ |
| 31 | H | H | Me, F, COOR | Me |
| 32 | H | H | C(O)H | F |
| 33 | H | H | OMe, $B(OR)_2$, COOR, C(O)R | OMe |
| 34 | H | H | Me | C(O)R |
| 35 | $NH_2$ | $NH_2$, Me, $CF_3$ | H | H |
| 36 | $CF_3$ | F, COOR | H | H |
| 37 | COOR | NHR | H | H |
| 38 | CN | $B(OR)_2$ | H | H |
| 39 | F | C(O)R | H | H |
| 40 | $NO_2$ | Me | H | H |
| 41 | $NO_2$ | H | H | OMe |
| 42 | COOR | H | H | $CF_3$ |
| 43 | NHR | H | H | $CH_2OR$, COOR |
| 44 | H | Me, COOR | NHR | H |
| 45 | H | Me | $NO_2$ | H |

**7.** The iridium(III) complex according to any one of claims 1 to 6, wherein said iridium(III) complex is of any one of the following formulae (IV), (V), (VI) and (VII):

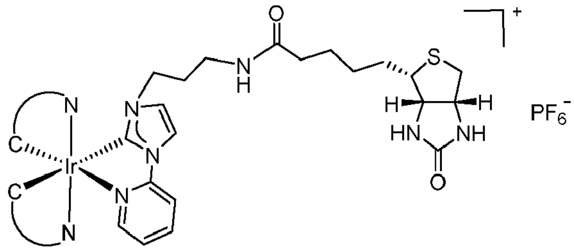

IV

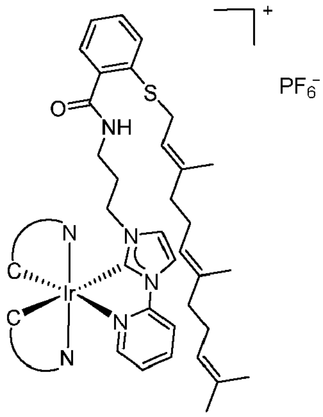

V

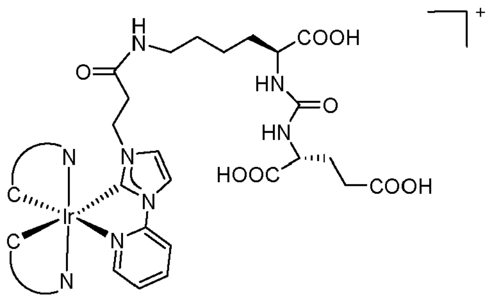

VI

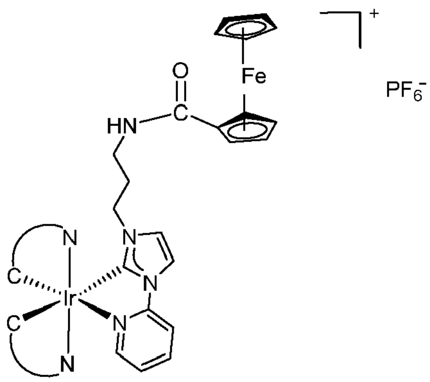

VII

8. The iridium(III) complex according to any one of claims 1 to 7, wherein said ligands C^N is selected from the group consisting of 2-phenylpyridine (ppy (**a**)), 2-*p*-tolylpyridine (4-Me-ppy **(b)),** methyl 4-(pyridin-2-yl)benzoate (4-COOMe-ppy **(c)),** methyl 3-(pyridin-2-yl)benzoate (3-COOMe-ppy **(d)),** methyl 6-phenylnicotinate (*p*-3-COOMe-ppy **(e)),** 6-phenylnicotinic acid (*p*-3-COOH-ppy **(f)),** 2-phenylquinoline (pq **(g)),** 1-phenylisoquinoline (piq **(h)),** 7,8-benzoquinoline (bzq **(i)),** 2-(2-pyridyl)benzothiophene (thpy **(j)),** 2-phenylbenzothiazole (pbt **(k)),** and 2-(2,4-difluorophenyl)pyridine (2,4-F-ppy (I)).

**9.** The iridium(III) complex according to any one of claims 1 to 8, for use as a medicament.

**10.** A pharmaceutical composition comprising, as active ingredient, an iridium(III) complex according to any one of claims 1 to 8 and a pharmaceutically acceptable vehicle.

**11.** The iridium(III) complex according to any one of claims 1 to 8 or the pharmaceutical composition according to claim 10, for use in the treatment and/or prevention of cancer.

**12.** The iridium(III) complex or the pharmaceutical composition, for use according to claim 11, wherein said cancer is selected from the group consisting of melanoma, colorectal cancer, colon cancer, Kaposi's sarcoma, glioblastoma, ovarian cancer, breast cancer, liver cancer, pancreatic cancer, lung cancer, bladder cancer, prostate cancer, or neuroendocrine tumors.

**13.** The iridium(III) complex or the pharmaceutical composition, for use according to claim 11 or 12, wherein

- the iridium(III) complex is of formula (IV) as defined in claim 8 in which the C^N ligands are either 2-phenylquinolines (pq **(g)**) or 2-phenylbenzothiazoles (pbt **(k)**) and the cancer to be treated and/or prevented is bladder cancer, or
- the iridium(III) complex is of formula (V) as defined in claim 8 in which the C^N ligands are selected in the group consisting of 1-phenylisoquinolines (piq **(h)),** 2-(2-pyridyl)benzothiophenes (thpy **(j)**) and 2-phenylbenzothiazoles (pbt **(k)**) and the cancer to be treated and/or prevented is bladder cancer, or
- the iridium(III) complex is of formula (VI) as defined in claim 8 in which the C^N ligands are either 1-phenylisoquinolines (piq **(h)**) or 2-phenylbenzothiazoles (pbt **(k)**) and the cancer to be treated and/or prevented is prostate cancer, or
- the iridium(III) complex is of formula (VII) as defined in claim 8 in which the C^N ligands are either 1-phenylisoquinolines (piq **(h)**) or 2-(2-pyridyl)benzothiophenes (thpy (j)) and the cancer to be treated and/or prevented is bladder cancer or prostate cancer.

**14.** The iridium(III) complex according to any one of claims 1 to 8 or the pharmaceutical composition according to claim 10, for use in diagnosis and, in particular, in photodynamic diagnosis.

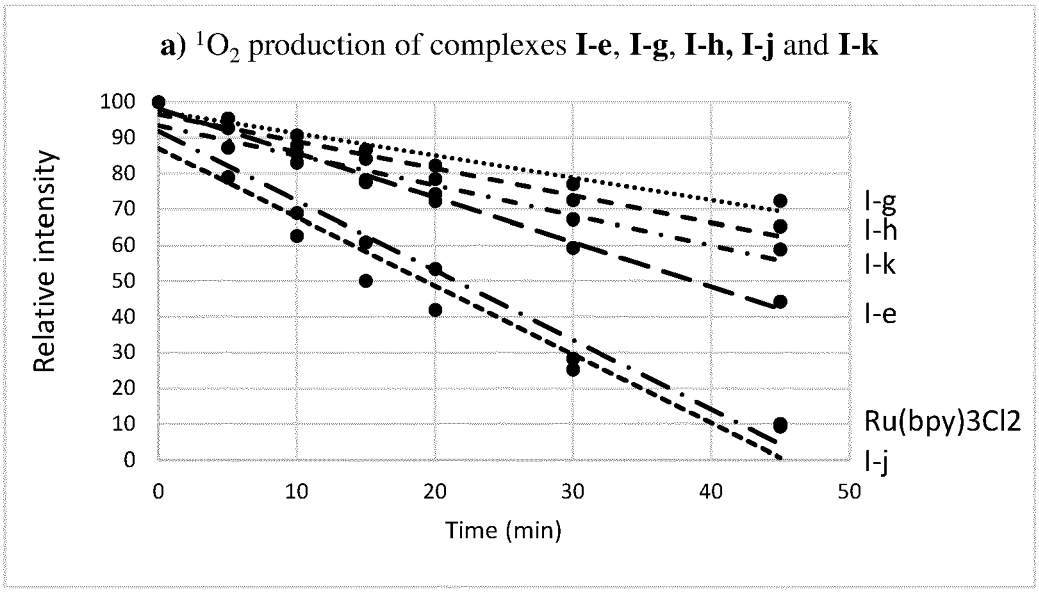
a) $^1O_2$ production of complexes **I-e**, **I-g**, **I-h, I-j** and **I-k**
Relative intensity
100
90
80
70
60
50
40
30
20
10
0
0
10
20
30
40
50
Time (min)
I-g
I-h
I-k
I-e
Ru(bpy)3Cl2
I-j

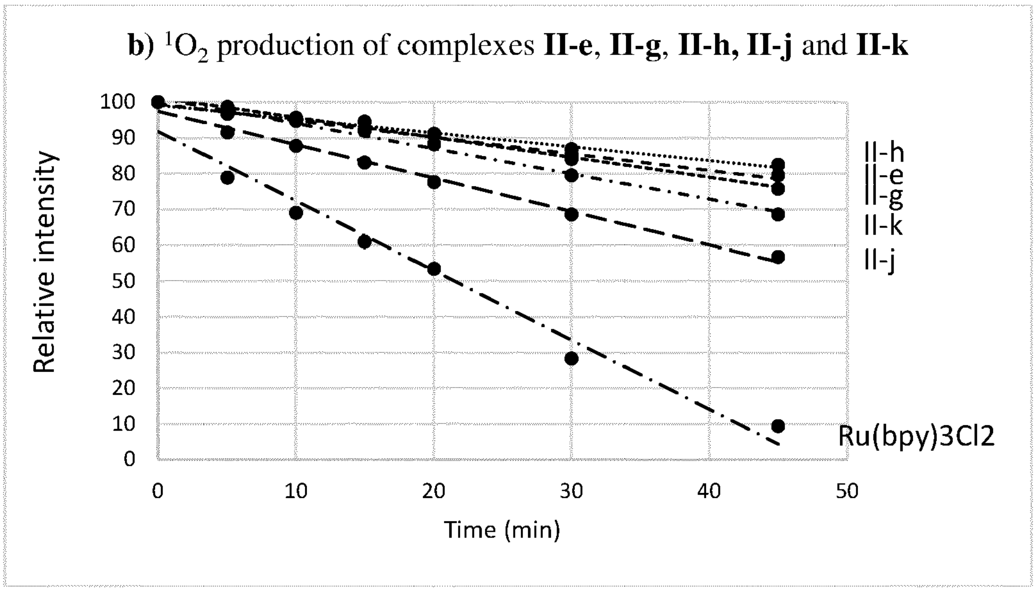
b) $^1O_2$ production of complexes **II-e**, **II-g**, **II-h, II-j** and **II-k**
Relative intensity
100
90
80
70
60
50
40
30
20
10
0
0
10
20
30
40
50
Time (min)
II-h
II-e
II-g
II-k
II-j
Ru(bpy)3Cl2

Fig. 1

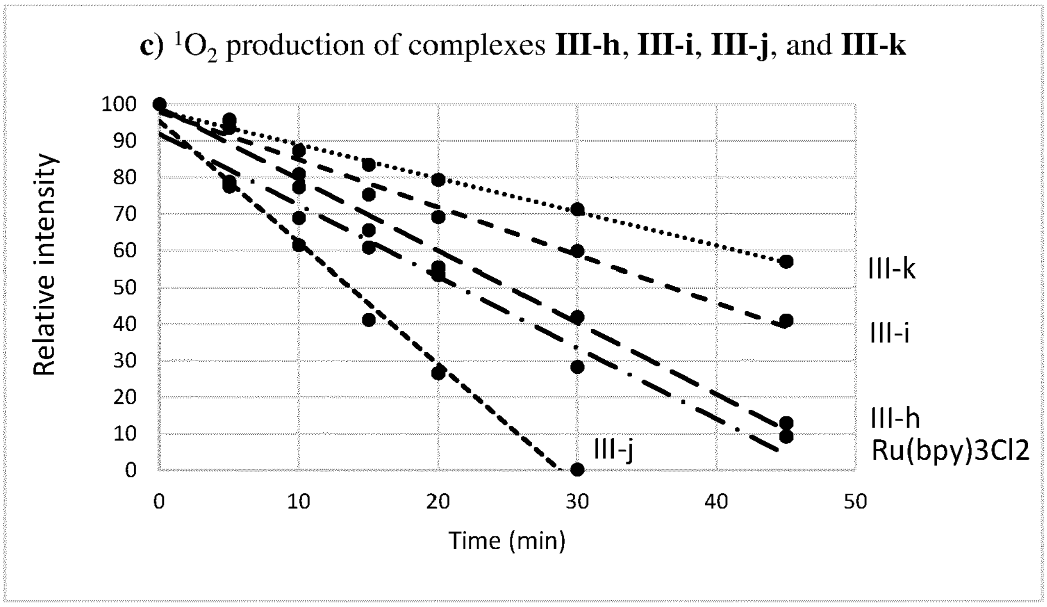


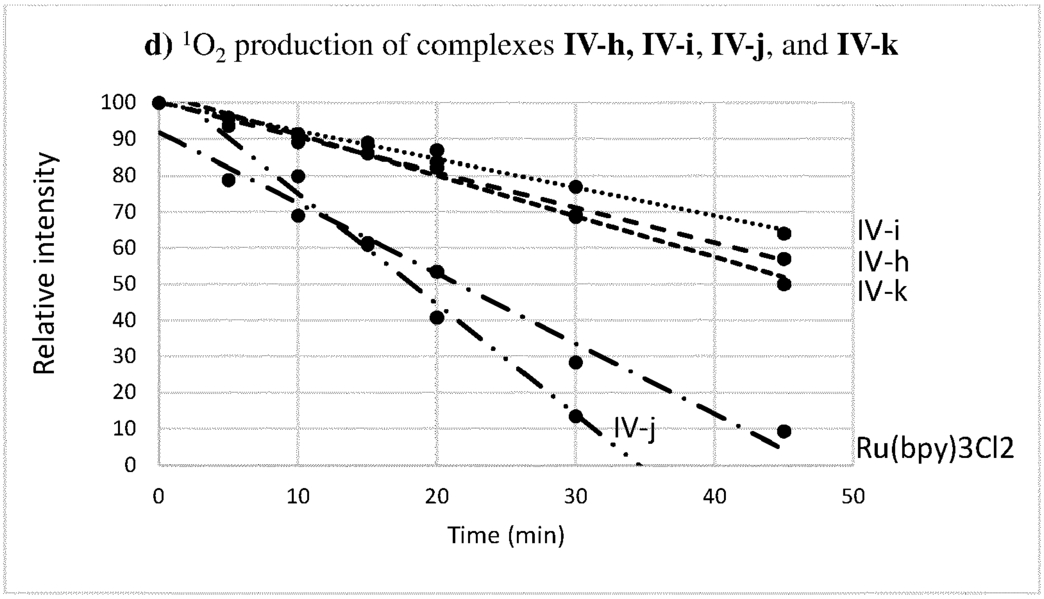


Fig. 1 (continued)

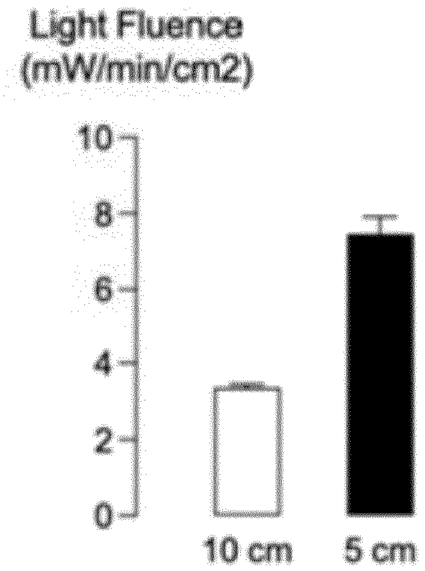
Light Fluence
(mW/min/cm2)
10
8
6
4
2
0
10 cm
5 cm

Fig. 2A

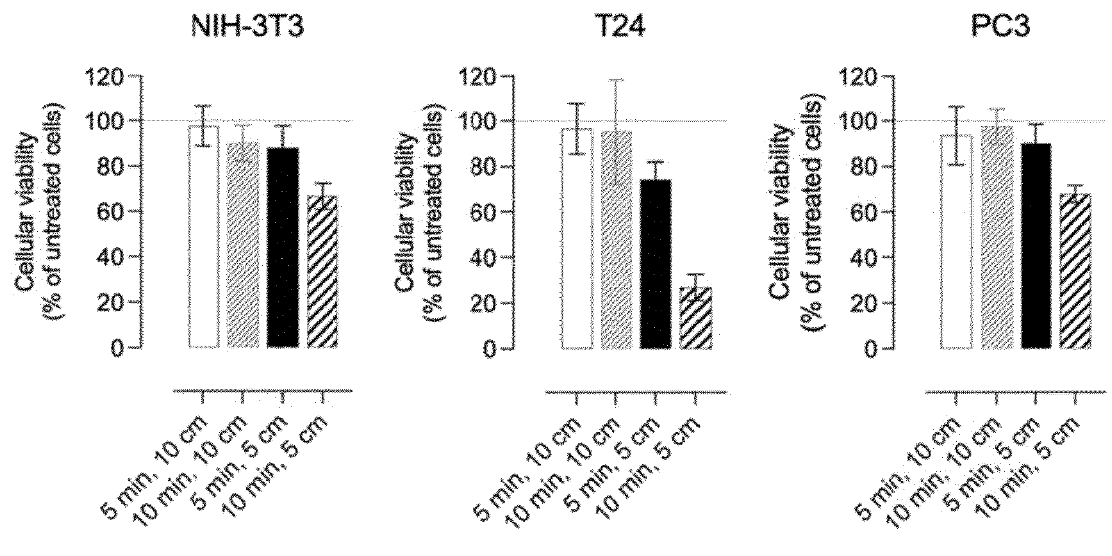
NIH-3T3
T24
PC3
Cellular viability
(% of untreated cells)
120
100
80
60
40
20
0
5 min, 10 cm
10 min, 10 cm
5 min, 5 cm
10 min, 5 cm

Fig. 2B

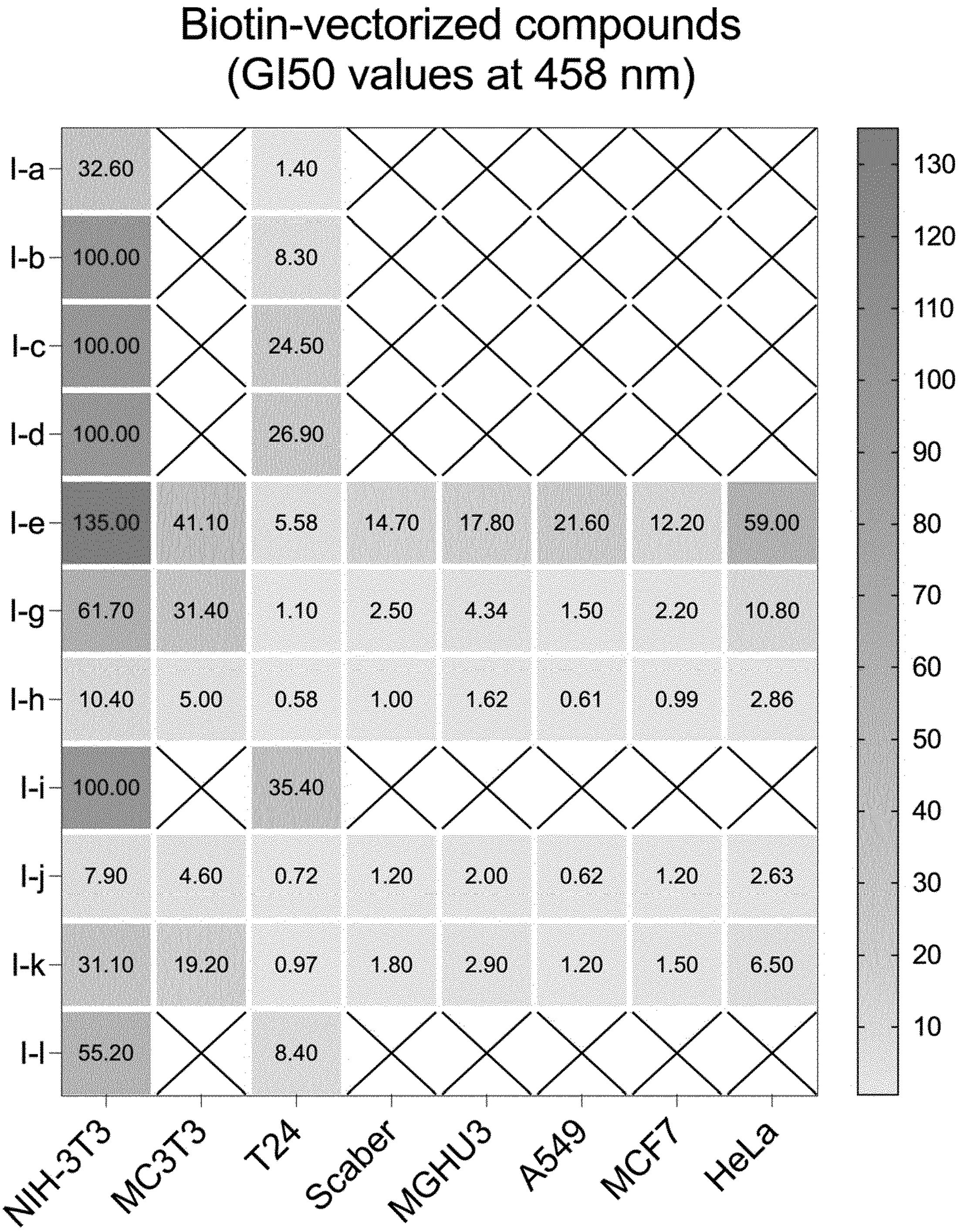
Biotin-vectorized compounds
(GI50 values at 458 nm)
I-a
32.60
1.40
I-b
100.00
8.30
I-c
100.00
24.50
I-d
100.00
26.90
I-e
135.00
41.10
5.58
14.70
17.80
21.60
12.20
59.00
I-g
61.70
31.40
1.10
2.50
4.34
1.50
2.20
10.80
I-h
10.40
5.00
0.58
1.00
1.62
0.61
0.99
2.86
I-i
100.00
35.40
I-j
7.90
4.60
0.72
1.20
2.00
0.62
1.20
2.63
I-k
31.10
19.20
0.97
1.80
2.90
1.20
1.50
6.50
I-l
55.20
8.40
NIH-3T3
MC3T3
T24
Scaber
MGHU3
A549
MCF7
HeLa
130
120
110
100
90
80
70
60
50
40
30
20
10

Fig. 3

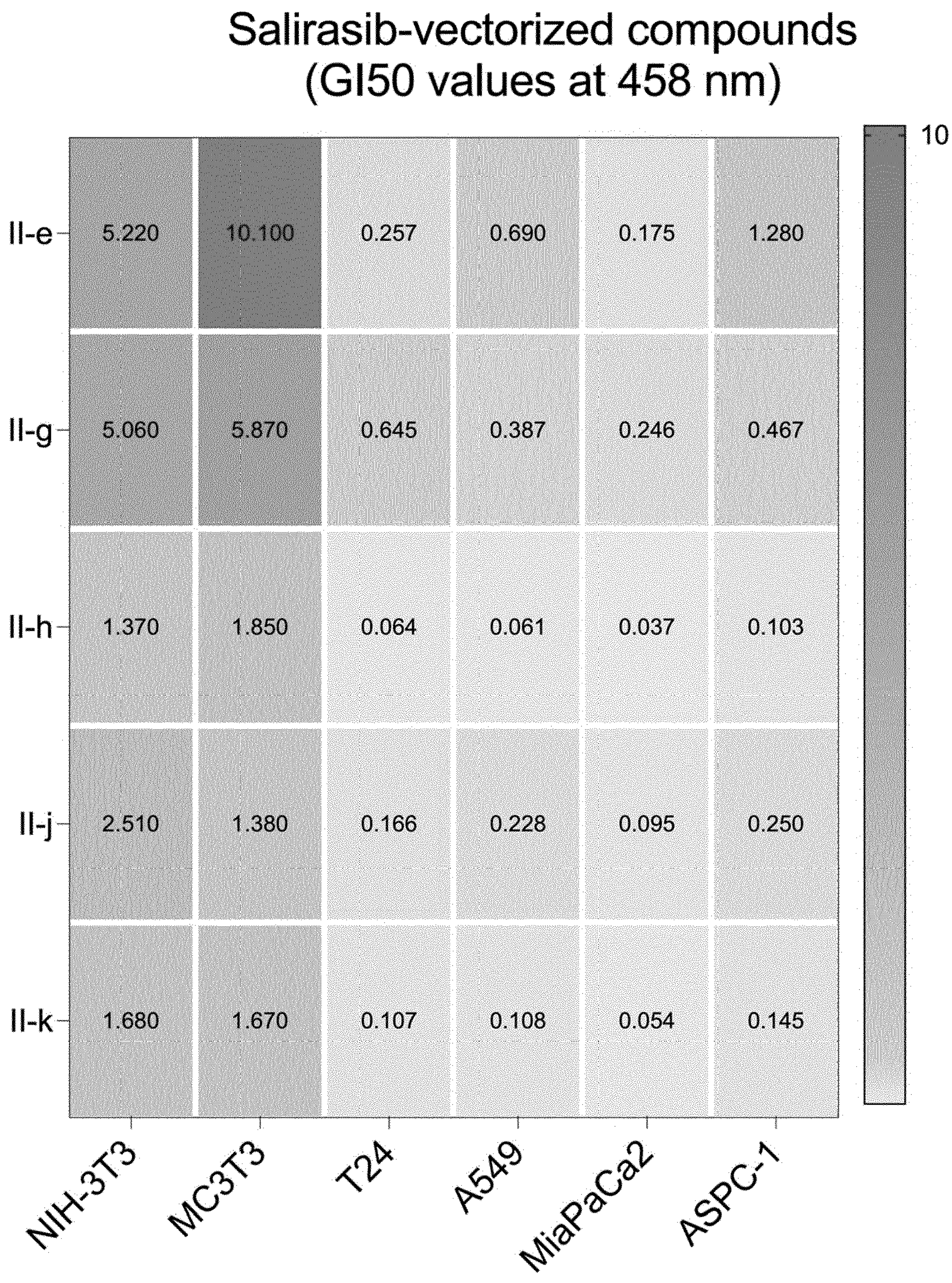
Salirasib-vectorized compounds
(GI50 values at 458 nm)
10
II-e
II-g
II-h
II-j
II-k
5.220
10.100
0.257
0.690
0.175
1.280
5.060
5.870
0.645
0.387
0.246
0.467
1.370
1.850
0.064
0.061
0.037
0.103
2.510
1.380
0.166
0.228
0.095
0.250
1.680
1.670
0.107
0.108
0.054
0.145
NIH-3T3
MC3T3
T24
A549
MiaPaCa2
ASPC-1

Fig. 4

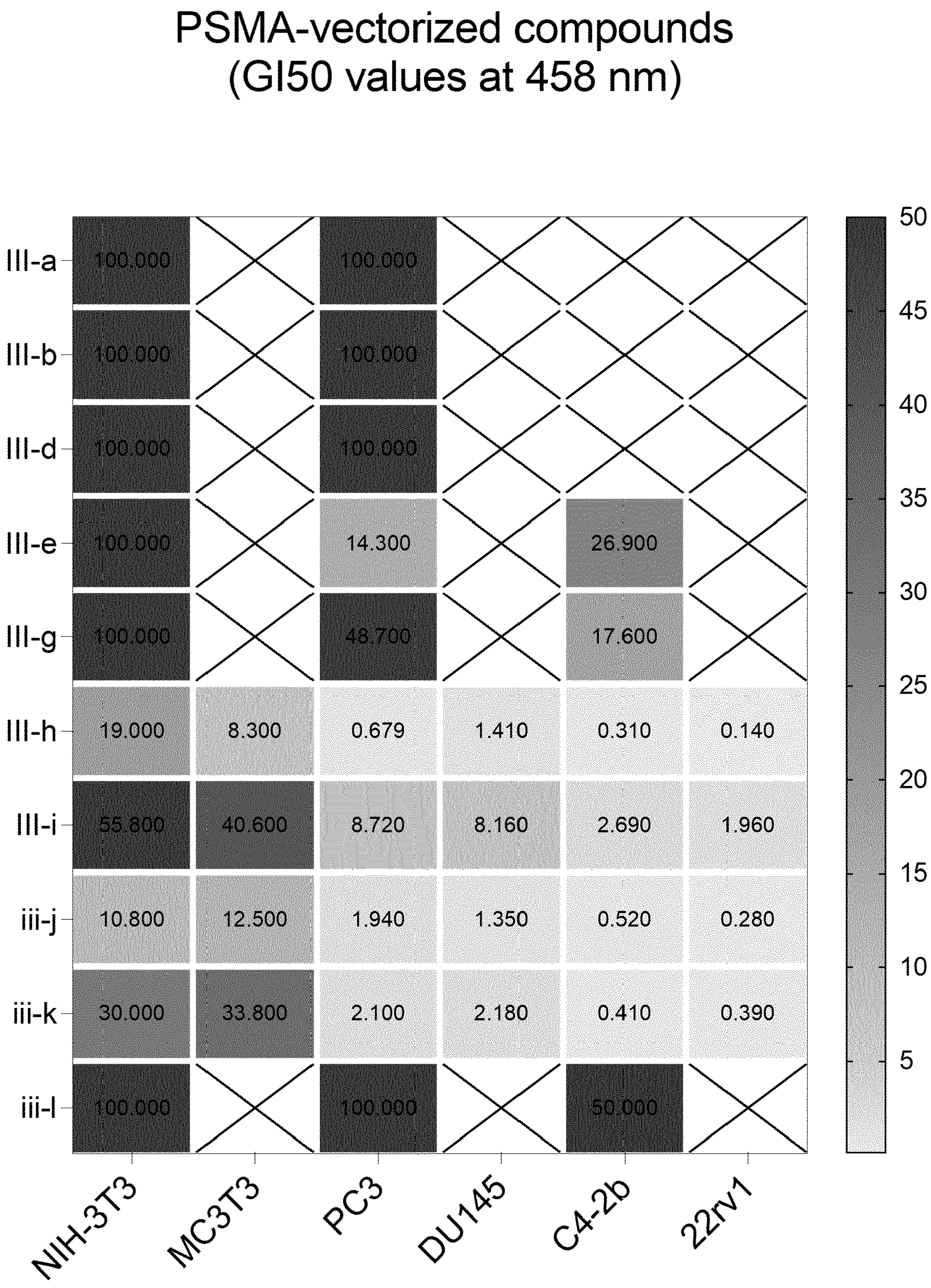
PSMA-vectorized compounds
(GI50 values at 458 nm)
III-a
III-b
III-d
III-e
III-g
III-h
III-i
iii-j
iii-k
iii-l
100.000
100.000
100.000
100.000
100.000
100.000
100.000
100.000
14.300
26.900
100.000
48.700
17.600
19.000
8.300
0.679
1.410
0.310
0.140
55.800
40.600
8.720
8.160
2.690
1.960
10.800
12.500
1.940
1.350
0.520
0.280
30.000
33.800
2.100
2.180
0.410
0.390
100.000
100.000
50.000
NIH-3T3
MC3T3
PC3
DU145
C4-2b
22rv1
50
45
40
35
30
25
20
15
10
5

Fig. 5

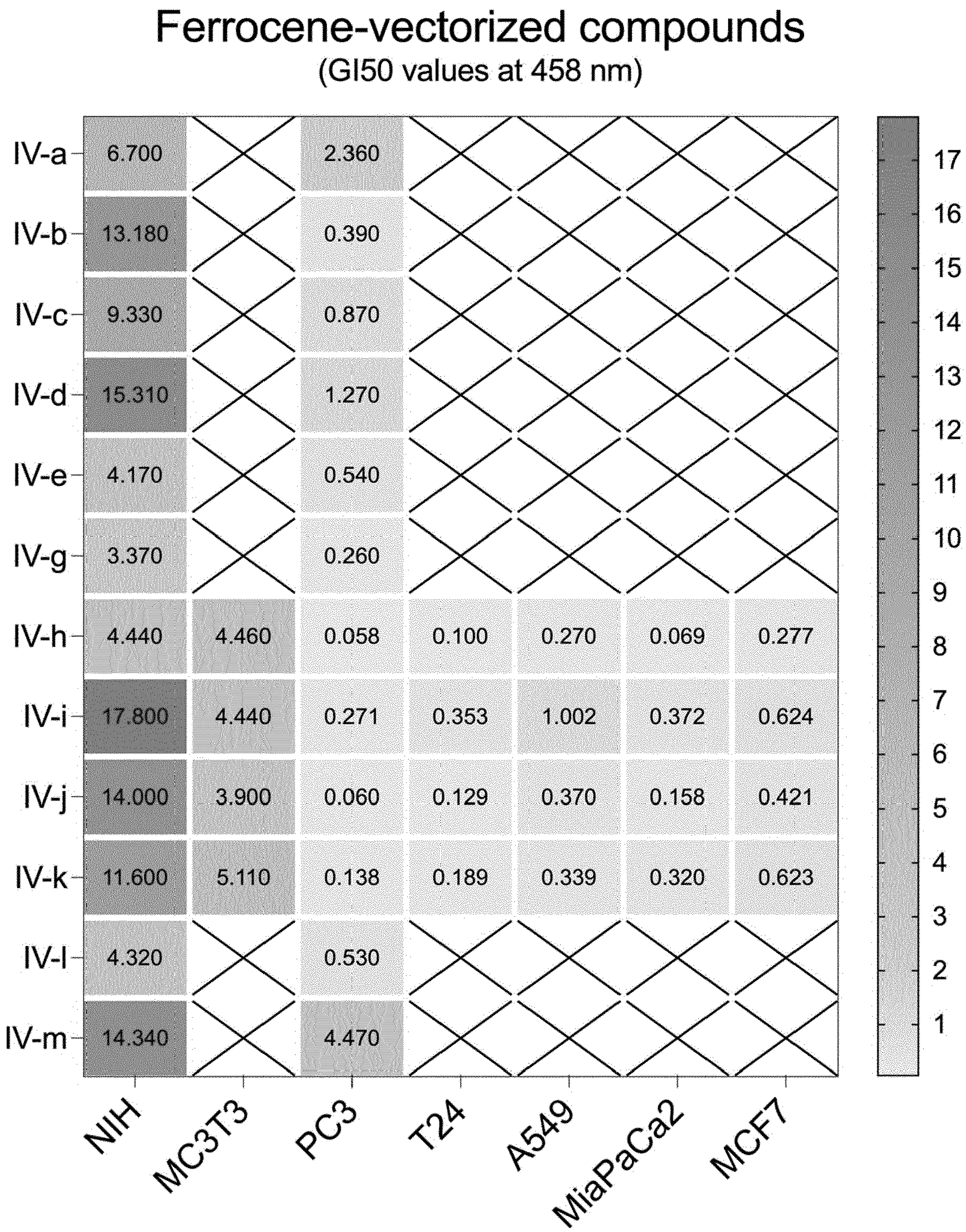
Ferrocene-vectorized compounds
(GI50 values at 458 nm)
IV-a
IV-b
IV-c
IV-d
IV-e
IV-g
IV-h
IV-i
IV-j
IV-k
IV-l
IV-m
6.700
2.360
13.180
0.390
9.330
0.870
15.310
1.270
4.170
0.540
3.370
0.260
4.440
4.460
0.058
0.100
0.270
0.069
0.277
17.800
4.440
0.271
0.353
1.002
0.372
0.624
14.000
3.900
0.060
0.129
0.370
0.158
0.421
11.600
5.110
0.138
0.189
0.339
0.320
0.623
4.320
0.530
14.340
4.470
NIH
MC3T3
PC3
T24
A549
MiaPaCa2
MCF7
17
16
15
14
13
12
11
10
9
8
7
6
5
4
3
2
1

Fig. 6

Europäisches Patentamt
European Patent Office
Office européen des brevets

# EUROPEAN SEARCH REPORT

Application Number
EP 22 30 6855

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CHEN HAIJIE ET AL: "Isomeric Ir (III) complexes for tracking mitochondrial pH fluctuations and inducing mitochondrial dysfunction during photodynamic therapy", DALTON TRANSACTIONS, vol. 48, no. 46, 1 January 2019 (2019-01-01), pages 17200-17209, XP093045672, Cambridge ISSN: 1477-9226, DOI: 10.1039/C9DT03453F * complex 1 figure 1 page 17201 * ----- | 1-14 | INV. C07F15/00 C07F17/02 A61K31/351 A61P35/00 C12N5/095 |

TECHNICAL FIELDS SEARCHED (IPC)
C07F
C12N
A61K
A61P

The present search report has been drawn up for all claims

1

| Place of search | Date of completion of the search | Examiner |
|---|---|---|
| The Hague | 10 May 2023 | Bourghida, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **ZHU et al.** Simple Copper Salt-Catalyzed N-Arylation of Nitrogen-Containing Heterocycles with Aryl and Heteroaryl Halides. *J. Org. Chem.,* 2007, vol. 72, 8535-8538
- **SUSUMU et al.** Enhancing the Stability and Biological Functionalities of Quantum Dots via Compact Multifunctional Ligands. *J. Am. Chem. Soc.,* 2007, vol. 129, 13987-13996
- **PORTA et al.** Repositioning Salirasib as a New Antimalarial Agent. *Med. Chem. Commun.,* 2019, vol. 10, 1599-1605
- **MARESCA et al.** A Series of Halogenated Heterodimeric Inhibitors of Prostate Specific Membrane Antigen (PSMA) as Radiolabeled Probes for Targeting Prostate Cancer. *J. Med. Chem.,* 2009, vol. 52, 347-357
- **LINDSAY ; HAUSER.** Aminomethylation of Ferrocene to Form N,N-Dimethylaminomethylferrocene and Its Conversion to the Corresponding Alcohol and Aldehyde. *C. R. J. Org. Chem.,* 1957, vol. 22, 355-358
- **GARCES et al.** Synthesis, Structure, Electrochemistry, and Photophysics of Methyl-Substituted Phenylpyridine Ortho-Metalated Iridium(III) Complexes. *Inorg. Chem,* 1988, vol. 27, 3464-3471
- **KING ; WATTS.** Dual Emission from an Ortho-Metalated Ir(III) Complex. *J. Am. Chem. Soc,* 1987, vol. 109, 1589-1590
- **LEO et al.** Partition coefficients and their uses. *Chem. Rev.,* 1971, vol. 71, 525-616
- **DEMAS ; CROSBY.** The Measurement of Photoluminescence Quantum Yields. *J. Phys. Chem.,* 1971, vol. 75, 991-1024
- **WU et al.** Dynamics of the Excited States of [Ir(Ppy)2bpy]+ with Triple Phosphorescence. *J. Phys. Chem. A,* 2010, vol. 114, 10339-10344
- **SAUVAGEOT et al.** Iridium(III) Dipyridylamine Complexes: Synthesis, Characterization and Catalytic Activities in Photoredox Reactions. *Org. Chem. Front.,* 2014, vol. 1, 639-644
- **LAU et al.** Luminescent Cyclometalated Iridium(III) Polypyridine Indole Complexes-Synthesis, Photophysics, Electrochemistry, Protein-Binding Properties, Cytotoxicity, and Cellular Uptake. *Inorg. Chem.,* 2009, vol. 48, 708-718
- **LI et al.** Cyclometalated Iridium(III)-Polyamine Complexes with Intense and Long-Lived Multicolor Phosphorescence: Synthesis, Crystal Structure, Photophysical Behavior, Cellular Uptake, and Transfection Properties. *Chem. Eur. J.,* 2012, vol. 18, 13342-13354
- **YANG et al.** Iridium Metal Complexes Containing N-Heterocyclic Carbene Ligands for Blue-Light-Emitting Electrochemical Cells. *Inorg. Chem.,* 2010, vol. 49, 9891-9901
- **MONTI et al.** Charged Bis-Cyclometalated Iridium(III) Complexes with Carbene-Based Ancillary Ligands. *Inorg. Chem.,* 2013, vol. 52, 10292-10305
- **YANG et al.** Electron Storage Capability and Singlet Oxygen Productivity of a RuII Photosensitizer Containing a Fused Naphthaloylenebenzene Moiety at the 1,10-Phenanthroline Ligand. *Chem. Eur. J.,* 2020, vol. 26, 17027-17034
- **FENG et al.** Hyperbranched Phosphorescent Conjugated Polymer Dots with Iridium(III) Complex as the Core for Hypoxia Imaging and Photodynamic Therapy. *ACS Appl. Mater. Interfaces,* 2017, vol. 9, 28319-28330
- **MOSMANN.** Rapid colorimetric assay for cellular growth and survival: application to proliferation and cytotoxicity assays. *J Immunol Methods,* 1983, vol. 65, 55-63
- **ZHAO et al.** Mediated Imaging and Improved Targeting of Farnesylthiosalicylic Acid Delivery for Pancreatic Cancer via Conjugation with Near-Infrared Fluorescence Heptamethine Carbocyanine Dye. *ACS Applied Biomaterials,* 2020, vol. 2, 1129-1138